# EUROPÄISCHE PATENTANMELDUNG

(11) **EP 1 903 037 A1**
(43) Veröffentlichungstag der Anmeldung: **26.03.2008**
(21) Anmeldenummer: 06090158.4
(22) Anmeldetag: 07.09.2006
(51) Int. Cl.: C07D 401/04, A61K 31/4709, A61K 31/4725, A61K 31/502, A61K 31/517, A61P 15/00

(54) **1-(Het)aryl-3-[hetaryl-piperidin-4-yl]-thioharnstoffe als Modulatoren des EP2-Rezeptors**

(71) Anmelder: Bayer Schering Pharma Aktiengesellschaft, 13353 Berlin (DE)
(72) Erfinder: Bräuer, Nico, 07743 Jena (DE); Buchmann, Bernd, 16540 Hohen Neuendorf (DE); Huwe, Christoph, 13505 Berlin (DE); Koppitz, Marcus, 10115 Berlin (DE); Ter Laak, Antonius, 12159 Berlin (DE); Langer, Gernot, 14612 Falkensee (DE); Lindenthal, Bernhard, 13507 Berlin (DE); Peters, Olaf, 99891 Tabarz (DE); Wintermantel, Tim, 10178 Berlin (DE)

(57) **Zusammenfassung**

Die vorliegende Erfindung betrifft Aryl-3-[(het)aryl-piperidin-4-yl]-thioharnstoffe, Verfahren zu ihrer Herstellung sowie deren Verwendung zur Herstellung von pharmazeutischen Mitteln zur Behandlung von Erkrankungen und Indikationen, die im Zusammenhang stehen mit dem EP₂-Rezeptor.

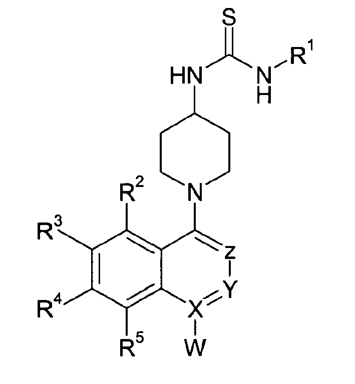

## Beschreibung

Die vorliegende Erfindung betrifft 1-(Het)aryl-3-[hetaryl-piperidin-4-yl]-thioharnstoffe als EP₂ Rezeptor Modulatoren, Verfahren zu ihrer Herstellung sowie ihre Verwendung als Arzneimittel.

Es ist schon lange bekannt, dass Prostaglandine die Schlüsselmoleküle bei den Prozessen der weiblichen Reproduktionsbiologie, wie z. B. der Regulation der Ovulation, der Fertilisation, der Nidation, der Dezidualisierung (z.B. Plazentabildung) und der Menstruation, sind. Prostaglandine spielen ebenfalls eine wichtige Rolle bei den pathologischen Veränderungen im reproduktiven Trakt, einschließlich der Menorrhagie, Dysmenorrhoe, Endometriose und Krebs. Bisher ist der Mechanismus, durch den Prostaglandine diese Veränderungen bewirken, noch nicht vollständig geklärt. Neuere Erkenntnisse weisen darauf hin, dass Prostaglandine, ihre Rezeptoren und deren Signaltransduktionswege an Prozessen wie Angiogenese, Apoptose, Proliferation sowie an inflammatorischen/antiinflammatorischen und immunologischen Prozessen beteiligt sind.

Die Wirkungen der Prostaglandine werden durch ihre G-Protein-gekoppelten Rezeptoren, die auf der Zelloberfläche lokalisiert sind, vermittelt. Von besonderem Interesse ist das Prostaglandin E₂ (PGE₂), das unterschiedlichste zelluläre Wirkungen erzielt, indem es an funktionell verschiedene Rezeptoresubtypen, nämlich die EP₁, EP₂, EP₃ und EP₄ Rezeptoren, bindet. Die Rezeptor-Subtypen, an denen das Prostaglandin E₂ bindet, scheinen für die Rezeptor-vermittelten Wirkungen, die bei der Fertilitätsregulation eine Rolle spielen, von besonderem Interesse zu sein. So konnte gezeigt werden, dass die reproduktiven Funktionen bei EP₂ Knock-out Mäusen (EP₂ ^{-/-}), d.h. bei Mäusen, die den PGE₂-Rezeptor Subtyp EP₂ nicht mehr tragen, beeinträchtigt sind, und dass diese Tiere eine kleinere "Wurfanzahl" haben (Matsumoto et al., 2001, Biology of Reproduction 64, 1557-1565). Ebenso konnte gezeigt werden, dass diese EP₂ Knock-out Mäuse (Hizaki et al. Proc Natl Acad Sci U. S. A. 1999 Aug 31; 96(18), 10501-10506) eine deutlich verminderte Kumulus Expansion und starke Subfertilität aufweisen, was die Bedeutung des Prostaglandin EP₂-Rezeptors für diesen Prozess demonstriert. Der EP₂-Rezeptor stellt demnach ein wichtiges Ziel für die Entwicklung von Arzneimitteln für die Regulation der weiblichen Fertilität dar. Die Existenz der 4 Subklassen des PGE₂-Rezeptors eröffnet die Möglichkeit zur gezielten Entwicklung selektiv wirksamer Verbindungen. Bisher sind allerdings kaum selektive EP₂-Rezeptor-Liganden bekannt, die an den EP₂-Subtypen des PGE₂-Rezeptors binden, da die meisten bekannten Verbindungen auch an die anderen PGE₂-Rezeptor-Subtypen, wie beispielsweise an den EP₄-Rezeptor, binden.

EP₂-Rezeptor Antagonisten werden beispielsweise in der Anmeldung US2005059742 beschrieben (Jabbour, Medical Research Concil). Beansprucht wird eine Methode, bei der ein EP₂- und/oder ein EP₄-Antagonist zur Behandlung von Menorrhagie und Dysmenorrhoe eingesetzt werden kann. AH6809 wird als Antagonist des EP₂- oder EP₄-Rezeptors offenbart, es werden keine anderen spezifischen Antagonisten und keine neuen Verbindungen offenbart.

In einer früheren Anmeldung derselben Gruppe (EP 1467738) werden EP₂ oder EP₄-Antagonisten zur Behandlung von pathologischen Zuständen, wie z.B. Uteruscarcinom, Myom und Endometriose, beansprucht. Es werden ebenfalls keine neuen Verbindungen offenbart.

Ono Pharmaceutical beansprucht in der Anmeldung WO03/016254 die Herstellung von Benzolsäure oder gesättigten Carbonsäure-Derivaten, die mit Aryl oder Heterocyclen substituiert sind, unter anderem als PGE₂-Rezeptor Antagonisten. Die offenbarten Verbindungen werden für die Behandlung einer Vielzahl von Erkrankungen beansprucht, darunter auch allergische Erkrankungen, Morbus Alzheimer, Schmerz, Abort, Menstruationsbeschwerden, Menorrhagie und Dysmenorrhoe, Endometriose, Erkrankungen der Knochen, Ischämie usw. Die beschriebenen Verbindungen zeichnen sich jedoch durch eine besonders hohe Affinität zum EP₃-Rezeptor aus. In einer weiteren Anmeldung (WO04/032964) werden neue Verbindungen beschrieben, die sich ebenfalls durch eine besonders hohe Affinität zum EP₃-Rezeptor auszeichnen, auch als EP₂ Antagonisten, für die Behandlung und Prophylaxe von allergischen Erkrankungen, Verwendung finden.

In der Anmeldung WO04/39807 der Firma Merck Frosst, Canada, wi1, 2 und 6rd die Herstellung von Pyridopyrrolizinen und Pyridoindolizinen offenbart. Diese Verbindungen zeichnen sich jedoch durch gute Bindung an den PGD₂₋ Rezeptor aus, dieser Rezeptor stellt einen anderen Subtyp des Prostaglandinrezeptors dar.

Naphathalenderivate als EP₄-Rezeptor Liganden werden von der SmithKline Beecham Cooperation in der Anmeldung US2004102508 offenbart. Die beanspruchten Verbindungen finden ihre Verwendung für die Behandlung oder Prophylaxe von Schmerz, allergischen Reaktionen und neurodegenerativen Erkrankungen.

EP₄-Antagonisten (γ-Lactame) werden in der Anmeldung WO03/103604 beansprucht (Applied Research Systems). Die Verbindungen binden ca. 60fach besser an den EP₄- als an den EP₂-Rezeptor und werden unter anderem zur Behandlung von vorzeitigen Wehen, Dysmenorrhoe, Asthma, Unfruchtbarkeit oder Fertilitätsstörungen beansprucht. Von derselben Firma werden in den Anmeldungen WO03/053923 (substituierte Pyrrolidine) oder WO03/035064 (substituierte Pyrazolidione) Verbindungen für die Behandlung von Erkrankungen, die mit Prostaglandinen assoziiert sind, wie beispielsweise Infertilität, Hypertension und Osteoporose, beansprucht. Die Verbindungen binden an den EP₄-und an den EP₂-Rezeptor-Subtypen. In der Anmeldung WO03/037433 werden ω-Cycloalkyl, 17 Heteroaryl-Prostaglandinderivate als EP₂-Rezeptor Antagonisten, insbesondere für die Behandlung von erhöhtem Augeninnendruck, beansprucht.

In der Anmeldung WO03/064391 (Pfizer Products) werden Metabolite von [3-[[N-(4-tert-butylbenzyl)(pyridin-3-ylsulfonyl)amino]methyl] Essigsäure beschrieben, die die Bindung von [³H] Prostaglandin-E₂ an den EP₂-Rezeptor inhibieren. Die Verwendung dieser Metabolite zur Behandlung von Osteoporose wird offenbart.

Tani *et al.* beanspruchen in der Anmeldung US2005124577 8-Azaprostaglandinderivate für die Behandlung von immunologischen Erkrankungen, allergischen Erkrankungen, vorzeitige Wehen, Abort usw. Die Verbindungen binden an den EP₂-und an den EP₄-Rezeptor.

In dem europäischen Patent EP 1306087 werden EP₂-Rezeptor Agonisten beschrieben, die ihre Verwendung bei der Behandlung der erektilen Dysfunktion finden. Die gleiche Strukturklasse wird in dem europäischen Patent EP 860430 beschrieben, ihre Verwendung für die Herstellung eines Medikaments für die Behandlung von immunologischen Erkrankungen, Asthma und Abort wird beansprucht. Die Anmeldung WO04/32965 beschreibt die EP₂-Rezeptor Agonisten, die für die Behandlung und Prävention von Erkrankungen verwendet werden, die verursacht werden durch einen durch Ischämie verursachten Organausfall. In der WO04/009117 werden EP₂- und EP₄-Rezeptor Agonisten für die Behandlung von Erkrankungen beschrieben, die verursacht werden durch die Uteruskontraktion, beispielsweise Menstruationsbeschwerden.

In den Anmeldungen WO 03/74483 und WO03/09872 werden Agonisten beschrieben, die gleichermaßen an den EP₂-und an den EP₄-Rezeptor binden (Ono Pharmaceuticals).

Die Agonisten des EP₂- und des EP₄-Rezeptors werden häufig im Zusammenhang mit der Behandlung der Osteoporose beschrieben (WO99/19300, US2003/0166631, WO03/77910, WO03/45371, WO 03/74483 und WO03/09872) und für die Glaukombehandlung (WO04/37813, WO04/37786, WO04/19938, WO03/103772, WO03/103664, US6747037, US6410591, WO03/40123, WO03/47513, WO03/47417).

In der Patentanmeldung WO04/12656 werden EP₂-Rezeptor Agonisten im Zusammenhang mit Inflammation beansprucht.

In der Patentanmeldung WO03/77919 werden EP₄-Rezeptor Agonisten für die Behandlung der Fertilität beansprucht.

Bisher sind jedoch keine selektiven EP₂-Rezeptor Agonisten und Antagonisten bekannt, die die Prozesse regulieren, die letztendlich für die Nidation und Dezidualisierung verantwortlich sind und somit zur Förderung oder Hemmung der Fertilität beitragen.

Daraus ergibt sich die Aufgabe, stabile und wirksame Verbindungen, die selektiv an den EP₂-Rezeptor binden, für die Entwicklung neuer Arzneimittel bereit zu stellen.

Überraschenderweise wurde nun gefunden, dass Verbindungen der allgemeinen Formel I 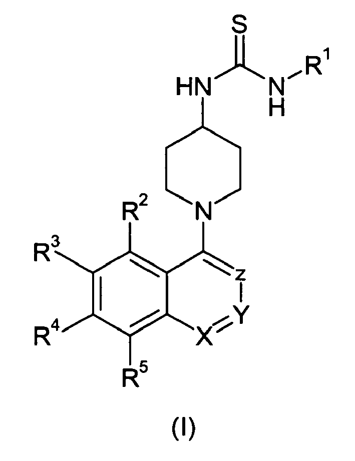 wobei
- X, Y, Z: unabhängig voneinander ein Stickstoffrest oder ein Kohlenstoffrest -C-R⁸, wobei R⁸ Wasserstoff oder ein C₁-C₄-Alkylrest sein kann, unter der Voraussetzung, dass mindestens ein, höchstens jedoch 2 der Gruppen X, Y, Z ein Stickstoffrest ist,
- R¹: ein 5-12-gliedriger, mono- oder bicyclischer Aryl- oder Heteroarylring, der unsubstituiert oder gegebenenfalls ein- bis dreifach substituiert sein kann,
- R²- R⁵: unabhängig voneinander ein Wasserstoff, Halogen, Cyano,
- oder eine OR⁶, OC(O)R⁶, S(O)ₙR⁶, wobei n = 0, 1, 2 bedeutet, SO₂NHR⁶, SO₂NHC(O)R⁶, NR⁶R⁷, NHC(O)R⁶, CH₂NR⁶R⁷, CH₂NHC(O)R⁶, C(OH)R⁶R⁷, C(O)R⁶, CO₂R⁶, C(O)NR⁶R⁷-Gruppe,
- eine C₁-C₆-Alkylgruppe, welche unsubstituiert oder gegebenenfalls substituiert sein kann,
- ein C₃-C₁₀-Cycloalkylring, der unsubstituiert oder gegebenenfalls substituiert sein kann,
- eine C₂-C₆-Alkenyl-oder C₂-C₆-Alkinylgruppe, die unsubstituiert oder gegebenenfalls substituiert sein kann,
- ein 5-12-gliedriger, mono-ober bicyclischer Aryl- oder Heteroarylring, der unsubstituiert oder gegebenenfalls substituiert sein kann,
- R⁶, R⁷: ein Wasserstoff, ein C₁-C₆-Alkyl, ein C₃-C₁₀-Cycloalkylring, ein 5-12-gliedriger mono- oder bicyclischer Aryl- oder Heteroarylring, wobei die Alkyl-, Cycloalkyl- und (Het)arylgruppen unsubstituiert oder gegebenenfalls substituiert sein können, oder
- R⁶, R⁷: zusammen einen 3-8-gliedrigen Ring bilden, bedeuten, sowie deren Isomere, Salze und deren Cyclodextrinclathraten,
die bekannten Nachteile überwinden, und eine bessere Selektivität zum EP₂ Rezeptor und somit eine bessere Wirksamkeit und längere Wirkdauer erzielen können.

Bei dem unter R⁸ bis R¹⁰ angegebenen gesättigten, unverzweigten C₁-C₄-Alkylsubstituenten handelt es sich beispielsweise um eine Methyl-, Ethyl-, n-Propyl-, *n*-Butyl-, bei den verzweigten C₃-C₄-Alkylgruppen um eine *iso*-Propyl-, *iso*-Butyl-, sec-Butyl, *tert*-Butylgruppe.

Die Alkylgruppen können gegebenenfalls ein- bis mehrfach substituiert sein mit Halogenatomen, z.B. Fluor, Chlor oder Brom.

Bei dem unter R² bis R⁷ angegebenen gesättigten, unverzweigten C₁-C₆-Alkylsubstituenten handelt es sich beispielsweise um eine Methyl-, Ethyl-, *n-*Propyl-, *n*-Butyl-, *n*-Pentyl-, *n*-Hexyl-, bei den verzweigten C₃-C₆-Alkylgruppen um eine *iso*-Propyl-, *iso*-Butyl-, *sec*-Butyl, *tert*-Butyl-, *iso*-Pentyl-, *neo*-Pentyl-, 2-Methylpentyl-, 2,2-Dimethylbutyl-, 2,3-Dimethylbutylgruppe.

Die Alkylgruppen können gegebenenfalls ein- bis fünffach substituiert sein mit Halogenatomen (z.B. Fluor, Chlor oder Brom), sowie mit Cyano-, Hydroxy-, Amino-, Carboxylgruppen.

Die C₂-C₆-Alkenyl-Substituenten in R²-R⁵ bzw. die C₂-C₄-Alkenyl-Substituenten in R⁹-R¹⁰ sind jeweils geradkettig oder verzweigt, wobei beispielsweise folgenden Reste gemeint sind:

Vinyl-, Allyl-, Homoallyl-, (*E*)-But-2-enyl-, (*Z*)-But-2-enyl-, Pent-4-enyl-, (*E*)-Pent-3-enyl-, (*Z*)-Pent-3-enyl-, (*E*)-Pent-2-enyl-, (*Z*)-Pent-2-enyl-, 2-Methylvinyl-, 3-Methylbut-3-enyl-, 2-Methylbut-3-enyl-, (*E*)-2-Methylbut-2-enyl-, (*Z*)-2-Methylbut-2-enyl-, 2-Ethylprop-2-enyl-, Hex-5-enyl-, (*E*)-Hex-4-enyl-, (*Z*)-Hex-4-enyl-, (*E*)-Hex-3-enyl-, (*Z*)-Hex-3-enyl-, (*E*)-Hex-2-enyl-, (*Z*)-Hex-2-enyl-, 1-Methylpent-4-enyl-, (*E*)-1-Methylpent-3-enyl-, (*Z*)-1-Methylpent-3-enyl-, 1-Ethylbut-3-enyl-, (*E*)-1-Methylpent-2-enyl-, (*Z*)-1-Methylpent-2-enyl-).

Die Alkenylgruppen können gegebenenfalls ein- bis dreifach substituiert sein mit Halogenatomen (z.B. Fluor, Chlor oder Brom), sowie mit Cyano-, Carboxylgruppen.

Die C₂-C₆-Alkinyl-Substituenten in R²-R⁵ bzw. die C₂-C₄-Alkenyl-Substituenten in R⁹-R¹⁰ sind jeweils geradkettig oder verzweigt, wobei beispielsweise folgenden Reste gemeint sind: Ethinyl, Prop-1-in-1-yl, But-1-in-1-yl, But-2-in-1-yl.

Die Alkinylgruppen können gegebenenfalls einfach substituiert sein mit Halogenatomen (z.B. Fluor, Chlor oder Brom), sowie mit Cyano-, Carboxylgruppen.

Unter Halogen werden folgende verstanden: Fluor, Chlor, Brom, lod.

Unter den in R²-R⁷ angegebenen C₃-C₁₀-Cycloalkyl-Substituenten bzw. den in R^{9 -} R¹⁰ angegebenen C₃-C₆-Cycloalkyl-Substituenten sind Cycloalkylringe wie Cyclopropyl, Cyclobutyl, Cyclopentyl, Cyclohexyl oder Cycloheptyl, Cyclooctyl, Adamantyl oder Decalinyl zu verstehen.

Die Cycloalkylgruppen können gegebenenfalls ein- bis zweifach substituiert sein mit Halogenatomen (Fluor, Chlor oder Brom), sowie mit Cyano-, Hydroxy-, Amino-, Carboxylgruppen.

Unter dem in R¹ bis R⁷ angegebenen 5-12-gliedrigen, mono- oder bicyclischen Aryl- oder Heteroarylrest, der gegebenenfalls ein- oder dreifach substituiert sein kann, werden 5-12-gliedrige Ringsysteme verstanden, die anstelle des Kohlenstoffs ein- oder mehrere, gleiche oder verschiedene Heteroatome, wie Sauerstoff, Stickstoff oder Schwefel im Ring enthalten können, mono- oder bicyclisch sein können und zusätzlich jeweils benzokondensiert sein können und über eine der möglichen Verknüpfungsstellen mit dem Gerüst verbunden sind.

Beispielsweise seien für einen 5-12-gliedrigen, mono- oder bicyclischen Arylrest folgende genannt: Cyclopentadienyl, Phenyl, Tropyl, Cyclooctadienyl, Indenyl, Naphthyl, Azulenyl, Biphenyl.

Bei den 5-12-gliedrigen, mono- oder bicyclischen Heteroarylgruppen kann es sich um eine über eine der substituierbaren Stellen verknüpfte Pyridinyl-, Pyrimidinyl-, Chinolinyl-, Isochinolinyl-, Phthalazinyl-, Chinazolinyl-, Chinoxalinyl-, Cinnolinyl-, Benzofuranyl-, Benzothiophenyl-, 1,3-Benzodioxolyl-, Benzimidazolyl-, 2,1,3-Benzothiadiazolyl-, Indolyl-, Furanyl-, Thiophenyl-, Oxazolyl-, Isoxazolyl-, Thiazolyl-, Pyrrolyl-, Pyrazolyl-, Pyrazinyl-, Pyridazinyl-Triazolyl-, Tetrazolyl- oder eine Imidazolyl-Gruppe handeln.

Unter dem in R⁹ bis R¹⁰ angegebenen 5-6-gliedrigen Aryl- oder Heteroarylrest, der gegebenenfalls ein- oder dreifach substituiert sein kann, werden 5-6-gliedrige Ringsysteme verstanden, die anstelle des Kohlenstoffs ein- oder mehrere, gleiche oder verschiedene Heteroatome, wie Sauerstoff, Stickstoff oder Schwefel im Ring enthalten können und über eine der möglichen Verknüpfungsstellen mit dem Gerüst verbunden sind.

Beispielsweise seien für einen 5-6-gliedrigen, mono- oder bicyclischen Arylrest folgende genannt: Cyclopentadienyl, Phenyl.

Bei den 5-6-gliedrigen Heteroarylgruppen kann es sich um eine über eine der substituierbaren Stellen verknüpfte Pyridinyl-, Pyrimidinyl-, Furanyl-, Thiophenyl-, Oxazolyl-, Isoxazolyl-, Thiazolyl-, Pyrrolyl-, Pyrazolyl-, Pyrazinyl-, Pyridazinyl- oder eine lmidazolyl-Gruppe handeln.

Bei dem 3-8-gliedrigen Ring, der durch Ringschluss von R⁶ und R⁷ oder R⁹ und R¹⁰ gebildet werden kann, kann es sich um einen Cycloalkyl oder einen Stickstoff-haltigen Heterocyclus handeln. Als Beispiele für einen 3-8-gliedrigen Cycloalkylring seinen beispielsweise folgende genannt: Cyclopropyl, Cyclobutyl, Cyclopentyl, Cyclohexyl oder Cycloheptyl, Cyclooctyl.

Als Beispiel für einen 3-8-gliedrigen, Stickstoff-haltigen Heterocyclus seinen beispielsweise folgende genannt: Aziridinyl, Azetidinyl, Pyrrolidinyl, Piperidinyl, Piperazinyl, Morpholinyl, Azepanyl, [1, 4]-Diazepanyl.

Die freien Alkohole der erfindungsgemäßen Verbindungen können auch als Ester vorliegen und sind so Prodrugs der physiologischen Verbindungen der allgemeinen Formel I, die im Organismus zu Verbindungen der allgemeinen Formel I metabolisieren.

Geeignete Verbindungen sind beispielsweise bei Hans Bundgaard (Herausg.), Design of Prodrugs, Elsevier, Amsterdam 1985, aufgeführt.

Ist eine saure Funktion enthalten, sind als Salze die physiologisch verträglichen Salze organischer und anorganischer Basen geeignet, wie beispielsweise die gut löslichen Alkali- und Erdalkalisalze sowie N-Methyl-glukamin, Dimethylglukamin, Ethyl-glukamin, Lysin, 1,6-Hexadiamin, Ethanolamin, Glukosamin, Sarkosin, Serinol, Tris-hydroxy-methyl-amino-methan, Aminopropandiol, Sovak-Base, 1-Amino-2,3,4-butantriol.

Ist eine basische Funktion enthalten, kommen für die Bildung von physiologisch verträglichen Salzen der erfindungsgemäßen Verbindungen der allgemeinen Formel I, nach den dem Fachmann bekannten Methoden, als anorganische Säuren unter anderem Chlorwasserstoffsäure, Bromwasserstoffsäure, Schwefelsäure und Phosphorsäure, Salpetersäure, als Carbonsäuren unter anderem Essigsäure, Propionsäure, Hexansäure, Octansäure, Decansäure, Oleinsäure, Stearinsäure, Maleinsäure, Fumarsäure, Bernsteinsäure, Benzoesäure, Ascorbinsäure, Oxalsäure, Salicylsäure, Weinsäure, Zitronensäure, Milchsäure, Glykolsäure, Äpfelsäure, Mandelsäure, Zimtsäure, Glutaminsäure, Asparaginsäure, als Sulfonsäuren unter anderem Methansulfonsäure, Ethansulfonsäure, Toluolsulfonsäure, Benzolsulfonsäure sowie Naphthalinsulfonsäure in Betracht.

Bevorzugt sind die Verbindungen der allgemeinen Formel 1, wobei
- X: ein Stickstoffrest,
- Y, Z: ein Kohlenstoffrest -C-R⁸,
- R⁸: ein Wasserstoff, ein C₁-C₄-Alkylrest,
- R¹: ein 5-12-gliedriger, mono- oder bicyclischer, Aryl- oder Heteroarylring, der unsubstituiert und gegebenenfalls ein- bis dreifach substituiert sein kann,
- R²- R⁵: unabhängig voneinander ein Wasserstoff, Halogen, Cyano,
- oder eine OR⁶, OC(O)R⁶, S(O)ₙR⁶, wobei n = 0, 1, 2 bedeutet, SO₂NHR⁶, SO₂NHC(O)R⁶, NR⁶R⁷, NHC(O)R⁶, CH₂NR⁶R⁷, CH₂NHC(O)R⁶, C(OH)R⁶R⁷, C(O)R⁶, CO₂R⁶, C(O)NR⁶R⁷-Gruppe,
- eine C₁-C₆-Alkylgruppe, die unsubstituiert ist oder gegebenenfalls substituiert sein kann,
- ein C₃-C₁₀-Cycloalkylring, der unsubstituiert ist oder gegebenenfalls substituiert sein kann,
- eine C₂-C₆-Alkenyl- oder C₂-C₆-Alkinylgruppe, die unsubstituiert ist oder gegebenenfalls substituiert sein kann,
- ein 5-12-gliedriger mono- oder bicyclischer Aryl- oder Heteroarylring, der unsubstituiert ist oder gegebenenfalls substituiert sein kann,
- R⁶, R⁷: ein Wasserstoff, ein C₁-C₆-Alkyl, ein C₃-C₁₀-Cycloalkylring, ein 5-12-gliedriger mono- oder bicyclischer Aryl- oder Heteroarylring, wobei die Alkyl-, Cycloalkyl- und (Het)arylgruppen unsubstituiert sind oder gegebenenfalls substituiert sein können, oder
- R⁶, R⁷: zusammen einen 3-8-gliedrigen Ring bilden, bedeuten.

Ebenfalls bevorzugt sind die Verbindungen der allgemeinen Formel I, wobei
- Y und: Z ein Stickstoffrest,
- X: ein Kohlenstoffrest -C-R⁸,
- R⁸: ein Wasserstoff oder ein C₁-C₄-Alkylrest,
- R¹: ein 5-12-gliedriger mono oder bicyclischer Aryl- oder Heteroarylring, der unsubstituiert ist oder gegebenenfalls ein- bis dreifach substituiert sein kann,
- R²-R⁵: unabhängig voneinander ein Wasserstoff, Halogen, Cyano,
- oder eine OR⁶, OC(O)R⁶, S(O)ₙR⁶, wobei n = 0, 1, 2 bedeutet, SO₂NHR⁶, SO₂NHC(O)R⁶, NR⁶R⁷, NHC(O)R⁶, CH₂NR⁶R⁷, CH₂NHC(O)R⁶, C(OH)R⁶R⁷, C(O)R⁶, CO₂R⁶, C(O)NR⁶R⁷-Gruppe,
- eine C₁-C₆-Alkylgruppe, die unsubstituiert ist oder gegebenenfalls substituiert sein kann,
- ein C₃-C₁₀-Cycloalkylring, der unsubstituiert ist oder gegebenenfalls substituiert sein kann,
- eine C₂-C₆-Alkenyl-oder C₂-C₆-Alkinylgruppe, die unsubstituiert ist oder gegebenenfalls substituiert sein kann,
- ein 5-12-gliedriger mono- oder bicyclischer Aryl- oder Heteroarylring, der unsubstituiert ist oder gegebenenfalls substituiert sein kann,
- R⁶, R⁷: ein Wasserstoff, ein C₁-C₆-Alkyl, ein C₃-C₁₀-Cycloalkylring, ein 5-12-gliedriger mono- oder bicyclischer Aryl- oder Heteroarylring, wobei die Alkyl-, Cycloalkyl- und (Het)arylgruppen unsubstituiert sind oder gegebenenfalls substituiert sein können, oder
- R⁶, R⁷: zusammen einen 3-8-gliedrigen Ring bilden,
bedeuten.

Ebenfalls bevorzugt sind die Verbindungen der allgemeinen Formel I, wobei
- X und Z: jeweils ein Stickstoffrest,
- Y: ein Kohlenstoffrest -C-R⁸,
- R⁸: ein Wasserstoff, ein C₁-C₄-Alkylrest,
- R¹: ein 5-12-gliedriger mono- oder bicyclischer Aryl- oder Heteroarylring, der unsubstituiert ist und gegebenenfalls ein- bis dreifach substituiert sein kann,
- R² - R⁵: unabhängig voneinander ein Wasserstoff, Halogen, Cyano,
- oder eine OR⁶, OC(O)R⁶, S(O)ₙR⁶, wobei n = 0, 1, 2 bedeutet, SO₂NHR⁶, SO₂NHC(O)R⁶, NR⁶R⁷, NHC(O)R⁶, CH₂NR⁶R⁷, CH₂NHC(O)R⁶, C(OH)R⁶R⁷, C(O)R⁶, CO₂R⁶, C(O)NR⁶R⁷-Gruppe,
- eine C₁-C₆-Alkylgruppe, die unsubstituiert ist oder gegebenenfalls substituiert sein kann,
- ein C₃-C₁₀-Cycloalkylring, der unsubstituiert ist oder gegebenenfalls substituiert sein kann,
- eine C₂-C₆-Alkenyl-oder C₂-C₆-Alkinylgruppe, die unsubstituiert ist oder gegebenenfalls substituiert sein kann,
- ein 5-12-gliedriger mono- oder bicyclischer Aryl- oder Heteroarylring, der unsubstituiert ist oder gegebenenfalls substituiert sein kann,
- R⁶, R⁷: ein Wasserstoff, ein C₁-C₆-Alkyl, ein C₃-C₁₀-Cycloalkylring, ein 5-12-gliedriger, mono- oder bicyclischer Aryl- oder Heteroarylring, wobei die Alkyl-, Cycloalkyl- und (Het)arylgruppen unsubstituiert sind oder gegebenenfalls substituiert sein können, oder
- R⁶, R⁷: zusammen einen 3-8-gliedrigen Ring bilden,
bedeuten.

Ebenfalls bevorzugt sind die Verbindungen der allgemeinen Formel I, wobei
- X und Y: jeweils ein Kohlenstoffrest -C-R⁸,
- Z: ein Stickstoffrest,
- R⁸: ein Wasserstoff, ein C₁-C₄-Alkylrest,
- R¹: ein 5-12-gliedriger oder mono- oder bicyclischer Aryl- oder Heteroarylring, der unsubstituiert ist oder gegebenenfalls ein- bis dreifach substituiert sein kann,
- R² - R⁵: unabhängig voneinander ein Wasserstoff, Halogen, Cyano,
- oder eine OR⁶, OC(O)R⁶, S(O)ₙR⁶, wobei n = 0, 1, 2 bedeutet, SO₂NHR⁶, SO₂NHC(O)R⁶, NR⁶R⁷, NHC(O)R⁶, CH₂NR⁶R⁷, CH₂NHC(O)R⁶, C(OH)R⁶R⁷, C(O)R⁶, CO₂R⁶, C(O)NR⁶R⁷-Gruppe,
- eine C₁-C₆-Alkylgruppe, die unsubstituiert ist oder gegebenenfalls substituiert sein kann,
- ein C₃-C₁₀-Cycloalkylring, der unsubstituiert ist oder gegebenenfalls substituiert sein kann,
- eine C₂-C₆-Alkenyl-oder C₂-C₆-Alkinylgruppe, die unsubstituiert ist oder gegebenenfalls substituiert sein kann,
- ein 5-12-gliedriger mono- oder bicyclischer Aryl- oder Heteroarylring, der unsubstituiert ist oder gegebenenfalls substituiert sein kann,
- R⁶, R⁷: ein Wasserstoff, ein C₁-C₆-Alkyl, ein C₃-C₁₀-Cycloalkylring, ein 5-12-gliedriger, mono- oder bicyclischer Aryl- oder Heteroarylring, wobei die Alkyl-, Cycloalkyl- und (Het)arylgruppen unsubstituiert sind oder gegebenenfalls substituiert sein können, oder
- R⁶, R⁷: zusammen einen 3-8-gliedrigen Ring bilden,
bedeuten.

Ebenfalls bevorzugt sind die Verbindungen der allgemeinen Formel 1, wobei
- X: ein Stickstoffrest,
- Y, Z: ein Kohlenstoffrest -C-R⁸,
- R⁸: ein Wasserstoff,
- R¹: ein unsubstituierter 5-12-gliedriger mono- oder bicyclischer Aryl-oder Heteroarylring, der gegebenenfalls ein- bis dreifach substituiert ist,
wobei die Substituenten ausgewählt sein können aus der
Gruppe
Halogen,
-C₁-C₄-Alkyl, welches unsubstituiert ist und gegebenenfalls substituiert sein kann,
-OR⁶, -OC(O)R⁶, -S(O)ₙR⁶, wobei n = 0, 1, 2 bedeutet,
-SO₂NHR⁶, -SO₂NHC(O)R⁶, NR⁶R⁷, -NHC(O)R⁶, -CN, -CO₂-R⁶, -C(O)-N-R⁶R⁷, -C(O)R⁶, -C(OH)R⁶R⁷,
wobei es sich bei dem 5-12-gliedrigen mono- oder bicyclischen
Aryl- oder Heteroarylring beispielsweise, aber nicht ausschließlich, um eine Naphthyl-, Chinolinyl-, Isochinolinyl-, Phthalazinyl-, Chinazolinyl-, Chinoxalinyl-, Cinnolinyl-, Benzothiophenyl-, 1,3-Benzodioxolyl-, 2,1,3-Benzothiadiazolyl-, Phenyl-, Pyridinyl-, Pyrimidinyl-, Furanyl-, Thiophenyl-, Oxazolyl-, Isoxazolyl-, Thiazolyl-, Pyrrolyl-, Pyrazolyl-, Imidazolyl-, Pyrazinyl-, Pyridazinyl-, Triazolyl-, Tetrazolyl-, Indolyl-, Benzofuranyl- oder Benzimidazolyl-Gruppe handeln kann,
- R²: ein Wasserstoff,
- R³- R⁵: unabhängig voneinander ein Wasserstoff, Halogen, Cyano, oder eine OR⁶, OC(O)R⁶, S(O)ₙR⁶ wobei n = 0, 1, 2 bedeutet, SO₂NHR⁶, SO₂NHC(O)R⁶, NR⁶R⁷, NHC(O)R⁶, CH₂NR⁶R⁷, CH₂NHC(O)R⁶, C(OH)R⁶R⁷, C(O)R⁶, CO₂R⁶, C(O)NR⁶R⁷-Gruppe,
eine C₁-C₆-Alkylgruppe, welche gegebenenfalls substituiert oder unsubstituiert ist,
ein C₃-C₁₀-Cycloalkylring, der gegebenenfalls substituiert oder unsubstituiert ist,
eine C₂-C₆-Alkenyl-oder C₂-C₆-Alkinylgruppe, die gegebenenfalls substituiert oder unsubstituiert ist,
ein 5-12-gliedriger mono- oder bicyclischer Aryl- oder
Heteroarylring, der gegebenenfalls ein- oder mehrfach substituiert ist,
wobei die Substituenten ausgewählt sein können aus der Gruppe
- Halogen,
- -C₁-C₄-Alkyl, welches unsubstituiert ist und gegebenenfalls substituiert sein kann,
- -OR⁹, -OC(O)R⁹, -S(O)ₙR⁹, wobei n = 0, 1, 2 bedeutet, -SO₂NHR⁹, -SO₂NHC(O)R⁹, NR⁹R¹⁰,-NHC(O)R⁹, -CN, -CO₂-R⁹, -C(O)-N-R⁹R¹⁰, -C(O)R⁹,-C(OH)R⁹R¹⁰,
wobei es sich bei dem 5-12-gliedrigen mono oder bicyclischen Aryl- oder Heteroarylring beispielsweise, aber nicht ausschließlich, um eine Naphthyl-, Chinolinyl-, Isochinolinyl-, Phthalazinyl-, Chinazolinyl-, Chinoxalinyl-, Cinnolinyl-, Benzothiophenyl-, 1,3-Benzodioxolyl-, 2,1,3-Benzothiadiazolyl-, Phenyl-, Pyridinyl-, Pyrimidinyl-, Furanyl-, Thiophenyl-, Oxazolyl-, Isoxazolyl-, Thiazolyl-, Pyrrolyl-, Pyrazolyl-, Imidazolyl-, Pyrazinyl-, Pyridazinyl-, Triazolyl-, Tetrazolyl-, Indolyl-, Benzofuranyl- oder Benzimidazolyl-Gruppe handeln kann,
- R⁶, R⁷: ein Wasserstoff, eine C₁-C₆-Alkylgruppe, welche unsubstituiert ist oder gegebenenfalls bis zu 5fach halogeniert sein kann, ein C₃-C₈-Cycloalkylrest,
ein 5-12-gliedriger, mono-oder bicyclischer Aryl- oder
Heteroarylring, der gegebenenfalls ein- oder mehrfach substituiert ist,
wobei die Substituenten ausgewählt sein können aus der Gruppe
- Halogen,
- Cyano,
- R⁹, -OR⁹, -OC(O)R⁹, -S(O)ₙR⁹, wobei n = 0, 1, 2 bedeutet, -SO₂NHR⁹, NR⁹R¹⁰, -NHC(O)R⁹, -CO₂-R⁹, -C(O)-N-R⁹R¹⁰,
wobei es sich bei dem 5-12-gliedrigen mono- oder bicyclischen Aryl- oder Heteroarylring beispielsweise, aber nicht ausschließlich, um eine Naphthyl- Chinolinyl-, Isochinolinyl-, Phthalazinyl-, Chinazolinyl-, Chinoxalinyl-, Cinnolinyl-, Benzothiophenyl-, 1,3-Benzodioxolyl-, 2,1,3-Benzothiadiazolyl-, Phenyl-, Pyridinyl-, Pyrimidinyl-, Furanyl-, Thiophenyl-, Oxazolyl-, Isoxazolyl-, Thiazolyl-, Pyrrolyl-, Pyrazolyl-, Imidazolyl-, Pyrazinyl-, Pyridazinyl-, Triazolyl-, Tetrazolyl-, Indolyl-, Benzofuranyl- oder Benzimidazolyl Gruppe handeln kann, oder
- R⁶, R⁷: zusammen einen 3-8-gliedrigen Ring bilden,
- R⁹, R¹⁰: unabhängig voneinander Wasserstoff,
- eine C₁-C₄-Alkylgruppe, welche unsubstituiert ist oder gegebenenfalls bis zu fünffach fluoriert sein kann,
- eine C₂-C₄-Alkenylgruppe, welche unsubstituiert ist oder gegebenenfalls bis zu dreifach fluoriert sein kann,
- eine C₂-C₄-Alkinylgruppe, welche unsubstituiert ist oder gegebenenfalls einfach fluoriert sein kann,
- eine C₃-C₆-Cycloalkylgruppe,
- ein 5-6-gliedriger Aryl- oder Heteroarylring, wobei es sich dabei beispielsweise, aber nicht ausschließlich, um einen Phenyl-, Pyridinyl-, Pyrimidinyl-, Furanyl-, Thiophenyl-, Oxazolyl-, Isoxazolyl-, Thiazolyl-, Pyrrolyl-, Pyrazolyl-, Imidazolyl-, Pyrazinyl-, Pyridazinyl-, Triazolyl-, Tetrazolyl-Ring handeln kann, der unsubstituiert ist oder gegebenenfalls bis zu zweifach mit Fluor, Chlor, Trifluormethyl substituiert sein kann, oder
- R⁹, R¹⁰: zusammen einen 3-8-gliedrigen Ring bilden,
bedeuten.

Ebenfalls bevorzugt sind die Verbindungen der allgemeinen Formel I, wobei
- Y und Z: ein Stickstoffrest,
- X: ein Kohlenstoffrest -C-R⁸,
- R⁸: ein Wasserstoff oder eine Methylgruppe,
- R¹: ein 5-12-gliedriger mono- oder bicyclischer Aryl- oder Heteroarylring, der gegebenenfalls ein- bis dreifach substituiert ist,
wobei die Substituenten ausgewählt sein können aus der Gruppe Halogen,
-C₁-C₄-Alkyl, welches unsubstituiert ist und gegebenenfalls substituiert sein kann,
-OR⁶, -OC(O)R⁶, -S(O)ₙR⁶, wobei n = 0, 1, 2 bedeutet,-SO₂NHR⁶, -SO₂NHC(O)R⁶, NR⁶R⁷, -NHC(O)R⁶, -CN, -CO₂-R⁶, -C(O)-N-R⁶R⁷, -C(O)R⁶, -C(OH)R⁶R⁷,
wobei es sich bei dem 5-12-gliedrigen mono- oder bicyclischen Aryl- oder Heteroarylring beispielsweise, aber nicht ausschließlich, um eine Naphthyl-, Chinolinyl-, Isochinolinyl-, Phthalazinyl-, Chinazolinyl-, Chinoxalinyl-, Cinnolinyl-, Benzothiophenyl-, 1,3-Benzodioxolyl-, 2,1,3-Benzothiadiazolyl-, Phenyl-, Pyridinyl-, Pyrimidinyl-, Furanyl-, Thiophenyl-, Oxazolyl-, Isoxazolyl-, Thiazolyl-, Pyrrolyl-, Pyrazolyl-, Imidazolyl-, Pyrazinyl-, Pyridazinyl-, Triazolyl-, Tetrazolyl-, Indolyl-, Benzofuranyl-, oder Benzimidazolyl-Gruppe handeln kann,
- R²: ein Wasserstoff,
- R³- R⁵: unabhängig voneinander ein Wasserstoff, Halogen, Cyano,
- oder eine OR⁶, OC(O)R⁶, S(O)ₙR⁶, wobei n = 0,1, 2 bedeutet, SO₂NHR⁶, SO₂NHC(O)R⁶, NR⁶R⁷, NHC(O)R⁶, CH₂NR⁶R⁷, CH₂NHC(O)R⁶, C(OH)R⁶R⁷, C(O)R⁶, CO₂R⁶, C(O)NR⁶R⁷ Gruppe,
- eine C₁-C₆-Alkylgruppe, die unsubstituiert ist oder gegebenenfalls substituiert sein kann,
- ein C₃-C₁₀-Cycloalkylring, der unsubstituiert ist oder gegebenenfalls substituiert sein kann,
- eine C₂-C₆-Alkenyl-oder C₂-C₆-Alkinylgruppe, die unsubstituiert ist oder gegebenenfalls substituiert sein kann,
- ein unsubstituierter 5-12-gliedriger mono- oder bicyclischer Aryl- oder Heteroarylring, der gegebenenfalls ein- oder mehrfach substituiert sein kann,
   wobei die Substituenten ausgewählt sein können aus der
   Gruppe
   Halogen,
   -C₁-C₄-Alkyl, welches unsubstituiert ist und gegebenenfalls substituiert sein kann,
   -OR⁹, -OC(O)R⁹, -S(O)ₙR⁹ wobei n = 0, 1, 2 bedeutet,
   -SO₂NHR⁹, -SO₂NHC(O)R⁹, NR⁹R¹⁰, -NHC(O)R⁹, -CN,-CO₂-R⁹, -C(O)-N-R⁹R¹⁰, -C(O)R⁹, -C(OH)R⁹R¹⁰,
   wobei es sich bei dem 5-12-gliedrigen mono- oder bicyclischen Aryl- oder Heteroarylring beispielsweise, aber nicht ausschließlich, um eine Naphthyl-, Chinolinyl-, lsochinolinyl-, Phthalazinyl-, Chinazolinyl-, Chinoxalinyl-, Cinnolinyl-, Benzothiophenyl-, 1,3-Benzodioxolyl-, 2,1,3-Benzothiadiazolyl-, Phenyl-, Pyridinyl-, Pyrimidinyl-, Furanyl-, Thiophenyl-, Oxazolyl-, lsoxazolyl-, Thiazolyl-, Pyrrolyl-, Pyrazolyl-, Imidazolyl-, Pyrazinyl-, Pyridazinyl-, Triazolyl-, Tetrazolyl-, Indolyl-, Benzofuranyl- oder Benzimidazolyl-Gruppe handeln kann,
- R⁶, R⁷: ein Wasserstoff, eine unsubstituierte C₁-C₆-Alkylgruppe, die gegebenenfalls bis zu fünffach halogeniert sein kann, ein C₃-C₈-Cycloalkylrest,
ein unsubstituierter 5-12-gliedriger, mono-oder bicyclischer Aryl-oder Heteroarylring, der gegebenenfalls ein- oder mehrfach substituiert sein kann, wobei die Substituenten ausgewählt sein können aus der Gruppe
- Halogen,
- Cyano,
- R⁹, -OR⁹, -OC(O)R⁹, -S(O)ₙR⁹, wobei n = 0, 1, 2 bedeutet, -SO₂NHR⁹, NR⁹R¹⁰, -NHC(O)R⁹, -CO₂-R⁹, -C(O)-N-R⁹R¹⁰,
wobei es sich bei dem 5-12-gliedrigen mono oder bicyclischen Aryl- oder Heteroarylring beispielsweise, aber nicht ausschließlich, um eine Naphthyl-, Chinolinyl-, Isochinolinyl-, Phthalazinyl-, Chinazolinyl-, Chinoxalinyl-, Cinnolinyl-, Benzothiophenyl-, 1,3-Benzodioxolyl-, 2,1,3-Benzothiadiazolyl-, Phenyl-, Pyridinyl-, Pyrimidinyl-, Furanyl-, Thiophenyl-, Oxazolyl-, Isoxazolyl-, Thiazolyl-, Pyrrolyl-, Pyrazolyl-, Imidazolyl-, Pyrazinyl-, Pyridazinyl-, Triazolyl-, Tetrazolyl-, Indolyl-, Benzofuranyl- oder Benzimidazolyl-Gruppe handeln kann, oder
- R⁶, R⁷: zusammen einen 3-8-gliedrigen Ring bilden,
- R⁹, R¹⁰: unabhängig voneinander Wasserstoff,
- eine C₁-C₄-Alkylgruppe, welche unsubstituiert ist oder gegebenenfalls bis zu fünffach fluoriert sein kann,
- eine C₂-C₄-Alkenylgruppe, welche unsubstituiert ist oder gegebenenfalls bis zu dreifach fluoriert sein kann,
- eine C₂-C₄-Alkinylgruppe, welche unsubstituiert ist oder gegebenenfalls einfach fluoriert sein kann,
- eine C₃-C₆-Cycloalkylgruppe,
- ein 5-6gliedriger Aryl- oder Heteroarylring, wobei es sich dabei beispielsweise, aber nicht ausschließlich, um einen Phenyl-, Pyridinyl-, Pyrimidinyl-, Furanyl-, Thiophenyl-, Oxazolyl-, Isoxazolyl-, Thiazolyl-, Pyrrolyl-, Pyrazolyl-, Imidazolyl-, Pyrazinyl-, Pyridazinyl-, Triazolyl-, Tetrazolyl-Ring handeln kann, der unsubstituiert ist oder gegebenenfalls bis zu zweifach mit Fluor, Chlor, Trifluormethyl substituiert sein kann, oder
- R⁹, R¹⁰: zusammen einen 3-8-gliedrigen Ring bilden,
bedeuten.

Ebenfalls bevorzugt sind die Verbindungen der allgemeinen Formel 1, wobei
- X und Z: jeweils ein Stickstoffrest,
- Y: ein Kohlenstoffrest -C-R⁸,
- R⁸: ein Wasserstoff,
- R¹: ein unsubstituierter 5-12-gliedriger mono oder bicyclischer Aryl-oder Heteroarylring, der gegebenenfalls ein- bis dreifach substituiert ist,
wobei die Substituenten ausgewählt sein können aus der Gruppe Halogen,
-C₁-C₄-Alkyl, welches unsubstituiert ist und gegebenenfalls substituiert sein kann,
-OR⁶, -OC(O)R⁶, -S(O)ₙR⁶, wobei n = 0, 1, 2 bedeutet,-SO₂NHR⁶, -SO₂NHC(O)R⁶, NR⁶R⁷, -NHC(O)R⁶, -CN, -CO₂-R⁶, -C(O)-N-R⁶R⁷, -C(O)R⁶, -C(OH)R⁶R⁷,
wobei es sich bei dem 5-12-gliedrigen mono oder bicyclischen Aryl- oder Heteroarylring beispielsweise, aber nicht ausschließlich, um eine Naphthyl-, Chinolinyl-, Isochinolinyl-, Phthalazinyl-, Chinazolinyl-, Chinoxalinyl-, Cinnolinyl-, Benzothiophenyl-, 1,3-Benzodioxolyl-, 2,1,3-Benzothiadiazolyl-, Phenyl-, Pyridinyl-, Pyrimidinyl-, Furanyl-, Thiophenyl-, Oxazolyl-, Isoxazolyl-, Thiazolyl-, Pyrrolyl-, Pyrazolyl-, Imidazolyl-, Pyrazinyl-, Pyridazinyl-, Triazolyl-, Tetrazolyl-, Indolyl-, Benzofuranyl- oder Benzimidazolyl-Gruppe handeln kann,
- R²: ein Wasserstoff,
- R³- R⁵: unabhängig voneinander ein Wasserstoff, Halogen, Cyano,
- oder eine OR⁶, OC(O)R⁶, S(O)ₙR⁶, wobei n = 0, 1, 2 bedeutet, SO₂NHR⁶, SO₂NHC(O)R⁶, NR⁶R⁷, NHC(O)R⁶, CH₂NR⁶R⁷, CH₂NHC(O)R⁶, C(OH)R⁶R⁷, C(O)R⁶, CO₂R⁶, C(O)NR⁶R⁷ Gruppe,
- eine unsubstituierte C₁-C₆-Alkylgruppe, die substituiert sein kann,
- ein unsubstituierter C₃-C₁₀-Cycloalkylring, der gegebenenfalls substituiert sein kann,
- eine unsubstituierte C₂-C₆-Alkenyl-oder C₂-C₆-Alkinylgruppe, die gegebenenfalls substituiert sein kann,
- ein unsubstituierter 5-12gliedriger, mono- oder bicyclischer Aryl- oder Heteroarylring, der gegebenenfalls ein-oder mehrfach substituiert sein kann,
wobei die Substituenten ausgewählt sein können aus der Gruppe Halogen, -C₁-C₄-Alkyl, welches unsubstituiert ist und gegebenenfalls substituiert sein kann,
-OR⁹, -OC(O)R⁹, -S(O)ₙR⁹, wobei =0,1,2 bedeutet,-SO₂NHR⁹, -SO₂NHC(O)R⁹, NR⁹R¹⁰, -NHC(O)R⁹, -CN, -CO₂-R⁹, -C(O)-N-R⁹R¹⁰, -C(O)R⁹, -C(OH)R⁹R¹⁰,
wobei es sich bei dem 5-12-gliedrigen mono- oder bicyclischen Aryl- oder Heteroarylring beispielsweise, aber nicht ausschließlich, um eine Naphthyl-, Chinolinyl-, Isochinolinyl-, Phthalazinyl-, Chinazolinyl-, Chinoxalinyl-, Cinnolinyl-, Benzothiophenyl-, 1,3-Benzodioxolyl-, 2,1,3-Benzothiadiazolyl-, Phenyl-, Pyridinyl-, Pyrimidinyl-, Furanyl-, Thiophenyl-, Oxazolyl-, lsoxazolyl-, Thiazolyl-, Pyrrolyl-, Pyrazolyl-, Imidazolyl-, Pyrazinyl-, Pyridazinyl-, Triazolyl-, Tetrazolyl-, Indolyl-, Benzofuranyl- oder Benzimidazolyl-Gruppe handeln,
- R⁶, R⁷: ein Wasserstoff, eine C₁-C₆-Alkylgruppe, die unsubstituiert oder gegebenenfalls bis zu fünffach halogeniert sein kann, ein unsubstituierter C₃-C₈-Cycloalkylrest,
ein unsubstituierter 5-12-gliedriger mono- oder bicyclischer Aryl-oder Heteroarylring, der gegebenenfalls ein- oder mehrfach substituiert sein kann, wobei die Substituenten ausgewählt sein können aus der Gruppe
- Halogen,
- Cyano,
- R⁹, -OR⁹, -OC(O)R⁹, -S(O)ₙR⁹, wobei n = 0, 1, 2 bedeutet, -SO₂NHR⁹, NR⁹R¹⁰, -NHC(O)R⁹, -CO₂-R⁹, -C(O)-N-R⁹R¹⁰,
wobei es sich bei dem 5-12-gliedrigen mono- oder bicyclischen Aryl- oder Heteroarylring beispielsweise, aber nicht ausschließlich, um eine Naphthyl-, Chinolinyl-, Isochinolinyl-, Phthalazinyl-, Chinazolinyl-, Chinoxalinyl-, Cinnolinyl-, Benzothiophenyl-, 1,3-Benzodioxolyl-, 2,1,3-Benzothiadiazolyl-, Phenyl-, Pyridinyl-, Pyrimidinyl-, Furanyl-, Thiophenyl-, Oxazolyl-, Isoxazolyl-, Thiazolyl-, Pyrrolyl-, Pyrazolyl-, Imidazolyl-, Pyrazinyl-, Pyridazinyl-, Triazolyl-, Tetrazolyl-, Indolyl-, Benzofuranyl- oder Benzimidazolyl-Gruppe handeln kann, oder
- R⁶, R⁷: zusammen einen 3-8-gliedrigen Ring bilden,
- R⁹, R¹⁰: unabhängig voneinander Wasserstoff,
- eine unsubstituierte C₁-C₄-Alkylgruppe, welche gegebenenfalls bis zu fünffach fluoriert sein kann,
- eine unsubstituierte C₂-C₄-Alkenylgruppe, welche gegebenenfalls bis zu dreifach fluoriert sein kann,
- eine unsubstituierte C₂-C₄-Alkinylgruppe, welche gegebenenfalls einfach fluoriert sein kann,
- eine unsubstituierte C₃-C₆-Cycloalkylgruppe,
- ein unsubstituierter 5-6gliedriger Aryl- oder Heteroarylring, wobei es sich dabei beispielsweise, aber nicht ausschließlich, um einen Phenyl-, Pyridinyl-, Pyrimidinyl-, Furanyl-, Thiophenyl-, Oxazolyl-, Isoxazolyl-, Thiazolyl-, Pyrrolyl-, Pyrazolyl-, Imidazolyl-, Pyrazinyl-, Pyridazinyl-, Triazolyl-, Tetrazolyl-Ring handeln kann, der gegebenenfalls bis zu zweifach mit Fluor, Chlor, Trifluormethyl substituiert sein kann, oder
- R⁹, R¹⁰: zusammen einen 3-8-gliedrigen Ring bilden,
bedeuten.

Ebenfalls bevorzugt sind die Verbindungen der allgemeinen Formel I, wobei
- X und Y: jeweils ein Kohlenstoffrest -C-R⁸,
- Z: ein Stickstoffrest,
- R⁸: eine Wasserstoff- oder Methylgruppe,
- R¹: ein unsubstituierter 5-12-gliedriger, mono- oder bicyclischer Aryl-oder Heteroarylring, der gegebenenfalls ein- bis dreifach substituiert ist,
wobei die Substituenten ausgewählt sein können aus der Gruppe Halogen, -C₁-C₄-Alkyl, das unsubstituiert ist oder gegebenenfalls substituiert sein kann,
-OR⁶, -OC(O)R⁶, -S(O)ₙR⁶, wobei n = 0, 1, 2 bedeutet,-SO₂NHR⁶, -SO₂NHC(O)R⁶, NR⁶R⁷, -NHC(O)R⁶, -CN, -CO₂-R⁶, -C(O)-N-R⁶R⁷, -C(O)R⁶, -C(OH)R⁶R⁷,
wobei es sich bei dem 5-12-gliedrigen mono- oder bicyclischen Aryl- oder Heteroarylring beispielsweise, aber nicht ausschließlich, um eine Naphthyl-, Chinolinyl-, Isochinolinyl-, Phthalazinyl-, Chinazolinyl-, Chinoxalinyl-, Cinnolinyl-, Benzothiophenyl-, 1,3-Benzodioxolyl-, 2,1,3-Benzothiadiazolyl-, Phenyl-, Pyridinyl-, Pyrimidinyl-, Furanyl-, Thiophenyl-, Oxazolyl-, Isoxazolyl-, Thiazolyl-, Pyrrolyl-, Pyrazolyl-, Imidazolyl-, Pyrazinyl-, Pyridazinyl-, Triazolyl-, Tetrazolyl-, Indolyl-, Benzofuranyl- oder Benzimidazolyl-Gruppe handeln,
- R²: ein Wasserstoff,
- R³ - R⁵: unabhängig voneinander ein Wasserstoff, Halogen, Cyano, oder
- eine OR⁶, OC(O)R⁶, S(O)ₙR⁶, wobei n = 0, 1, 2, bedeutet, SO₂NHR⁶, SO₂NHC(O)R⁶, NR⁶R⁷, NHC(O)R⁶, CH₂NR⁶R⁷, CH₂NHC(O)R⁶, C(OH)R⁶R⁷, C(O)R⁶, CO₂R⁶, C(O)NR⁶R⁷ Gruppe,
- eine unsubstituierte C₁-C₆-Alkylgruppe, die gegebenenfalls substituiert sein kann,
- ein unsubstituierter C₃-C₁₀-Cycloalkylring, gegebenenfalls substituiert sein kann,
- eine unsubstituierte C₂-C₆-Alkenyl-oder C₂-C₆-Alkinylgruppe, die gegebenenfalls substituiert sein kann,
- ein unsubstituierter 5-12gliedriger mono- oder bicyclischer Aryl- oder Heteroarylring, der gegebenenfalls ein- oder mehrfach substituiert ist,
wobei die Substituenten ausgewählt sein können aus der Gruppe Halogen, -C₁-C₄-Alkyl, das unsubstituiert ist oder gegebenenfalls substituiert sein kann,
-OR⁹, -OC(O)R⁹, -S(O)ₙR⁹ wobei n = 0, 1, 2 bedeutet, -SO₂NHR⁹, -SO₂NHC(O)R⁹, NR⁹R¹⁰, -NHC(O)R⁹, -CN,-CO₂-R⁹, -C(O)-N-R⁹R¹⁰, -C(O)R⁹, -C(OH)R⁹R¹⁰,
wobei es sich bei dem 5-12-gliedrigen mono- oder bicyclischen Aryl- oder Heteroarylring beispielsweise, aber nicht ausschließlich, um eine Naphthyl-, Chinolinyl-, Isochinolinyl-, Phthalazinyl-, Chinazolinyl-, Chinoxalinyl-, Cinnolinyl-, Benzothiophenyl-, 1,3-Benzodioxolyl-, 2,1,3-Benzothiadiazolyl-, Phenyl-, Pyridinyl-, Pyrimidinyl-, Furanyl-, Thiophenyl-, Oxazolyl-, Isoxazolyl-, Thiazolyl-, Pyrrolyl-, Pyrazolyl-, Imidazolyl-, Pyrazinyl-, Pyridazinyl-, Triazolyl-, Tetrazolyl-, Indolyl-, Benzofuranyl- oder Benzimidazolyl-Gruppe handeln kann,
- R⁶, R⁷: ein Wasserstoff, eine C₁-C₆-Alkylgruppe, die unsubstituiert ist oder gegebenenfalls bis zu fünffach halogeniert sein kann, ein unsubstituierter C₃-C₈-Cycloalkylrest, ein unsubstituierter 5-12-gliedriger, mono- oder bicyclischer Aryl-oder Heteroarylring, der gegebenenfalls ein- oder mehrfach substituiert sein kann, wobei die Substituenten ausgewählt sein können aus der Gruppe
Halogen,
Cyano,
R⁹, -OR⁹, -OC(O)R⁹, -S(O)ₙR⁹ wobei n = 0, 1, 2,
bedeutet, -SO₂NHR⁹, -SO₂NHC(O)R⁹, NR⁹R¹⁰,-NHC(O)R⁹, -CO₂-R⁹, -C(O)-N-R⁹R¹⁰,
wobei es sich bei dem 5-12-gliedrigen mono- oder bicyclischen Aryl- oder Heteroarylring beispielsweise, aber nicht ausschließlich, um eine Naphthyl-, Chinolinyl-, Isochinolinyl-, Phthalazinyl-, Chinazolinyl-, Chinoxalinyl-, Cinnolinyl-, Benzothiophenyl-, 1,3-Benzodioxolyl-, 2,1,3-Benzothiadiazolyl-, Phenyl-, Pyridinyl-, Pyrimidinyl-, Furanyl-, Thiophenyl-, Oxazolyl-, Isoxazolyl-, Thiazolyl-, Pyrrolyl-, Pyrazolyl-, Imidazolyl-, Pyrazinyl-, Pyridazinyl-, Triazolyl-, Tetrazolyl-, Indolyl-, Benzofuranyl- oder Benzimidazolyl-Gruppe handeln kann, oder
- R⁶, R⁷: zusammen einen 3-8-gliedrigen Ring bilden,
- R⁹, R¹⁰: unabhängig voneinander Wasserstoff,
- eine unsubstituierte C₁-C₄-Alkylgruppe, die gegebenenfalls bis zu fünffach fluoriert sein kann,
- eine unsubstituierte C₂-C₄-Alkenylgruppe, welche gegebenenfalls bis zu dreifach fluoriert sein kann,
- eine unsubstituierte C₂-C₄-Alkinylgruppe, welche gegebenenfalls einfach fluoriert sein kann,
- eine unsubstituierte C₃-C₆-Cycloalkylgruppe,
- ein unsubstituierter 5-6 gliedriger Aryl- oder Heteroarylring, wobei es sich dabei beispielsweise, aber nicht ausschließlich, um einen Phenyl-, Pyridinyl-, Pyrimidinyl-, Furanyl-, Thiophenyl-, Oxazolyl-, Isoxazolyl-, Thiazolyl-, Pyrrolyl-, Pyrazolyl-, Imidazolyl-, Pyrazinyl-, Pyridazinyl-, Triazolyl-, Tetrazolyl-Ring handeln kann, der gegebenenfalls bis zu zweifach mit Fluor, Chlor, Trifluormethyl substituiert sein kann, oder
- R⁹, R¹⁰: zusammen einen 3-8-gliedrigen Ring bilden,
bedeuten.

Ebenfalls bevorzugt sind die Verbindungen der allgemeinen Formel I, wobei
- X: ein Stickstoffrest,
- Y und Z: ein Kohlenstoffrest -C-R⁸,
- R⁸: ein Wasserstoff,
- R¹: ein unsubstituierter 5-6-gliedriger Aryl- oder Heteroarylring, der gegebenenfalls ein- bis dreifach substituiert ist,
wobei die Substituenten ausgewählt sein können aus der Gruppe Halogen, -C₁-C₄-Alkyl, das unsubstituiert ist oder gegebenenfalls substituiert sein kann, -OR⁶, -OC(O)R⁶, -S(O)ₙR⁶, wobei n = 0, 1, 2 bedeutet, -SO₂NHR⁶, -SO₂NHC(O)R⁶, NR⁶R⁷, -NHC(O)R⁶, -CN, -CO₂-R⁶, -C(O)-N-R⁶R⁷, -C(O)R⁶, -C(OH)R⁶R⁷,
wobei es sich bei dem 5-6-gliedrigen Aryl- oder Heteroarylring beispielsweise, aber nicht ausschließlich, um eine Phenyl-, Pyridinyl-, Pyrimidinyl-, Furanyl-, Thiophenyl-, Oxazolyl-, Isoxazolyl-, Thiazolyl-, Pyrrolyl-, Pyrazolyl-, Imidazolyl-, Pyrazinyl-, Pyridazinyl-,
Triazolyl-, Tetrazolyl- Gruppe handeln kann,
- R²: ein Wasserstoffrest,
- R³ - R⁵: unabhängig voneinander ein Wasserstoff, Halogen, Cyano, oder
- eine OR⁶, OC(O)R⁶, S(O)ₙR⁶, wobei n = 0, 1, 2, bedeutet, SO₂NHR⁶, SO₂NHC(O)R⁶, NR⁶R⁷, NHC(O)R⁶, CH₂NR⁶R⁷, CH₂NHC(O)R⁶, C(OH)R⁶R⁷, C(O)R⁶, CO₂R⁶, C(O)NR⁶R⁷-Gruppe,
- eine unsubstituierte C₁-C₆-Alkylgruppe, die gegebenenfalls substituiert sein kann,
- ein unsubstituierter C₃-C₁₀-Cycloalkylring, der gegebenenfalls substituiert sein kann,
- eine unsubstituierte C₂-C₆-Alkenyl-oder C₂-C₆-Alkinylgruppe, die gegebenenfalls substituiert sein kann,
- ein unsubstituierter 5-6-gliedriger Aryl- oder Heteroarylring, der gegebenenfalls ein-oder mehrfach substituiert ist,
wobei die Substituenten ausgewählt sein können aus der Gruppe
Halogen,
-C₁-C₄-Alkyl, das unsubstituiert ist und gegebenenfalls auch halogeniert oder aber mit -OH,
-CN, -CO₂H substituiert sein kann,
-OR⁹, -OC(O)R⁹, -S(O)ₙR⁹, wobei n = 0, 1, 2 bedeutet, -SO₂NHR⁹, -SO₂NHC(O)R⁹, NR⁹R¹⁰,-NHC(O)R⁹, -CN, -CO₂-R⁹, -C(O)-N-R⁹R¹⁰, -C(O)R⁹,-C(OH)R⁹R¹⁰,
wobei es sich bei dem 5-6-gliedrigen Aryl- oder
Heteroarylring beispielsweise, aber nicht ausschließlich, um eine Phenyl-, Pyridinyl-, Pyrimidinyl-, Furanyl-, Thiophenyl-, Oxazolyl-, Isoxazolyl-, Thiazolyl-, Pyrrolyl-, Pyrazolyl-, Imidazolyl-, Pyrazinyl-, Pyridazinyl-,Triazolyl-, Tetrazolyl-, Gruppe handeln kann,
- R⁶, R⁷: ein Wasserstoff, eine unsubstituierte C₁-C₆-Alkylgruppe, die gegebenenfalls bis zu fünffach halogeniert sein kann, ein unsubstituierter C₃-C₈-Cycloalkylrest, ein unsubstituierter 5-6-gliedriger Aryl- oder Heteroarylring, der gegebenenfalls ein- oder mehrfach substituiert sein kann, wobei die Substituenten ausgewählt sein können aus der Gruppe
Halogen, Cyano, -R⁹ -OR⁹, -OC(O)R⁹, -S(O)ₙR⁹ wobei n = 0, 1, 2 bedeutet, -SO₂NHR⁹, NR⁹R¹⁰, -NHC(O)R⁹, -CO₂-R⁹, -C(O)-N-R⁹R¹⁰,
wobei es sich bei dem 5-6-gliedrigen Aryl- oder Heteroarylring beispielsweise, aber nicht ausschließlich, um eine Phenyl-, Pyridinyl-, Pyrimidinyl-, Furanyl-, Thiophenyl-, Oxazolyl-, Isoxazolyl-, Thiazolyl-, Pyrrolyl-, Pyrazolyl-, Imidazolyl-, Pyrazinyl-, Pyridazinyl-Triazolyl-, Tetrazolyl-Gruppe handeln kann, oder
- R⁶, R⁷: zusammen einen 3-8-gliedrigen Ring bilden,
- R⁹, R¹⁰: unabhängig voneinander Wasserstoff, eine C₁-C₄-Alkylgruppe, welche unsubstituiert ist oder gegebenenfalls bis zu fünffach fluoriert sein kann,
eine unsubstituierte C₂-C₄-Alkenylgruppe, welche gegebenenfalls bis zu dreifach fluoriert sein kann,
eine unsubstituierte C₂-C₄-Alkinylgruppe, welche gegebenenfalls einfach fluoriert sein kann,
eine unsubstituierte C₃-C₆-Cycloalkylgruppe,
ein unsubstituierter 5-6-gliedriger Aryl- oder Heteroarylring, wobei es sich dabei beispielsweise, aber nicht ausschließlich, um einen Phenyl-, Pyridinyl-, Pyrimidinyl-, Furanyl-, Thiophenyl-, Oxazolyl-, Isoxazolyl-, Thiazolyl-, Pyrrolyl-, Pyrazolyl-, Imidazolyl-, Pyrazinyl-, Pyridazinyl-;Triazolyl-, Tetrazolyl-Ring handeln kann, der gegebenenfalls bis zu zweifach mit Fluor, Chlor, Trifluormethyl substituiert sein kann, oder
- R⁹, R¹⁰: zusammen einen 3-8-gliedrigen Ring bilden,
bedeuten.

Ebenfalls bevorzugt sind die Verbindungen der allgemeinen Formel 1, wobei
- Y und Z: ein Stickstoffrest,
- X: ein Kohlenstoffrest -C-R⁸,
- R⁸: ein Wasserstoff,
- R¹: ein 5-6-gliedriger Aryl- oder Heteroarylring, der gegebenenfalls ein- bis dreifach substituiert ist,
wobei die Substituenten ausgewählt sein können aus der Gruppe Halogen, -C₁-C₄-Alkyl, das unsubstituiert ist oder gegebenenfalls substituiert sein kann,
-OR⁶, -OC(O)R⁶, -S(O)ₙR⁶, wobei n = 0, 1, 2 bedeutet, -SO₂NHR⁶, -SO₂NHC(O)R⁶, NR⁶R⁷, -NHC(O)R⁶, -CN, -CO₂-R⁶, -C(O)-N-R⁶R⁷, -C(O)R⁶, -C(OH)R⁶R⁷,
wobei es sich bei dem 5-6-gliedrigen Aryl- oder Heteroarylring beispielsweise, aber nicht ausschließlich, um eine Phenyl-, Pyridinyl-, Pyrimidinyl-, Furanyl-, Thiophenyl-, Oxazolyl-, Isoxazolyl-, Thiazolyl-, Pyrrolyl-, Pyrazolyl-, Imidazolyl-, Pyrazinyl-, Pyridazinyl-,
Triazolyl-, Tetrazolyl- Gruppe handeln kann,
- R²: ein Wasserstoffrest,
- R³ - R⁵: unabhängig voneinander ein Wasserstoff, Halogen, Cyano, oder
- eine OR⁶, OC(O)R⁶, S(O)ₙR⁶, wobei n = 0, 1, 2, bedeutet, SO₂NHR⁶, SO₂NHC(O)R⁶, NR⁶R⁷, NHC(O)R⁶, CH₂NR⁶R⁷, CH₂NHC(O)R⁶, C(OH)R⁶R⁷, C(O)R⁶, CO₂R⁶, C(O)NR⁶R⁷ Gruppe,
- eine unsubstituierte C₁-C₆-Alkylgruppe, die gegebenenfalls substituiert sein kann,
- ein unsubstituierter C₃-C₁₀-Cycloalkylring, der gegebenenfalls substituiert sein kann,
- eine unsubstituierte C₂-C₆-Alkenyl-oder C₂-C₆-Alkinylgruppe, die gegebenenfalls substituiert sein kann,
- ein unsubstituierter 5-6gliedriger Aryl- oder Heteroarylring, der gegebenenfalls ein- oder mehrfach substituiert ist,
wobei die Substituenten ausgewählt sein können aus der
Gruppe
Halogen,
-C₁-C₄-Alkyl, das unsubstituiert ist und gegebenenfalls halogeniert oder aber mit -OH, -CN, -CO₂H substituiert sein kann,
-OR⁹, -OC(O)R⁹, -S(O)ₙR⁹ wobei n = 0, 1, 2 bedeutet,-SO₂NHR⁹, -SO₂NHC(O)R⁹, NR⁹R¹⁰, -NHC(O)R⁹, -CN,-CO₂-R⁹, -C(O)-N-R⁹R¹⁰, -C(O)R⁹, -C(OH)R⁹R¹⁰,
wobei es sich bei dem 5-6-gliedrigen Aryl- oder
Heteroarylring beispielsweise, aber nicht ausschließlich, um eine Phenyl-, Pyridinyl-, Pyrimidinyl-, Furanyl-, Thiophenyl-, Oxazolyl-, Isoxazolyl-, Thiazolyl-, Pyrrolyl-, Pyrazolyl-, Imidazolyl-, Pyrazinyl-, Pyridazinyl-,Triazolyl-, Tetrazolyl-, Gruppe handeln kann,
- R⁶, R⁷: ein Wasserstoff,
eine unsubstituierte C₁-C₆-Alkylgruppe, die gegebenenfalls bis zu fünffach halogeniert sein kann,
ein unsubstituierter C₃-C₈-Cycloalkylrest, ein unsubstituierter 5-6gliedriger Aryl- oder Heteroarylring, der gegebenenfalls ein- oder mehrfach substituiert sein kann, wobei die Substituenten ausgewählt sein können aus der Gruppe
Halogen, Cyano, -R⁹ -OR⁹, -OC(O)R⁹, -S(O)ₙR⁹ wobei n = 0, 1, 2 bedeutet, -SO₂NHR⁹, NR⁹R¹⁰, -NHC(O)R⁹, -CO₂-R⁹, -C(O)-N-R⁹R¹⁰,
wobei es sich bei dem 5-6-gliedrigen Aryl- oder Heteroarylring beispielsweise, aber nicht ausschließlich, um eine Phenyl-, Pyridinyl-, Pyrimidinyl-, Furanyl-, Thiophenyl-, Oxazolyl-, Isoxazolyl-, Thiazolyl-, Pyrrolyl-, Pyrazolyl-, Imidazolyl-, Pyrazinyl-, Pyridazinyl-,
Triazolyl-, Tetrazolyl-Gruppe handeln kann, oder
- R⁶, R⁷: zusammen einen 3-8-gliedrigen Ring bilden,
- R⁹, R¹⁰: unabhängig voneinander Wasserstoff, eine C₁-C₄-Alkylgruppe, welche unsubstituiert oder gegebenenfalls bis zu fünffach fluoriert sein kann,
- eine unsubstituierte C₂-C₄-Alkenylgruppe, welche gegebenenfalls bis zu dreifach fluoriert sein kann,
- eine unsubstituierte C₂-C₄-Alkinylgruppe, welche gegebenenfalls einfach fluoriert sein kann,
- eine unsubstituierte C₃-C₆-Cycloalkylgruppe,
- ein unsubstituierter 5-6-gliedriger Aryl- oder Heteroarylring,
wobei es sich dabei beispielsweise, aber nicht ausschließlich, um einen Phenyl-, Pyridinyl-, Pyrimidinyl-, Furanyl-, Thiophenyl-, Oxazolyl-, Isoxazolyl-, Thiazolyl-, Pyrrolyl, Pyrazolyl-, Imidazolyl-, Pyrazinyl-, Pyridazinyl-, Triazolyl-, Tetrazolyl-Ring handeln kann, der gegebenenfalls bis zu zweifach mit Fluor, Chlor, Trifluormethyl substituiert sein kann, oder
- R⁹, R¹⁰: zusammen einen 3-8-gliedrigen Ring bilden,
bedeuten.

Ebenfalls bevorzugt sind die Verbindungen der allgemeinen Formel I, wobei
- X und Z: ein Stickstoffrest,
- Y und Z: ein Kohlenstoffrest -C-R⁸,
- R⁸: ein Wasserstoff,
- R¹: ein unsubstituierter 5-6-gliedriger Aryl- oder Heteroarylring, der gegebenenfalls ein- bis dreifach substituiert ist,
wobei die Substituenten ausgewählt sein können aus der Gruppe Halogen, -C₁-C₄-Alkyl, das unsubstituiert ist oder gegebenenfalls substituiert sein kann,
-OR⁶, -OC(O)R⁶, -S(O)ₙR⁶, wobei n = 0, 1, 2 bedeutet,-SO₂NHR⁶, -SO₂NHC(O)R⁶, NR⁶R⁷, -NHC(O)R⁶, -CN, -CO₂-R⁶, -C(O)-N-R⁶R⁷, -C(O)R⁶, -C(OH)R⁶R⁷,
wobei es sich bei dem 5-6-gliedrigen Aryl- oder Heteroarylring beispielsweise, aber nicht ausschließlich, um eine Phenyl-, Pyridinyl-, Pyrimidinyl-, Furanyl-, Thiophenyl-, Oxazolyl-,
Isoxazolyl-, Thiazolyl-, Pyrrolyl-, Pyrazolyl-, Imidazolyl-, Pyrazinyl-, Pyridazinyl-,
Triazolyl-, Tetrazolyl- Gruppe handeln kann,
- R²: ein Wasserstoffrest,
- R³- R⁵: unabhängig voneinander ein Wasserstoff, Halogen, Cyano, oder
- eine OR⁶, OC(O)R⁶, S(O)ₙR⁶, wobei n = 0, 1, 2, bedeutet, SO₂NHR⁶, SO₂NHC(O)R⁶, NR⁶R⁷, NHC(O)R⁶, CH₂NR⁶R⁷, CH₂NHC(O)R⁶, C(OH)R⁶R⁷, C(O)R⁶, CO₂R⁶, C(O)NR⁶R⁷-Gruppe,
- eine unsubstituierte C₁-C₆-Alkylgruppe, die gegebenenfalls substituiert sein kann,
- ein unsubstituierter C₃-C₁₀-Cycloalkylring, der gegebenenfalls substituiert sein kann,
- eine unsubstituierte C₂-C₆-Alkenyl-oder C₂-C₆-Alkinylgruppe, die gegebenenfalls substituiert sein kann,
- ein unsubstituierter 5-6-gliedriger Aryl- oder Heteroarylring, der gegebenenfalls ein- oder mehrfach substituiert ist,
wobei die Substituenten ausgewählt sein können aus der Gruppe
Halogen,
-C₁-C₄-Alkyl, das unsubstituiert ist und gegebenenfalls auch halogeniert oder aber mit -OH, -CN, -CO₂H substituiert sein kann,
-OR⁹, -OC(O)R⁹, -S(O)ₙR⁹ wobei n = 0, 1, 2 bedeutet, -SO₂NHR⁹, -SO₂NHC(O)R⁹, NR⁹R¹⁰, -NHC(O)R⁹, -CN, -CO₂-R⁹, -C(O)-N-R⁹R¹⁰, -C(O)R⁹, -C(OH)R⁹R¹⁰,
wobei es sich bei dem 5-6-gliedrigen Aryl- oder
Heteroarylring beispielsweise, aber nicht ausschließlich, um eine Phenyl-, Pyridinyl-, Pyrimidinyl-, Furanyl-, Thiophenyl-, Oxazolyl-, Isoxazolyl-, Thiazolyl-, Pyrrolyl-, Pyrazolyl-, Imidazolyl-, Pyrazinyl-, Pyridazinyl-,Triazolyl-, Tetrazolyl-, Gruppe handeln kann,
- R⁶, R⁷: ein Wasserstoff,
eine unsubstituierte C₁-C₆-Alkylgruppe, die gegebenenfalls bis zu fünffach halogeniert sein kann,
ein unsubstituierter C₃-C₈-Cycloalkylrest,
ein unsubstituierter 5-6-gliedriger Aryl- oder Heteroarylring, der gegebenenfalls ein- oder mehrfach substituiert sein kann, wobei die Substituenten ausgewählt sein können aus der Gruppe
Halogen, Cyano, -R⁹ -OR⁹, -OC(O)R⁹, -S(O)ₙR⁹ wobei n = 0, 1, 2 bedeutet, -SO₂NHR⁹, NR⁹R¹⁰, -NHC(O)R⁹, -CO₂-R⁹, -C(O)-N-R⁹R¹⁰,
wobei es sich bei dem 5-6-gliedrigen Aryl- oder Heteroarylring beispielsweise, aber nicht ausschließlich, um eine Phenyl-, Pyridinyl-, Pyrimidinyl-, Furanyl-, Thiophenyl-, Oxazolyl-, Isoxazolyl-, Thiazolyl-, Pyrrolyl-, Pyrazolyl-, Imidazolyl-, Pyrazinyl-, Pyridazinyl-,
Triazolyl-, Tetrazolyl-Gruppe handeln kann, oder
- R⁶, R⁷: zusammen einen 3-8-gliedrigen Ring bilden,
- R⁹, R¹⁰: unabhängig voneinander Wasserstoff, eine C₁-C₄-Alkylgruppe, welche unsubstituiert oder gegebenenfalls bis zu fünffach fluoriert sein kann,
- eine unsubstituierte C₂-C₄-Alkenylgruppe, welche gegebenenfalls bis zu dreifach fluoriert sein kann,
- eine unsubstituierte C₂-C₄-Alkinylgruppe, welche gegebenenfalls einfach fluoriert sein kann, eine C₃-C₆-Cycloalkylgruppe,
- ein unsubstituierter 5-6-gliedriger Aryl- oder Heteroarylring, wobei es sich dabei beispielsweise, aber nicht ausschließlich, um einen Phenyl-, Pyridinyl-, Pyrimidinyl-, Furanyl-, Thiophenyl-, Oxazolyl-, Isoxazolyl-, Thiazolyl-, Pyrrolyl-Pyrazolyl-, Imidazolyl-, Pyrazinyl-, Pyridazinyl-,Triazolyl-, Tetrazolyl-Ring handeln kann, der gegebenenfalls bis zu zweifach mit Fluor, Chlor, Trifluormethyl substituiert sein kann, oder
- R⁹, R¹⁰: zusammen einen 3-8-gliedrigen Ring bilden,
bedeuten.

Ebenfalls bevorzugt sind die Verbindungen der allgemeinen Formell, wobei
- X und Y: ein Kohlenstoffrest -C-R⁸,
- Z: ein Stickstoffrest,
- R⁸: ein Wasserstoff,
- R¹: ein unsubstituierter 5-6-gliedriger Aryl- oder Heteroarylring, der gegebenenfalls ein- bis dreifach substituiert ist,
wobei die Substituenten ausgewählt sein können aus der Gruppe
Halogen,
-C₁-C₄-Alkyl, das unsubstituiert ist oder gegebenenfalls substituiert sein kann,
-OR⁶, -OC(O)R⁶, -S(O)ₙR⁶, wobei n = 0, 1, 2 bedeutet, -SO₂NHR⁶, -SO₂NHC(O)R⁶, NR⁶R⁷,-NHC(O)R⁶, -CN, -CO₂-R⁶, -C(O)-N-R⁶R⁷, -C(O)R⁶,-C(OH)R⁶R⁷,
wobei es sich bei dem 5-6-gliedrigen Aryl- oder Heteroarylring beispielsweise, aber nicht ausschließlich, um eine Phenyl-, Pyridinyl-, Pyrimidinyl-, Furanyl-, Thiophenyl-, Oxazolyl-, Isoxazolyl-, Thiazolyl-, Pyrrolyl-, Pyrazolyl-, Imidazolyl-, Pyrazinyl-, Pyridazinyl-,
Triazolyl-, Tetrazolyl- Gruppe handeln kann,
- R²: ein Wasserstoffrest,
- R³ - R⁵: unabhängig voneinander ein Wasserstoff, Halogen, Cyano, oder
- eine OR⁶, OC(O)R⁶, S(O)ₙR⁶, wobei n = 0, 1, 2, bedeutet, SO₂NHR⁶, SO₂NHC(O)R⁶, NR⁶R⁷, NHC(O)R⁶, CH₂NR⁶R⁷, CH₂NHC(O)R⁶, C(OH)R⁶R⁷, C(O)R⁶, CO₂R⁶, C(O)NR⁶R⁷ Gruppe,
- eine unsubstituierte C₁-C₆-Alkylgruppe, die gegebenenfalls substituiert sein kann,
- ein unsubstituierter C₃-C₁₀-Cycloalkylring, der gegebenenfalls substituiert sein kann,
- eine unsubstituierte C₂-C₆-Alkenyl-oder C₂-C₆-Alkinylgruppe, die gegebenenfalls substituiert sein kann,
- ein unsubstituierter 5-6-gliedriger Aryl- oder Heteroarylring, der gegebenenfalls ein-oder mehrfach substituiert ist,
wobei die Substituenten ausgewählt sein können aus der Gruppe
Halogen,
-C₁-C₄-Alkyl, das unsubstituiert ist und gegebenenfalls auch halogeniert oder aber mit -OH, -CN, -CO₂H substituiert sein kann,
-OR⁹, -OC(O)R⁹, -S(O)ₙR⁹ wobei n = 0, 1, 2 bedeutet, -SO₂NHR⁹, -SO₂NHC(O)R⁹, NR⁹R¹⁰, -NHC(O)R⁹, -CN, -CO₂-R⁹, -C(O)-N-R⁹R¹⁰, -C(O)R⁹, -C(OH)R⁹R¹⁰,
wobei es sich bei dem 5-6-gliedrigen Aryl- oder Heteroarylring beispielsweise, aber nicht ausschließlich, um eine Phenyl-, Pyridinyl-, Pyrimidinyl-, Furanyl-, Thiophenyl-, Oxazolyl-, Isoxazolyl-, Thiazolyl-, Pyrrolyl-, Pyrazolyl-, Imidazolyl-, Pyrazinyl-, Pyridazinyl-,Triazolyl-, Tetrazolyl-, Gruppe handeln kann,
- R⁶, R⁷: ein Wasserstoff, eine unsubstituierte C₁-C₆-Alkylgruppe, die gegebenenfalls bis zu fünffach halogeniert sein kann,
ein unsubstituierter C₃-C₈-Cycloalkylrest,
ein unsubstituierter 5-6-gliedriger Aryl- oder Heteroarylring, der gegebenenfalls ein- oder mehrfach substituiert sein kann, wobei die Substituenten ausgewählt sein können aus der Gruppe
Halogen, Cyano, -R⁹ -OR⁹, -OC(O)R⁹, -S(O)ₙR⁹ wobei n = 0, 1, 2 bedeutet, -SO₂NHR⁹, NR⁹R¹⁰, -NHC(O)R⁹, -CO₂-R⁹, -C(O)-N-R⁹R¹⁰,
wobei es sich bei dem 5-6-gliedrigen Aryl- oder Heteroarylring beispielsweise, aber nicht ausschließlich, um eine Phenyl-, Pyridinyl-, Pyrimidinyl-, Furanyl-, Thiophenyl-, Oxazolyl-, Isoxazolyl-, Thiazolyl-, Pyrrolyl-, Pyrazolyl-, Imidazolyl-, Pyrazinyl-, Pyridazinyl-,
Triazolyl-, Tetrazolyl--Gruppe handeln kann, oder
- R⁶, R⁷: zusammen einen 3-8-gliedrigen Ring bilden,
- R⁹, R¹⁰: unabhängig voneinander Wasserstoff, eine C₁-C₄-Alkylgruppe, welche unsubstituiert oder gegebenenfalls bis zu fünffach fluoriert sein kann,
- eine unsubstituierte C₂-C₄-Alkenylgruppe, welche gegebenenfalls bis zu dreifach fluoriert sein kann,
- eine unsubstituierte C₂-C₄-Alkinylgruppe, welche gegebenenfalls einfach fluoriert sein kann,
- eine unsubstituierte C₃-C₆-Cycloalkylgruppe,
- ein unsubstituierter 5-6-gliedriger Aryl- oder Heteroarylring,
   wobei es sich dabei beispielsweise, aber nicht ausschließlich, um einen Phenyl-, Pyridinyl-, Pyrimidinyl-, Furanyl-, Thiophenyl-, Oxazolyl-, Isoxazolyl-, Thiazolyl-, Pyrrolyl-, Pyrazolyl-, Imidazolyl-, Pyrazinyl-, Pyridazinyl-, Triazolyl-, Tetrazolyl-Ring handeln kann, der gegebenenfalls bis zu zweifach mit Fluor, Chlor, Trifluormethyl substituiert sein kann, oder
- R⁹, R¹⁰: zusammen einen 3-8-gliedrigen Ring bilden,
bedeuten.

Ebenfalls bevorzugt sind die Verbindungen der allgemeinen Formel I, wobei
- X: ein Stickstoffrest,
- Y und Z: ein Kohlenstoffrest -C-R⁸,
- R⁸: ein Wasserstoff,
- R¹: ein Phenyl-, Thiophen-2-yl, Thiophen-3-yl, Furan-2-yl-, Furan-3-yl-, Pyrid-3-yl-, Pyrid-4-ylring, der gegebenenfalls ein- bis dreifach substituiert ist,
wobei die Substituenten ausgewählt sein können aus der Gruppe
Halogen,
-C₁-C₄-Alkyl, das unsubstituiert oder gegebenenfalls substituiert sein kann,
-OR⁶, -OC(O)R⁶, -S(O)ₙR⁶, wobei n = 0, 1, 2 bedeutet, -SO₂NHR⁶, -SO₂NHC(O)R⁶, NR⁶R⁷,-NHC(O)R⁶, -CN, -CO₂-R⁶, -C(O)-N-R⁶R⁷, -C(O)R⁶,-C(OH)R⁶R⁷,
- R²: ein Wasserstoffrest,
- R³ - R⁵: unabhängig voneinander ein Wasserstoff, Halogen, Cyano, oder
- eine OR⁶, OC(O)R^{b}, S(O)ₙR⁶, wobei n = 0, 1, 2, bedeutet, SO₂NHR⁶, SO₂NHC(O)R⁶, NR⁶R⁷, NHC(O)R⁶, CH₂NR⁶R⁷, CH₂NHC(O)R⁶, C(OH)R⁶R⁷, C(O)R⁶, CO₂R⁶, C(O)NR⁶R⁷ Gruppe,
- eine unsubstituierte C₁-C₆-Alkylgruppe, die gegebenenfalls bis zu fünffach halogeniert oder aber mit, -CN, -CO₂H substituiert sein kann,
- ein unsubstituierter C₃-C₁₀-Cycloalkylring,
- eine unsubstituierte C₂-C₆-Alkenylgruppe, die gegebenenfalls bis zu dreifach halogeniert oder aber mit - CN, -CO₂H substituiert sein kann,
- eine unsubstituierte C₂-C₆-Alkinylgruppe, die gegebenenfalls einfach halogeniert oder aber mit -CN,-CO₂H substituiert sein kann und,
- ein unsubstituierter 5-6-gliedriger Aryl- oder Heteroarylring, der gegebenenfalls ein-oder mehrfach substituiert ist,
wobei die Substituenten ausgewählt sein können aus der
Gruppe
Halogen,
-C₁-C₄-Alkyl, das unsubstituiert ist und gegebenenfalls bis zu fünffach halogeniert oder aber mit-OH, -CN, -CO₂H substituiert sein kann,
-OR⁹, -OC(O)R⁹, -S(O)ₙR⁹, wobei n = 0, 1, 2 bedeutet, -SO₂NHR⁹, -SO₂NHC(O)R⁹, NR⁹R¹⁰,-NHC(O)R⁹, -CN, -CO₂-R⁹, -C(O)-N-R⁹R¹⁰, -C(O)R⁹,-C(OH)R⁹R¹⁰,
wobei es sich bei dem 5-6-gliedrigen Aryl- oder Heteroarylring beispielsweise, aber nicht ausschließlich, um eine Phenyl-, Pyridinyl-, Pyrimidinyl-, Furanyl-, Thiophenyl-, Oxazolyl-, Isoxazolyl-, Thiazolyl-, Pyrrolyl-, Pyrazolyl-, Imidazolyl-, Pyrazinyl-, Pyridazinyl-,
Triazolyl-, Tetrazolyl-, -Gruppe handeln kann,
- R⁶, R⁷: ein Wasserstoff, eine unsubstituierte C₁-C₆-Alkylgruppe, die gegebenenfalls bis zu fünffach halogeniert sein kann,
eine unsubstituierte C₃-C₈-Cycloalkylgruppe,
ein unsubstituierter 5-6-gliedriger Aryl- oder Heteroarylring, der gegebenenfalls ein- oder mehrfach substituiert sein kann, wobei die Substituenten ausgewählt sein können aus der Gruppe
Halogen,
Cyano,
R⁹ -OR⁹, -OC(O)R⁹, -S(O)ₙR⁹, wobei n = 0, 1, 2 bedeutet,-SO₂NHR⁹, - NR⁹R¹⁰, -NHC(O)R⁹, -CO₂-R⁹, -C(O)-N-R⁹R¹⁰, wobei es sich bei dem 5-6-gliedrigen Aryl- oder
Heteroarylring beispielsweise, aber nicht ausschließlich, um eine Phenyl-, Pyridinyl-, Pyrimidinyl-, Furanyl-, Thiophenyl-, Oxazolyl-, Isoxazolyl-, Thiazolyl-, Pyrrolyl-, Pyrazolyl-, Imidazolyl-, Pyrazinyl-, Pyridazinyl-,Triazolyl-, Tetrazolyl-, Gruppe handeln kann, oder
- R⁶, R⁷: zusammen einen 3-8-gliedrigen Ring bilden,
- R⁹, R¹⁰: unabhängig voneinander Wasserstoff, eine C₁-C₄-Alkylgruppe, welche unsubstituiert ist oder gegebenenfalls bis zu fünffach fluoriert sein kann,
eine unsubstituierte C₂-C₄-Alkenylgruppe, welche gegebenenfalls bis zu dreifach fluoriert sein kann,
eine unsubstituierte C₂-C₄-Alkinylgruppe, welche gegebenenfalls einfach fluoriert sein kann,
eine unsubstituierte C₃-C₆-Cycloalkylgruppe,
ein unsubstituierter 5-6-gliedriger Aryl- oder Heteroarylring, wobei es sich dabei beispielsweise, aber nicht ausschließlich, um einen
Phenyl-, Pyridinyl-, Pyrimidinyl-, Furanyl-, Thiophenyl-, Oxazolyl-, Isoxazolyl-, Thiazolyl-, Pyrrolyl-, Pyrazolyl-, Imidazolyl-, Pyrazinyl-, Pyridazinyl-, Triazolyl-, Tetrazolyl-Ring handeln kann, der gegebenenfalls bis zu zweifach mit Fluor, Chlor, Trifluormethyl substituiert sein kann, oder
- R⁹, R¹⁰: zusammen einen 3-8-gliedrigen Ring bilden,
bedeuten.

Ebenfalls bevorzugt sind die Verbindungen der allgemeinen Formel I, wobei
- Y und Z: ein Stickstoffrest,
- X: ein Kohlenstoffrest -C-R⁸,
- R⁸: ein Wasserstoff,
- R¹: ein Phenyl-, Thiophen-2-yl, Thiophen-3-yl, Furan-2-yl-, Furan-3-yl-, Pyrid-3-yl-, Pyrid-4-ylring, der gegebenenfalls ein- bis dreifach substituiert ist,
wobei die Substituenten ausgewählt sein können aus der Gruppe Halogen, -C₁-C₄-Alkyl, das unsubstituiert oder gegebenenfalls substituiert sein kann,
-OR⁶, -OC(O)R⁶, -S(O)ₙR⁶, wobei n = 0, 1, 2 bedeutet,-SO₂NHR⁶, -SO₂NHC(O)R⁶, NR⁶R⁷, -NHC(O)R⁶, -CN, -CO₂-R⁶, -C(O)-N-R⁶R⁷, -C(O)R⁶, -C(OH)R⁶R⁷,
- R²: ein Wasserstoffrest,
- R³ - R⁵: unabhängig voneinander ein Wasserstoff, Halogen, Cyano, oder
- eine OR⁶, OC(O)R⁶, S(O)ₙR⁶, wobei n = 0, 1, 2, bedeutet, SO₂NHR⁶, SO₂NHC(O)R⁶, NR⁶R⁷, NHC(O)R⁶, CH₂NR⁶R⁷, CH₂NHC(O)R⁶, C(OH)R⁶R⁷, C(O)R⁶, CO₂R⁶, C(O)NR⁶R⁷ Gruppe,
- eine unsubstituierte C₁-C₆-Alkylgruppe, die gegebenenfalls bis zu fünffach halogeniert oder aber mit -CN, -CO₂H substituiert sein kann,
- ein unsubstituierter C₃-C₁₀-Cycloalkylring,
- eine unsubstituierte C₂-C₆-Alkenylgruppe, die gegebenenfalls bis zu dreifach halogeniert oder aber mit-CN, -CO₂H substituiert sein kann,
- eine unsubstituierte C₂-C₆-Alkinylgruppe, die gegebenenfalls einfach halogeniert oder aber mit -CN,-CO₂H substituiert sein kann und,
- ein unsubstituierter 5-6-gliedriger Aryl- oder Heteroarylring, der gegebenenfalls ein-oder mehrfach substituiert ist,
wobei die Substituenten ausgewählt sein können aus der
Gruppe
Halogen,
C₁-C₄-Alkyl, das unsubstituiert ist und gegebenenfalls bis zu fünffach halogeniert oder aber mit -OH, -CN,-CO₂H substituiert sein kann,
-OR⁹, -OC(O)R⁹, -S(O)ₙR⁹, wobei n = 0, 1, 2 bedeutet, -SO₂NHR⁹, -SO₂NHC(O)R⁹, NR⁹R¹⁰,-NHC(O)R⁹, -CN, -CO₂-R⁹, -C(O)-N-R⁹R¹⁰, -C(O)R⁹,-C(OH)R⁹R¹⁰,
wobei es sich bei dem 5-6-gliedrigen Aryl- oder Heteroarylring beispielsweise, aber nicht ausschließlich, um eine Phenyl-, Pyridinyl-, Pyrimidinyl-, Furanyl-, Thiophenyl-, Oxazolyl-, Isoxazolyl-, Thiazolyl-, Pyrrolyl-, Pyrazolyl-, Imidazolyl-, Pyrazinyl-, Pyridazinyl-,
Triazolyl-, Tetrazolyl-, -Gruppe handeln kann,
- R⁶, R⁷: ein Wasserstoff, eine unsubstituierte C₁-C₆-Alkylgruppe, die gegebenenfalls bis zu fünffach halogeniert sein kann,
eine unsubstituierte C₃-C₈-Cycloalkylgruppe,
ein unsubstituierter 5-6-gliedriger Aryl- oder Heteroarylring, der gegebenenfalls ein- oder mehrfach substituiert sein kann, wobei die Substituenten ausgewählt sein können aus der Gruppe
- Halogen,
- Cyano,
R⁹, -OR⁹, -OC(O)R⁹, -S(O)ₙR⁹, wobei n = 0, 1, 2 bedeutet, - SO₂NHR⁹, - NR⁹R¹⁰, -NHC(O)R⁹, -CO₂-R⁹, -C(O)-N-R⁹R¹⁰,
wobei es sich bei dem 5-6-gliedrigen Aryl- oder Heteroarylring beispielsweise, aber nicht ausschließlich, um eine Phenyl-, Pyridinyl-, Pyrimidinyl-, Furanyl-, Thiophenyl-, Oxazolyl-, Isoxazolyl-, Thiazolyl-, Pyrrolyl-, Pyrazolyl-, Imidazolyl-, Pyrazinyl-, Pyridazinyl-,
Triazolyl-, Tetrazolyl-, Gruppe handeln kann, oder
- R⁶, R⁷: zusammen einen 3-8-gliedrigen Ring bilden,
- R⁹, R¹⁰: unabhängig voneinander Wasserstoff, eine C₁-C₄-Alkylgruppe, welche unsubstituiert ist oder gegebenenfalls bis zu fünffach fluoriert sein kann,
eine unsubstituierte C₂-C₄-Alkenylgruppe, welche gegebenenfalls bis zu dreifach fluoriert sein kann,
eine unsubstituierte C₂-C₄-Alkinylgruppe, welche gegebenenfalls einfach fluoriert sein kann,
eine unsubstituierte C₃-C₆-Cycloalkylgruppe,
ein unsubstituierter 5-6-gliedriger Aryl- oder Heteroarylring, wobei es sich dabei beispielsweise, aber nicht ausschließlich, um einen
Phenyl-, Pyridinyl-, Pyrimidinyl-, Furanyl-, Thiophenyl-, Oxazolyl-, Isoxazolyl-, Thiazolyl-, Pyrrolyl-, Pyrazolyl-, Imidazolyl-, Pyrazinyl-, Pyridazinyl-, Triazolyl-, Tetrazolyl-Ring handeln kann, der gegebenenfalls bis zu zweifach mit Fluor, Chlor, Trifluormethyl substituiert sein kann, oder
- R⁹, R¹⁰: zusammen einen 3-8-gliedrigen Ring bilden,
bedeuten.

Ebenfalls bevorzugt sind die Verbindungen der allgemeinen Formel I, wobei
- X und Z: ein Stickstoffrest,
- Y: ein Kohlenstoffrest -C-R⁸,
- R⁸: ein Wasserstoff,
- R¹: ein Phenyl-, Thiophen-2-yl, Thiophen-3-yl, Furan-2-yl-, Furan-3-yl-, Pyrid-3-yl-, Pyrid-4-ylring, der gegebenenfalls ein- bis dreifach substituiert ist,
wobei die Substituenten ausgewählt sein können aus der Gruppe Halogen, -C₁-C₄-Alkyl, das unsubstituiert oder gegebenenfalls substituiert sein kann,
-OR⁶, -OC(O)R⁶, -S(O)ₙR⁶, wobei n = 0, 1, 2 bedeutet,-SO₂NHR⁶, -SO₂NHC(O)R⁶, NR⁶R⁷, -NHC(O)R⁶, -CN, -CO₂-R⁶, -C(O)-N-R⁶R⁷, -C(O)R⁶, -C(OH)R⁶R⁷,
- R²: ein Wasserstoffrest,
- R³- R⁵: unabhängig voneinander ein Wasserstoff, Halogen, Cyano, oder
- eine OR⁶, OC(O)R⁶, S(O)ₙR⁶, wobei n = 0, 1, 2, bedeutet, SO₂NHR⁶, SO₂NHC(O)R⁶, NR⁶R⁷, NHC(O)R⁶, CH₂NR⁶R⁷, CH₂NHC(O)R⁶, C(OH)R⁶R⁷, C(O)R⁶, CO₂R⁶, C(O)NR⁶R⁷-Gruppe,
- eine unsubstituierte C₁-C₆-Alkylgruppe, die gegebenenfalls bis zu fünffach halogeniert oder aber mit -CN, -CO₂H substituiert sein kann,
- ein unsubstituierter C₃-C₁₀-Cycloalkylring,
- eine unsubstituierte C₂-C₆-Alkenylgruppe, die gegebenenfalls bis zu dreifach halogeniert oder aber mit-CN, -CO₂H substituiert sein kann,
- eine unsubstituierte C₂-C₆-Alkinylgruppe, die gegebenenfalls einfach halogeniert oder aber mit -CN,-CO₂H substituiert sein kann, und
- ein unsubstituierter 5-6-gliedriger Aryl- oder Heteroarylring, der gegebenenfalls ein-oder mehrfach substituiert ist,
wobei die Substituenten ausgewählt sein können aus der
Gruppe
- Halogen,
-C₁-C₄-Alkyl, das unsubstituiert ist und
gegebenenfalls bis zu fünffach halogeniert oder aber mit -OH, -CN, -CO₂H substituiert sein kann,
-OR⁹, -OC(O)R⁹, -S(O)ₙR⁹, wobei n = 0, 1, 2 bedeutet, -SO₂NHR⁹, -SO₂NHC(O)R⁹, NR⁹R¹⁰,-NHC(O)R⁹, -CN, -CO₂-R⁹, -C(O)-N-R⁹R¹⁰, -C(O)R⁹,-C(OH)R⁹R¹⁰,
wobei es sich bei dem 5-6-gliedrigen Aryl- oder Heteroarylring beispielsweise, aber nicht ausschließlich, um eine Phenyl-, Pyridinyl-, Pyrimidinyl-, Furanyl-, Thiophenyl-, Oxazolyl-, Isoxazolyl-, Thiazolyl-, Pyrrolyl-, Pyrazolyl-, Imidazolyl-, Pyrazinyl-, Pyridazinyl-,
Triazolyl-, Tetrazolyl-, -Gruppe handeln kann,
- R⁶, R⁷: ein Wasserstoff,
eine C₁-C₆-Alkylgruppe, die unsubstituiert oder gegebenenfalls bis zu fünffach halogeniert sein kann,
eine C₃-C₈-Cycloalkylgruppe,
ein 5-6-gliedriger Aryl- oder Heteroarylring, der gegebenenfalls ein- oder mehrfach substituiert sein kann, wobei die Substituenten ausgewählt sein können aus der Gruppe
Halogen,
Cyano,
eine unsubstituierte C₃-C₈-Cycloalkylgruppe,
R⁹, -OR⁹, -OC(O)R⁹, -S(O)ₙR⁹, wobei n = 0, 1, 2 bedeutet,-SO₂NHR⁹, - NR⁹R¹⁰, -NHC(O)R⁹, -CO₂-R⁹, -C(O)-N-R⁹R¹⁰,
wobei es sich bei dem 5-6-gliedrigen Aryl- oder Heteroarylring beispielsweise, aber nicht ausschließlich, um eine Phenyl-, Pyridinyl-, Pyrimidinyl-, Furanyl-, Thiophenyl-, Oxazolyl-, Isoxazolyl-, Thiazolyl-, Pyrrolyl-, Pyrazolyl-, Imidazolyl-, Pyrazinyl-, Pyridazinyl-,
Triazolyl-, Tetrazolyl-, Gruppe handeln kann, oder
- R⁶, R⁷: zusammen einen 3-8-gliedrigen Ring bilden,
- R⁹, R¹⁰: unabhängig voneinander Wasserstoff, eine C₁-C₄-Alkylgruppe, welche unsubstituiert ist oder gegebenenfalls bis zu fünffach fluoriert sein kann,
eine unsubstituierte C₂-C₄-Alkenylgruppe, welche gegebenenfalls bis zu dreifach fluoriert sein kann,
eine unsubstituierte C₂-C₄-Alkinylgruppe, welche gegebenenfalls einfach fluoriert sein kann,
eine unsubstituierte C₃-C₆-Cycloalkylgruppe,
ein unsubstituierter 5-6-gliedriger Aryl- oder Heteroarylring, wobei es sich dabei beispielsweise, aber nicht ausschließlich, um einen
Phenyl-, Pyridinyl-, Pyrimidinyl-, Furanyl-, Thiophenyl-, Oxazolyl-, Isoxazolyl-, Thiazolyl-, Pyrrolyl-, Pyrazolyl-, Imidazolyl-, Pyrazinyl-, Pyridazinyl-, Triazolyl-, Tetrazolyl-Ring handeln kann, der gegebenenfalls bis zu zweifach mit Fluor, Chlor, Trifluormethyl substituiert sein kann, oder
- R⁹, R¹⁰: zusammen einen 3-8-gliedrigen Ring bilden,
bedeuten.

Ebenfalls bevorzugt sind die Verbindungen der allgemeinen Formel I, wobei
- X und Y: ein Kohlenstoffrest -C-R⁸,
- Z: ein Stickstoffrest,
- R⁸: ein Wasserstoff,
- R¹: ein Phenyl-, Thiophen-2-yl, Thiophen-3-yl, Furan-2-yl-, Furan-3-yl-, Pyrid-3-yl-, Pyrid-4-ylring, der gegebenenfalls ein- bis dreifach substituiert ist,
wobei die Substituenten ausgewählt sein können aus der Gruppe Halogen, -C₁-C₄-Alkyl, das unsubstituiert oder gegebenenfalls substituiert sein kann,
-OR⁶, -OC(O)R⁶, -S(O)ₙR⁶, wobei n = 0, 1, 2 bedeutet, - SO₂NHR⁶, -SO₂NHC(O)R⁶, NR⁶R⁷, -NHC(O)R⁶, -CN, -CO₂-R⁶, -C(O)-N-R⁶R⁷, -C(O)R⁶, -C(OH)R⁶R⁷,
- R²: ein Wasserstoffrest,
- R³- R⁵: unabhängig voneinander ein Wasserstoff, Halogen, Cyano, oder
- eine OR⁶, OC(O)R⁶, S(O)ₙR⁶, wobei n = 0, 1, 2, bedeutet, SO₂NHR⁶, SO₂NHC(O)R⁶, NR⁶R⁷, NHC(O)R⁶, CH₂NR⁶R⁷, CH₂NHC(O)R⁶, C(OH)R⁶R⁷, C(O)R⁶, CO₂R⁶, C(O)NR⁶R⁷⁻ Gruppe,
- eine unsubstituierte C₁-C₆-Alkylgruppe, die gegebenenfalls bis zu fünffach halogeniert oder aber mit -CN, -CO₂H substituiert sein kann,
- ein unsubstituierter C₃-C₁₀-Cycloalkylring,
- eine unsubstituierte C₂-C₆-Alkenylgruppe,die gegebenenfalls bis zu dreifach halogeniert oder aber mit-CN, -CO₂H substituiert sein kann,
- eine unsubstituierte C₂-C₆-Alkinylgruppe, die gegebenenfalls einfach halogeniert oder aber mit -CN,-CO₂H substituiert sein kann und,
- ein unsubstituierter 5-6-gliedriger Aryl- oder Heteroarylring, der gegebenenfalls ein-oder mehrfach substituiert ist,
wobei die Substituenten ausgewählt sein können aus der
Gruppe
- Halogen,
-C₁-C₄-Alkyl, das unsubstituiert ist und
gegebenenfalls bis zu fünffach halogeniertoder aber mit -OH, -CN, -CO₂H substituiert sein kann,
-OR⁹, -OC(O)R⁹, -S(O)ₙR⁹, wobei n = 0,1, 2 bedeutet, -SO₂NHR⁹, -SO₂NHC(O)R⁹, NR⁹R¹⁰,-NHC(O)R⁹, -CN, -CO₂-R⁹, -C(O)-N-R⁹R¹⁰, -C(O)R⁹,-C(OH)R⁹R¹⁰,
wobei es sich bei dem 5-6-gliedrigen Aryl- oder Heteroarylring beispielsweise, aber nicht ausschließlich, um eine Phenyl-, Pyridinyl-, Pyrimidinyl-, Furanyl-, Thiophenyl-, Oxazolyl-, Isoxazolyl-, Thiazolyl-, Pyrrolyl-, Pyrazolyl-, Imidazolyl-, Pyrazinyl-, Pyridazinyl-,Triazolyl-, Tetrazolyl-, -Gruppe handeln kann,
- R⁶, R⁷: ein Wasserstoff,
eine C₁-C₆-Alkylgruppe, die unsubstituiert ist oder gegebenenfalls bis zu fünffach halogeniert sein kann,
ein 5-6-gliedriger Aryl- oder Heteroarylring, der gegebenenfalls ein- oder mehrfach substituiert sein kann, wobei die Substituenten
ausgewählt sein können aus der Gruppe
Halogen,
Cyano,
eine C₃-C₈-Cycloalkylgruppe,
R⁹, -OR⁹, -OC(O)R⁹, -S(O)ₙR⁹, wobei n = 0, 1, 2 bedeutet,
-SO₂NHR⁹, - NR⁹R¹⁰, -NHC(O)R⁹, -CO₂-R⁹, -C(O)-N-R⁹R¹⁰,
wobei es sich bei dem 5-6-gliedrigen Aryl- oder Heteroarylring beispielsweise, aber nicht ausschließlich, um eine Phenyl-, Pyridinyl-, Pyrimidinyl-, Furanyl-, Thiophenyl-, Oxazolyl-, Isoxazolyl-, Thiazolyl-, Pyrrolyl-, Pyrazolyl-, Imidazolyl-, Pyrazinyl-, Pyridazinyl-,
Triazolyl-, Tetrazolyl-, Gruppe handeln kann, oder
- R⁶, R⁷: zusammen einen 3-8-gliedrigen Ring bilden,
- R⁹, R¹⁰: unabhängig voneinander Wasserstoff, eine C₁-C₄-Alkylgruppe, welche unsubstituiert ist oder gegebenenfalls bis zu fünffach fluoriert sein kann,
eine unsubstituierte C₂-C₄-Alkenylgruppe, welche gegebenenfalls bis zu dreifach fluoriert sein kann,
eine unsubstituierte C₂-C₄-Alkinylgruppe, welche gegebenenfalls einfach fluoriert sein kann,
eine unsubstituierte C₃-C₆-Cycloalkylgruppe,
ein unsubstituierter 5-6-gliedriger Aryl- oder Heteroarylring, wobei es sich dabei beispielsweise, aber nicht ausschließlich, um einen
Phenyl-, Pyridinyl-, Pyrimidinyl-, Furanyl-, Thiophenyl-, Oxazolyl-, Isoxazolyl-, Thiazolyl-, Pyrrolyl-, Pyrazolyl-, Imidazolyl-, Pyrazinyl-, Pyridazinyl-,Triazolyl-, Tetrazolyl-Ring handeln kann, der gegebenenfalls bis zu zweifach mit Fluor, Chlor, Trifluormethyl substituiert sein kann, oder
- R⁹, R¹⁰: zusammen einen 3-8-gliedrigen Ring bilden,
bedeuten.

Die folgenden Verbindungen entsprechend der vorliegenden Erfindung sind ganz besonders bevorzugt:
- 1-[1-(7-Chloro-quinolin-4-yl)-piperidin-4-yl]-3-(4-methylsulfanyl-phenyl)-thiourea
- 1-[1-(7-Chloro-quinolin-4-yl)-piperidin-4-yl]-3-phenyl-thiourea
- 1-(3-Chloro-phenyl)-3-[1-(7-chloro-quinolin-4-yl)-piperidin-4-yl]-thiourea
- 1-(2-Chloro-phenyl)-3-[1-(7-chloro-quinolin-4-yl)-piperidin-4-yl]-thiourea
- 1-(1-(7-Chloro-quinolin-4-yl)-piperidin-4-yl)-3-(4-methoxy-phenyl)-thiourea
- 1-(1-(7-Chloro-quinolin-4-yl)-piperidin-4-yl)-3-p-tolyl-thiourea
- 1-(1-(7-Chloro-quinolin-4-yl)-piperidin-4-yl)-3-o-tolyl-thiourea
- 1-(2-Bromo-phenyl)-3-(1-(7-chloro-quinolin-4-yl)-piperidin-4-yl)-thiourea
- 1-(3-Bromo-phenyl)-3-(1-(7-chloro-quinolin-4-yl)-piperidin-4-yl)-thiourea
- 1-(1-(7-Chloro-quinolin-4-yl)-piperidin-4-yl)-3-(3-methoxy-phenyl)-thiourea
- 4-(3-(1-(7-Chloro-quinolin-4-yl)-piperidin-4-yl)-thioureido)-benzoic acid methyl ester
- 1-(1-(7-Chloro-quinolin-4-yl)-piperidin-4-yl)-3-(3-cyano-phenyl)-thiourea
- 1-(1-(7-Chloro-quinolin-4-yl)-piperidin-4-yl)-3-(4-cyano-phenyl)-thiourea
- 1-(1-(7-Chloro-quinolin-4-yl)-piperidin-4-yl)-3-(2-methylsulfanyl-phenyl)-thiourea
- 1-(1-(7-Chloro-quinolin-4-yl)-piperidin-4-yl)-3-(2,3-dichloro-phenyl)-thiourea
- 1-(1-(7-Chloro-quinolin-4-yl)-piperidin-4-yl)-3-(3-methylsulfanyl-phenyl)-thiourea
- 3-(3-(1-(7-Chloro-quinolin-4-yl)-piperidin-4-yl)-thioureido)-benzoic acid methyl ester
- 1-(1-(7-Chloro-quinolin-4-yl)-piperidin-4-yl)-3-(2-trifluoromethoxy-phenyl)-thiourea
- 1-[1-(8-Chloro-quinolin-4-yl)-piperidin-4-yl]-3-(4-methylsulfanyl-phenyl)-thiourea
- 1-[1-(8-Chloro-quinolin-4-yl)-piperidin-4-yl]-3-phenyl-thiourea
- 1-(3-Chloro-phenyl)-3-[1-(8-chloro-quinolin-4-yl)-piperidin-4-yl]-thiourea
- 1-(2-Chloro-phenyl)-3-[1-(8-chloro-quinolin-4-yl)-piperidin-4-yl]-thiourea
- 1-(1-(8-Chloro-quinolin-4-yl)-piperidin-4-yl)-3-(4-methoxy-phenyl)-thiourea
- 1-(1-(8-Chloro-quinolin-4-yl)-piperidin-4-yl)-3-p-tolyl-thiourea
- 1-(1-(8-Chloro-quinolin-4-yl)-piperidin-4-yl)-3-o-tolyl-thiourea
- 1-(2-Bromo-phenyl)-3-(1-(8-chloro-quinolin-4-yl)-piperidin-4-yl)-thiourea
- 1-(3-Bromo-phenyl)-3-(1-(8-chloro-quinolin-4-yl)-piperidin-4-yl)-thiourea
- 1-(1-(8-Chloro-quinolin-4-yl)-piperidin-4-yl)-3-(3-methoxy-phenyl)-thiourea
- 4-(3-(1-(8-Chloro-quinolin-4-yl)-piperidin-4-yl)-thioureido)-benzoic acid methyl ester
- 1-(1-(8-Chloro-quinolin-4-yl)-piperidin-4-yl)-3-(3-cyano-phenyl)-thiourea
- 1-(1-(8-Chloro-quinolin-4-yl)-piperidin-4-yl)-3-(4-cyano-phenyl)-thiourea
- 1-(1-(8-Chloro-quinolin-4-yl)-piperidin-4-yl)-3-(2-methylsulfanyl-phenyl)-thiourea
- 1-(1-(8-Chloro-quinolin-4-yl)-piperidin-4-yl)-3-(2,3-dichloro-phenyl)-thiourea
- 1-(1-(8-Chloro-quinolin-4-yl)-piperidin-4-yl)-3-(3-methylsulfanyl-phenyl)-thiourea
- 3-(3-(1-(8-Chloro-quinolin-4-yl)-piperidin-4-yl)-thioureido)-benzoic acid methyl ester
- 1-(1-(8-Chloro-quinolin-4-yl)-piperidin-4-yl)-3-(2-trifluoromethoxy-phenyl)-thiourea
- 1-[1-(6-Chloro-quinolin-4-yl)-piperidin-4-yl]-3-(4-methylsulfanyl-phenyl)-thiourea
- 1-[1-(6-Chloro-quinolin-4-yl)-piperidin-4-yl]-3-phenyl-thiourea
- 1-(3-Chloro-phenyl)-3-[1-(6-chloro-quinolin-4-yl)-piperidin-4-yl]-thiourea
- 1-(2-Chloro-phenyl)-3-[1-(6-chloro-quinolin-4-yl)-piperidin-4-yl]-thiourea
- 1-(1-(6-Chloro-quinolin-4-yl)-piperidin-4-yl)-3-(4-methoxy-phenyl)-thiourea
- 1-(1-(6-Chloro-quinolin-4-yl)-piperidin-4-yl)-3-p-tolyl-thiourea
- 1-(1-(6-Chloro-quinolin-4-yl)-piperidin-4-yl)-3-o-tolyl-thiourea
- 1-(2-Bromo-phenyl)-3-(1-(6-chloro-quinolin-4-yl)-piperidin-4-yl)-thiourea
- 1-(3-Bromo-phenyl)-3-(1-(6-chloro-quinolin-4-yl)-piperidin-4-yl)-thiourea
- 1-(1-(6-Chloro-quinolin-4-yl)-piperidin-4-yl)-3-(3-methoxy-phenyl)-thiourea
- 4-(3-(1-(6-Chloro-quinolin-4-yl)-piperidin-4-yl)-thioureido)-benzoic acid methyl ester
- 1-(1-(6-Chloro-quinolin-4-yl)-piperidin-4-yl)-3-(3-cyano-phenyl)-thiourea
- 1-(1-(6-Chloro-quinolin-4-yl)-piperidin-4-yl)-3-(4-cyano-phenyl)-thiourea
- 1-(1-(6-Chloro-quinolin-4-yl)-piperidin-4-yl)-3-(2-methylsulfanyl-phenyl)-thiourea
- 1-(1-(6-Chloro-quinolin-4-yl)-piperidin-4-yl)-3-(2,3-dichloro-phenyl)-thiourea
- 1-(1-(6-Chloro-quinolin-4-yl)-piperidin-4-yl)-3-(3-methylsulfanyl-phenyl)-thiourea
- 3-(3-(1-(6-Chloro-quinolin-4-yl)-piperidin-4-yl)-thioureido)-benzoic acid methyl ester
- 1-(1-(6-Chloro-quinolin-4-yl)-piperidin-4-yl)-3-(2-trifluoromethoxy-phenyl)-thiourea
- 1-[1-(6-Bromo-phthalazin-1-yl)-piperidin-4-yl]-3-(4-methylsulfanyl-phenyl)-thiourea
- 1-[1-(6-Bromo-phthalazin-1-yl)-piperidin-4-yl]-3-phenyl-thiourea 1-(3-Chloro-phenyl)-3-[1-(6-bromo-phthalazin-1-yl)-piperidin-4-yl]-thiourea
- 1-(2-Chloro-phenyl)-3-[1-(6-bromo-phthalazin-1-yl)-piperidin-4-yl]-thiourea
- 1-(1-(6-Bromo-phthalazin-1-yl)-piperidin-4-yl)-3-(4-methoxy-phenyl)-thiourea
- 1-(1-(6-Bromo-phthalazin-1-yl)-piperidin-4-yl)-3-p-tolyl-thiourea
- 1-(1-(6-Bromo-phthalazin-1-yl)-piperidin-4-yl)-3-o-tolyl-thiourea
- 1-(2-Bromo-phenyl)-3-(1-(6-bromo-phthalazin-1-yl)-piperidin-4-yl)-thiourea
- 1-(3-Bromo-phenyl)-3-(1-(6-bromo-phthalazin-1-yl)-piperidin-4-yl)-thiourea
- 1-(1-(6-Bromo-phthalazin-1-yl)-piperidin-4-yl)-3-(3-methoxy-phenyl)-thiourea
- 4-(3-(1-(6-Bromo-phthalazin-1-yl)-piperidin-4-yl)-thioureido)-benzoic acid methyl ester
- 1-(1-(6-Bromo-phthalazin-1-yl)-piperidin-4-yl)-3-(3-cyano-phenyl)-thiourea
- 1-(1-(6-Bromo-phthalazin-1-yl)-piperidin-4-yl)-3-(4-cyano-phenyl)-thiourea
- 1-(1-(6-Bromo-phthalazin-1-yl)-piperidin-4-yl)-3-(2-methylsulfanyl-phenyl)-thiourea
- 1-(1-(6-Bromo-phthalazin-1-yl)-piperidin-4-yl)-3-(2,3-dichloro-phenyl)-thiourea
- 1-(1-(6-Bromo-phthalazin-1-yl)-piperidin-4-yl)-3-(3-methylsulfanyl-phenyl)-thiourea
- 3-(3-(1-(6-Bromo-phthalazin-1-yl)-piperidin-4-yl)-thioureido)-benzoic acid methyl ester
- 1-(1-(6-Bromo-phthalazin-1-yl)-piperidin-4-yl)-3-(2-trifluoromethoxy-phenyl)-thiourea
- 1-[1-(7-Bromo-phthalazin-1-yl)-piperidin-4-yl]-3-(4-methylsulfanyl-phenyl)-thiourea
- 1-[1-(7-Bromo-phthalazin-1-yl)-piperidin-4-yl]-3-(4-methylsulfanyl-phenyl)-thiourea
- 1-[1-(7-Bromo-phthalazin-1-yl)-piperidin-4-yl]-3-phenyl-thiourea
- 1-(3-Chloro-phenyl)-3-[1-(7-bromo-phthalazin-1-yl)-piperidin-4-yl]-thiourea
- 1-(2-Chloro-phenyl)-3-[1-(7-bromo-phthalazin-1-yl)-piperidin-4-yl]-thiourea
- 1-(1-(7-Bromo-phthalazin-1-yl)-piperidin-4-yl)-3-(4-methoxy-phenyl)-thiourea
- 1-(1-(7-Bromo-phthalazin-1-yl)-piperidin-4-yl)-3-p-tolyl-thiourea
- 1-(1-(7-Bromo-phthalazin-1-yl)-piperidin-4-yl)-3-o-tolyl-thiourea
- 1-(2-Bromo-phenyl)-3-(1-(7-bromo-phthalazin-1-yl)-piperidin-4-yl)-thiourea
- 1-(3-Bromo-phenyl)-3-(1-(7-bromo-phthalazin-1-yl)-piperidin-4-yl)-thiourea
- 1-(1-(7-Bromo-phthalazin-1-yl)-piperidin-4-yl)-3-(3-methoxy-phenyl)-thiourea
- 4-(3-(1-(7-Bromo-phthalazin-1-yl)-piperidin-4-yl)-thioureido)-benzoic acid methyl ester
- 1-(1-(7-Bromo-phthalazin-1-yl)-piperidin-4-yl)-3-(3-cyano-phenyl)-thiourea
- 1-(1-(7-Bromo-phthalazin-1-yl)-piperidin-4-yl)-3-(4-cyano-phenyl)-thiourea
- 1-(1-(7-Bromo-phthalazin-1-yl)-piperidin-4-yl)-3-(2-methylsulfanyl-phenyl)-thiourea
- 1-(1-(7-Bromo-phthalazin-1-yl)-piperidin-4-yl)-3-(2,3-dichloro-phenyl)-thiourea
- 1-(1-(7-Bromo-phthalazin-1-yl)-piperidin-4-yl)-3-(3-methylsulfanyl-phenyl)-thiourea
- 3-(3-(1-(7-Bromo-phthalazin-1-yl)-piperidin-4-yl)-thioureido)-benzoic acid methyl ester
- 1-(1-(7-Bromo-phthalazin-1-yl)-piperidin-4-yl)-3-(2-trifluoromethoxy-phenyl)-thiourea
- 1-[1-(7-Chloro-quinazolin-4-yl)-piperidin-4-yl]-3-(4-methylsulfanyl-phenyl)-thiourea
- 1-[1-(7-Chloro-quinazolin-4-yl)-piperidin-4-yl]-3-phenyl-thiourea
- 1-(3-Chloro-phenyl)-3-[1-(7-chloro-quinazolin-4-yl)-piperidin-4-yl]-thiourea
- 1-(2-Chloro-phenyl)-3-[1-(7-chloro-quinazolin-4-yl)-piperidin-4-yl]-thiourea
- 1-(1-(7-Chloro-quinazolin-4-yl)-piperidin-4-yl)-3-(4-methoxy-phenyl)-thiourea
- 1-(1-(7-Chloro-quinazolin-4-yl)-piperidin-4-yl)-3-p-tolyl-thiourea
- 1-(1-(7-Chloro-quinazolin-4-yl)-piperidin-4-yl)-3-o-tolyl-thiourea
- 1-(2-Bromo-phenyl)-3-(1-(7-chloro-quinazolin-4-yl)-piperidin-4-yl)-thiourea
- 1-(3-Bromo-phenyl)-3-(1-(7-chloro-quinazolin-4-yl)-piperidin-4-yl)-thiourea
- 1-(1-(7-Chloro-quinazolin-4-yl)-piperidin-4-yl)-3-(3-methoxy-phenyl)-thiourea
- 4-(3-(1-(7-Chloro-quinazolin-4-yl)-piperidin-4-yl)-thioureido)-benzoic acid methyl ester
- 1-(1-(7-Chloro-quinazolin-4-yl)-piperidin-4-yl)-3-(3-cyano-phenyl)-thiourea
- 1-(1-(7-Chloro-quinazolin-4-yl)-piperidin-4-yl)-3-(4-cyano-phenyl)-thiourea
- 1-(1-(7-Chloro-quinazolin-4-yl)-piperidin-4-yl)-3-(2-methylsulfanyl-phenyl)-thiourea
- 1-(1-(7-Chloro-quinazolin-4-yl)-piperidin-4-yl)-3-(2,3-dichloro-phenyl)-thiourea
- 1-(1-(7-Chloro-quinazolin-4-yl)-piperidin-4-yl)-3-(3-methylsulfanyl-phenyl)-thiourea
- 3-(3-(1-(7-Chloro-quinazolin-4-yl)-piperidin-4-yl)-thioureido)-benzoic acid methyl ester
- 1-(1-(7-Chloro-quinazolin-4-yl)-piperidin-4-yl)-3-(2-trifluoromethoxy-phenyl)-thiourea
- 1-(1-Isoquinolin-1-yl-piperidin-4-yl)-3-(4-methylsulfanyl-phenyl)-thiourea
- 1-(1-Isoquinolin-1-yl-piperidin-4-yl)-3-phenyl-thiourea
- 1-(3-Chloro-phenyl)-3-(1-lsoquinolin-1-yl-piperidin-4-yl)-thiourea
- 1-(2-Chloro-phenyl)-3-(1-Isoquinolin-1-yl-piperidin-4-yl)-thiourea
- 1-(1-Isoquinolin-1-yl-piperidin-4-yl)-3-(4-methoxy-phenyl)-thiourea
- 1-(1-Isoquinolin-1-yl-piperidin-4-yl)-3-p-tolyl-thiourea
- 1-(1-Isoquinolin-1-yl-piperidin-4-yl)-3-o-tolyl-thiourea
- 1-(2-Bromo-phenyl)-3-(1-Isoquinolin-1-yl-piperidin-4-yl)-thiourea
- 1-(3-Bromo-phenyl)-3-(1-Isoquinolin-1-yl-piperidin-4-yl)-thiourea
- 1-(1-Isoquinolin-1-yl-piperidin-4-yl)-3-(3-methoxy-phenyl)-thiourea
- 4-(3-(1-Isoquinolin-1-yl-piperidin-4-yl)-thioureido)-benzoic acid methyl ester
- 1-(1-Isoquinolin-1-yl-piperidin-4-yl)-3-(3-cyano-phenyl)-thiourea
- 1-(1-Isoquinolin-1-yl-piperidin-4-yl)-3-(4-cyano-phenyl)-thiourea
- 1-(1-Isoquinolin-1-yl-piperidin-4-yl)-3-(2-methylsulfanyl-phenyl)-thiourea
- 1-(1-Isoquinolin-1-yl-piperidin-4-yl)-3-(2,3-dichloro-phenyl)-thiourea
- 1-(1-Isoquinolin-1-yl-piperidin-4-yl)-3-(3-methylsulfanyl-phenyl)-thiourea
- 3-(3-(1-Isoquinolin-1-yl-piperidin-4-yl)-thioureido)-benzoic acid methyl ester
- 1-(1-Isoquinolin-1-yl-piperidin-4-yl)-3-(2-trifluoromethoxy-phenyl)-thiourea

Gegenstand der vorliegenden Erfindung ist die Verwendung der erfindungsgemäßen Verbindungen für die Herstellung von Arzneimitteln, die mindestens eine der Verbindungen gemäß Formel I enthalten.

Ebenfalls Gegenstand der vorliegenden Erfindung sind Arzneimittel, die die erfindungsgemäßen Verbindungen enthalten mit geeigneten Formulierungs-und Trägerstoffen.

Im Vergleich zu bekannten Prostaglandin E₂-Liganden zeichnen sich die neuen EP₂-Agonisten und Antagonisten durch größere Selektivität und Stabilität aus.

Gegenstand der vorliegenden Erfindung sind Arzneimittel zur Behandlung und Prophylaxe von Erkrankungen, zu denen Fertilitätsstörungen, infektiöse Erkrankungen, Krebs, virale Infektionen, Herz-Kreislauf-Erkrankungen, erhöhter Augeninnendruck, Glaukoma, Erkrankungen des Knochenapparates, angiogenetische Erkrankungen, Störungen der Uteruskontraktion, Schmerz, neuroinflammatorische Erkrankungen, immunomodulatorische Infektionen und nephrologische Erkrankungen zählen.

Unter Fertilitätsstörungen sind die Erkrankungen zu verstehen, die dazu führen, dass keine Ovulation erfolgt, dass es nicht zur Nidation einer befruchteten Eizelle kommt und keine Dezidualisierung stattfindet, unter infektiösen Erkrankungen sind durch unizelluläre Parasiten hervorgerufene Erkrankungen, unter Krebs solide Tumoren und Leukämie, unter viralen Infektionen z. B. Cytomegalus-Infektionen, Hepatitis, Hepatitis B und C und HIV-Erkrankungen, unter immunmodulatorischen Infektionen z.B. Vogelgrippe, unter Herz-Kreislauf-Erkrankungen ischämische Reperfusionserkrankung, Stenosen, Arteriosklerosen und Restenosen, unter angiogenetischen Erkrankungen z.B. Endometriose und Fibrose, unter erhöhtem Augeninnendruck Glaukom, unter Störungen der Uteruskontraktion z.B. Menstruationsbeschwerden, unter Erkrankungen des Knochenapparates Osteoporose, unter neuroinflammatorischen Erkrankungen Multiple Sklerose, Morbus Alzheimer, Schmerz und unter nephrologischen Erkrankungen Glomerulonephritis, zu verstehen.

Ebenfalls Gegenstand der vorliegenden Erfindung sind Arzneimittel zur Behandlung und Prophylaxe der oben aufgeführten Erkrankungen, die mindestens eine Verbindung gemäß der allgemeinen Formel I enthalten, sowie Arzneimittel mit geeigneten Formulierungs- und Trägerstoffen.

Zur Verwendung der erfindungsgemäßen Verbindungen als Arzneimittel werden diese in die Form eines pharmazeutischen Präparats gebracht, das neben dem Wirkstoff für die enterale oder parenterale Applikation geeignete pharmazeutische, organische oder anorganische inerte Trägermaterialien, wie zum Beispiel, Wasser, Gelatine, Gummi arabicum, Milchzucker, Stärke, Magnesiumstearat, Talk, pflanzliche Öle, Polyalkylenglykole usw. enthält. Die pharmazeutischen Präparate können in fester Form, zum Beispiel als Tabletten, Dragees, Suppositorien, Kapseln, in halbfester Form, zum Beispiel als Salben, Cremes, Gele, Suppositiorien, Emulsionen oder in flüssiger Form, zum Beispiel als Lösungen, Suspensionen oder Emulsionen vorliegen.

Gegebenenfalls enthalten sie Hilfsstoffe, die beispielsweise als Füllstoffe, Bindemeittel, Sprengmittel, Gleitmittel, Lösungsmittel, Lösungsvermittler, Geschmackskorrigentien, Farbstoff, Emulgatoren, fungieren sollen. Hilfsstoffarten im Sinne der Erfindung sind beispielsweise Saccharide (Mono-, Di-, Tri-, Oligo-, und/oder Polysaccharide), Fette, Wachse, Öle, Kohlenwasserstoffe, anionische, nichtionische, kationische natürliche, synthetische oder halbsynthestische Tenside. Gegebenenfalls enthalten sie darüber hinaus Hilfsstoffe, wie Konservierungs-, Stabilisierungs-, Netzmittel oder Emulgatoren; Salze zur Veränderung des osmotischen Drucks oder Puffer. Diese pharmazeutischen Präparate sind ebenfalls Gegenstand der vorliegenden Erfindung.

Zur Inhalation werden zweckmäßigerweise Aerosollösungen hergestellt.

Für die orale Anwendung sind insbesondere Tabletten, Dragees oder Kapseln mit Talkum und/oder Kohlenwasserstoffträger oder -binder, wie zum Beispiel Lactose, Mais- oder Kartoffelstärke, geeignet. Die Anwendung kann auch in flüssiger Form erfolgen, wie zum Beispiel als Saft, dem gegebenenfalls ein Süßstoff beigefügt ist. Für die orale Anwendung derartiger Verbindungen sind ebenfalls auch Clathrate geeignet, beispielsweise seien genannt die Clathrate mit alpha-, beta-, gamma-Cyclodextrin oder auch beta-hydroxypropyl-Cyclodextrin.

Für die parenterale Verabreichung werden sterile, injizierbare, wässrige oder ölige Lösungen benutzt. Besonders geeignet sind Injektionslösungen oder Suspensionen, insbesondere wässrige Lösungen der aktiven Verbindungen in polyethoxyliertem Rizinusöl.

Für die vaginale Applikation sind z. B. Zäpfchen, Tampons oder intrauteriner Device geeignet und üblich.

Für die intraartikuläre Injektion können entsprechend zubereitete Kristallsuspensionen verwendet werden.

Für die intramuskuläre Injektion können wässrige und ölige Injektionslösungen oder Suspensionen und entsprechende Depotpräparationen Verwendung finden.

Für die rektale Applikation können die neuen Verbindungen in Form von Suppositorien, Kapseln, Lösungen (z.B. in Form von Klysmen) und Salben sowohl zur systemischen, als auch zur lokalen Therapie verwendet werden.

Zur pulmonalen Applikation der neuen Verbindungen können diese in Form von Aerosolen und Inhalaten verwendet werden.

Für die lokale Anwendung an Augen, äußerem Gehörgang, Mittelohr, Nasenhöhle und Nasennebenhöhlen können die neuen Verbindungen als Tropfen, Salben und Tinkturen in entsprechenden pharmazeutischen Zubereitungen verwendet werden.

Für die topische Auftragung sind Formulierungen in Gelen, Salben, Fettsalben, Cremes, Pasten, Puder, Milch und Tinkturen möglich. Die Dosierung der Verbindungen der allgemeinen Formel I sollte in diesen Zubereitungen 0.01 % - 20 % betragen, um eine ausreichende pharmakologische Wirkung zu erzielen.

Als Trägersysteme können auch grenzflächenaktive Hilfsstoffe, wie Salze der Gallensäuren oder tierische oder pflanzliche Phospholipide, aber auch Mischungen davon sowie Liposome oder deren Bestandteile verwendet werden.

Die Dosierung der Wirkstoffe kann je nach Verabfolgungsweg, Alter und Gewicht des Patienten, Art und Schwere der zu behandelnden Erkrankung und ähnlichen Faktoren variieren. Die Behandlung kann durch Einzeldosierungen oder durch eine Vielzahl von Dosierungen über einen längeren Zeitraum erfolgen. Die tägliche Dosis beträgt 0,5 - 1000 mg, vorzugsweise 50 - 200 mg, wobei die Dosis als einmal zu verabreichende Einzeldosis oder unterteilt in 2 oder mehrere Tagesdosen gegeben werden kann.

Die oben beschrieben Formulierungen und Darreichungsformen sind ebenfalls Gegenstand der vorliegenden Erfindung.

Als Trägersysteme können auch grenzflächenaktive Hilfsstoffe, wie Salze der Gallensäuren oder tierische oder pflanzliche Phospholipide, aber auch Mischungen davon sowie Liposomen oder deren Bestandteile verwendet werden.

Die Administration der erfindungsgemäßen Verbindungen kann durch jede konventionelle Methode erfolgen, einschließlich oraler und parenteraler, z.B. durch subcutane oder intramuskuläre Injektionen. Die enteralen, parenteralen, vaginalen und oralen Applikationen sind ebenfalls Gegenstand der vorliegenden Erfindung.

Die erfindungsgemäßen Verbindungen der allgemeinen Formel I binden an den EP₂-Rezeptor und haben agonistische oder antagonistische Wirkung. Es kann durch einen Agonismustest (siehe Beispiel 1.2.1. der biologischen Beispiele) oder durch einen Antagonismustest (siehe Beispiel 1.2.2. der biologischen Beispiele) bestimmt werden, ob eine agonistische oder antagonistische Wirkung vorliegt.

Unter Antagonisten sind solche Moleküle zu verstehen, die an ihre entsprechenden Rezeptoren binden und üblicherweise mit dem natürlich vorkommenden Liganden des Rezeptors um die Bindung an den Rezeptor kompetitieren und welche die Initiierung des mit dem Rezeptor gekoppelten Signaltransduktionsweges inhibieren.

Rezeptorantagonisten binden typischerweise selektiv an ihren bestimmten Rezeptor und nicht an andere Rezeptoren. Sie weisen normalerweise eine höhere Bindungsaffinität als der natürliche Ligand auf. Obwohl Antagonisten, die eine höhere Affinität zum Rezeptor haben als der natürliche Ligand, bevorzugt sind, können ebenfalls Antagonisten mit einer geringeren Affinität eingesetzt werden.

Bevorzugterweise binden die Antagonisten reversibel an ihre korrespondierenden Rezeptoren.

Der EP₂-Rezeptor Antagonist hat eine bevorzugte Affinität für den EP₂ Rezeptor gegenüber jedem anderen EP-Rezeptor. Der Antagonismus wird in Gegenwart des natürlichen Agonisten gemessen (PGE₂).

Unter Agonisten sind solche Moleküle zu verstehen, die an ihre entsprechenden Rezeptoren binden und üblicherweise mit dem natürlich vorkommenden Liganden des Rezeptors um die Bindung an den Rezeptor kompetitieren und welche die Initiierung des mit dem Rezeptor gekoppelten Signaltransduktionsweges stimulieren. Agonisten können auch die Bindung des natürlichen Liganden unterstützen.

Rezeptoragonisten binden typischerweise selektiv an ihren bestimmten Rezeptor und nicht an andere Rezeptoren. Sie haben normalerweise eine höhere Bindungsaffinität als der natürliche Ligand. Obwohl Agonisten, die eine höhere Affinität zum Rezeptor haben als der natürliche Ligand, bevorzugt sind, können ebenfalls Agonisten mit einer geringeren Affinität eingesetzt werden.

Bevorzugterweise binden die Agonisten reversibel an ihre korrespondierenden Rezeptoren.

Agonisten werden über die Initiierung der entspechenden Rezeptor vermittelten Singaltransduktion und/oder physiologischen Wirkung getestet.

Als Liganden werden die Verbindungen oder niedermolekulare Substanzen bezeichnet, die an einen Rezeptor binden. Ihre Bindung ist üblicherweise reversibel. Durch die Bindung eines Liganden an den entsprechenden Rezeptor wird der mit dem Rezeptor gekoppelte Signaltransduktionsweg aktiviert oder inaktiviert. Auf diese Art und Weise vermittelt der Ligand seine intrazelluläre Wirkung. Unter Liganden sind Agonisten und Antagonisten eines Rezeptors zu verstehen.

Die Substanz gemäß Beispiel 8 zeigt im zellulären Agonismustest keine Inhibition, im Antagonismustest jedoch eine gute Wirksamkeit (IC₅₀ = 3,8 x 10 E-6).

Ebenfalls Gegenstand der vorliegenden Erfindung ist die Verwendung der erfindungsgemäßen Substanzen als EP₂-Rezeptor Antagonisten für die Behandlung von Erkrankungen, die verursacht werden durch Störungen in der Signaltransduktionskette, an der der EP₂-Rezeptor beteiligt ist, wie beispielsweise Schmerz und Fertilitätsstörungen, und die ebenfalls geeignet sind für die Fertilitätskontrolle.

Die erfindungsgemäßen Verbindungen der allgemeinen Formel I haben profertile Wirkung. Im prä-ovulatorischen antralen Follikel ist die Eizelle von Kumulus Zellen umgeben, die einen dichten Zellkranz um die Eizelle bilden. Nach dem Peak des Lutenisierenden Hormons (LH-Peak) wird eine Reihe von Prozessen aktiviert, die eine starke morphologische Veränderung dieses Zellkranzes aus Kumulus Zellen zur Folge hat. Hierbei bilden die Kumulus Zellen eine extrazelluläre Matrix, die zur sogenannten Kumulus Expansion führt (Vanderhyden et al. Dev Biol. 1990 Aug;140(2):307-317). Diese Kumulus Expansion ist ein wichtiger Bestandteil des ovulatorischen Prozesses und der nachfolgenden Möglichkeit zur Fertilisation.

Bei der Kumulus Expansion sind Prostaglandine und hier das Prostaglandin E₂, dessen Synthese durch den LH-Peak induziert wird, von entscheidender Bedeutung. Prostanoid EP₂ Knock-out Mäuse (Hizaki et al., Proc Natl Acad Sci U S A. 1999 Aug 31;96(18):10501-6.) zeigen eine deutlich verminderte Kumulus Expansion und starke Subfertilität, was die Bedeutung des Prostanoid EP₂-Rezeptors für diesen Prozess demonstriert.

Die erfindungsgemäßen Substanzen haben inhibitorische Wirkungen in Kumulus Expansionstests.

Gegenstand der vorliegenden Erfindung ist die Verwendung der erfindungsgemäßen Substanzen zur Fertilitätskontrolle.

Während der EP₂-Rezeptor Antagonist AH 6809 die Expansion des Kumulus erst bei einer Konzentration von 100 - 200 µM um nur ca 30 % unterdrückt, kann in Anwesenheit von der Substanz gemäß Beispiel 8 eine 20 % Unterdrückung der Kumulus Expansion bei einer 10fach geringeren Konzentration erreicht werden. Bei diesen Versuchen kompetitieren die Testsubstanzen mit dem natürlichen EP₂-Rezeptor Agonisten PGE₂.

Gegenstand der vorliegenden Erfindung ist die Verwendung der erfindungsgemäßen Substanzen für die Hemmung der Kumulus Expansion und dadurch der Ovualtion und Fertilisation zur Kontrazeption.

Prostaglandine spielen eine wichtige Rolle bei der Angiogenese (Sales, Jabbour, 2003, Reproduction 126, 559 - 567).

Endometriose ist eine chronische Erkrankung, die verursacht wird durch Störungen der Blutgefäße. Circa 10 % der Frauen leiden regelmäßig an starken Blutungen während der Menstruation, verursacht durch Änderungen der Blutgefäße des Endometriums. Zusätzlich wurden strukturelle Unterschiede in den Blutgefäßen beobachtet, wie zum Beispiel die unvollständige Ausbildung der glatten Muskelzellschicht (Abberton et al., 1999, Hum. Reprod. 14, 1072-1079). Da der Blutverlust während der Menstruation zum Teil durch die Konstriktion der Blutgefäße geregelt wird, ist es naheliegend, dass die Defekte der glatten Muskulatur wesentlich zur Blutung beitragen.

Gegenstand der vorliegenden Erfindung ist die Verwendung der Substanzen der allgemeinen Formel I für die Behandlung von Endometriose.

Prostaglandine spielen eine wichtige Rolle bei der Uteruskontraktion, zu starke Kontraktionen sind verantwortlich für Menstruationsbeschwerden (Sales, Jabbour, 2003, Reproduction 126, 559 - 567).

Gegenstand der vorliegenden Erfindung ist die Verwendung der Substanzen der allgemeinen Formel I für die Behandlung von Menstruationsbeschwerden.

Prostaglandine spielen eine wichtige Rolle bei der Entstehung und Verlauf von verschiedenen Krebserkrankungen, (S.W. Han, Biochemical and Biophysical Research Communications 314 (2004) 1093-1099; S.-H. Chang; Cancer Research 65 (2005); 4496-9; M. D. Castellone, Science 310 (2005) 1504 - 1510).

Gegenstand der vorliegenden Erfindung ist die Verwendung der Substanzen der allgemeinen Formel I für die Behandlung und Vorbeugung von Krebserkrankungen.

Prostaglandine spielen auch eine wichtige Rolle bei den Prozessen, die dem Knochenschwund entgegen wirken. Gegenstand der vorliegenden Erfindung ist daher die Verwendung der erfindungsgemäßen Substanzen für die Behandlung von Knochenschwund.

Reinold et al. (J. Clin. Invest. 115, 673-679 (2005)) beschreibt PGE₂ Rezeptoren vom EP₂-Subtyp als die Schlüssel-Signalelemente bei der inflammatorischen Hyperalgesie. Mäuse, die diesen Rezeptor nicht mehr tragen (EP₂^{-/-}), empfinden keinen spinalen inflammatorischen Schmerz. Es gibt Hinweise, dass eine inflammatorische, gesteigerte Schmerzempfindlichkeit behandelt werden kann, indem gezielt EP₂-Rezeptoren moduliert werden.

Gegenstand der vorliegenden Erfindung ist die Verwendung der erfindungsgemäßen Substanzen für die Behandlung von inflammatorischer Hyperalgesie.

Soweit die Herstellung der Ausgangsverbindungen nicht beschrieben wird, sind diese bekannt oder analog zu bekannten Verbindungen oder hier beschriebenen Verfahren herstellbar. Es ist ebenfalls möglich, alle hier beschriebenen Umsetzungen in Parallel-Reaktoren oder mittels kombinatorischer Arbeitstechniken durchzuführen.

Die Herstellung der Salze erfolgt in üblicher Weise, indem man eine Lösung der Verbindung der Formel I mit der äquivalenten Menge oder einem Überschuss einer Base oder Säure, die gegebenenfalls in Lösung ist, versetzt und den Niederschlag abtrennt oder in üblicher Weise die Lösung aufarbeitet.

Die Erfindung betrifft damit auch Arzneimittel auf Basis der Verbindungen der allgemeinen Formel I und der üblichen Hilfs- oder Trägerstoffe.

Die erfindungsgemäßen Verbindungen der allgemeinen Formel I lassen sich wie in den Beispielen beschrieben herstellen. Durch analoge Vorgehensweise unter Verwendung homologer Reagenzien zu den in den Beispielen beschriebenen Reagenzien lassen sich die weiteren Verbindungen der allgemeinen Formel I erhalten.

Ausgehend von den Verbindungen der allgemeinen Formel IVa-d lassen sich die erfindungungsgemäßen Verbindungen der allgemeinen Formel I durch Umsetzung mit N-Piperidin-4-yl-(het)arylthioharnstoffen der allgemeinen Formel V nach dem Fachmann bekannten Verfahren herstellen. Ebenfalls lassen sich die erfindungungsgemäßen Verbindungen der allgemeinen Formel I durch Umsetzung von Verbindungen der allgemeinen Formel IVa-d zu Verbindungen der allgemeinen Formel IIIa-d und nachfolgend Formel IIa-d nach dem Fachmann bekannten Verfahren herstellen. Durch analoge Vorgehensweise unter Verwendung homologer Reagenzien zu den in den Beispielen beschriebenen Reagenzien lassen sich die weiteren Verbindungen der allgemeinen Formel I erhalten.

Die Reste R²-R⁵ der auf diese Weise erhaltenen Verbindungen der allgemeinen Formel I lassen sich nach dem Fachmann bekannten Methoden weiter zu vielfältigen funktionellen Gruppen und damit weiteren Verbindungen der allgemeinen Formel I umsetzen.

Zum Beispiel lässt sich ein Bromid mittels Palladium(0)-katalysierter Reaktionen nach dem Fachmann bekannten Methoden durch einen Aryl- bzw. Heteroarylring, ein substituiertes Alken bzw. Alkin, Amin oder eine Cyanogruppe ersetzen.

Eine als R²-R⁵ fungierende Carboxyfunktion, Cyanogruppe oder ein Amin lässt sich beispielsweise nach dem Fachmann bekannten Methoden in Ester und Amide der allgemeinen Formel I überführen.

Ebenso lassen sich zum Beispiel Esterfunktionen bzw. eine Cyanogruppe in Verbindungen der allgemeinen Formel I nach Reduktion zum Aldehyd nach dem Fachmann bekannten Methoden in weitere Olefine bzw. mit Alkyl- oder Arylresten substituierte sekundäre Alkohole umgesetzen. Ebenfalls lässt sich eine Cyanogruppe in Verbindungen der allgemeinen Formel I nach dem Fachmann bekannten Methoden in mit Alkyl- oder Arylresten substituierte Ketone umsetzen, die sich dann zu den entsprechenden sekundären Alkoholen reduzieren lassen oder aber nach dem Fachmann bekannten Methoden in mit Alkyl- oder Arylresten substituierte tertiäre Alkohole überführt werden.

Die soeben beschriebenen beispielhaften Umsetzungen der Reste R²-R⁵ in den erfindungsgemäßen Verbindungen der allgemeinen Formel I lassen sich auf die selbe Art und Weise von einem Fachmann an Verbindungen der allgemeinen Formel IIa-d und IIIa-d durchführen. Die so erhaltenen Verbindungen der allgemeinen Formel IIa-d und IIIa-d lassen sich dann wie beschrieben in die der Formel I überführen.

Die zur Herstellung der erfindungsgemäßen Verbindungen der allgemeinen Formel I verwendeten Verbindungen der allgemeinen Formel IVa-d lassen sich nach dem Fachmann bekannten Verfahren in Abängigkeit von den Resten X, Y, Z und W herstellen.

Im Fall von X = Kohlenstoff, Y = CH, Z = Stickstoff und W = Wasserstoff geschieht dies nach dem Fachmann bekannten Verfahren, beispielsweise ausgehend von den Phthaliden der allgemeinen Formel X über die 2-Carboxymethylbenzoesäuren der allgemeinen Formel IX und Alkoxymethylidenisochroman-1,3-dione der allgemeinen Formel VIII zu den Isochinolinonen der allgemeinen Formeln VII und weiter zu denen Verbindungen der allgemeinen Formel IVd.

Im Fall von Y = CH, X und Z = jeweils Stickstoff geschieht dies nach dem Fachmann bekannten Verfahren, beispielsweise ausgehend von 2-Aminobenzoesäuren der allgemeinen Formel XII über die Chinazolinone der allgemeinen Formeln XI und weiter zu denen Verbindungen der allgemeinen Formel IVc.

Im Fall von X = Kohlenstoff, Y und Z = jeweils Stickstoff und W = Wasserstoff geschieht dies nach dem Fachmann bekannten Verfahren, beispielsweise ausgehend von den Phthaliden der allgemeinen Formel X bzw. Phthalsäureanhydriden der allgemeinen Formel XVII über die Phthalazine der allgemeinen Formeln XIII zu denen der allgemeinen Formel IVb. Im Falle von W = C₁-C₃-Alkyl geschieht dies ausgehend von den Phthalsäureanhydriden der allgemeinen Formel XVII über die 3-Hydroxy-3-alkyl-3H-isobenzofuran-1-one der allgemeinen Formel XV bzw. die 3-Alkyliden-3H-isobenzofuran-1-one der allgemeinen Formel XVI zu den Phthalazinen der allgemeinen Formeln XIII und weiter zu denen der allgemeinen Formel IVb.

Im Fall von Y, Z = jeweils CH und X = Stickstoff geschieht dies nach dem Fachmann bekannten Verfahren, beispielsweise ausgehend von Anilinen der allgemeinen Formel XXII über die Verbindungen der allgemeinen Formel XXI und die 3-Carboxychinoline der allgemeinen Formel XX zu denen der allgemeinen Formel XIX. Diese werden nach dem den Fachmann bekannten Verfahren zu Chinolinen der allgemeinen Formeln XVIII und weiter zu denen Verbindungen der allgemeinen Formel IVa umgesetzt.

Die zur Herstellung der erfindungsgemäßen Verbindungen der allgemeinen Formel I verwendeten N-Piperidin-4-yl-(het)arylthioharnstoffe der allgemeinen Formel V lassen sich nach dem Fachmann bekannten Methoden ausgehend von 4-Amino-piperidin-1-carbonsäure-tert-butylester über die Thioharnstoffe der allgemeinen Formel VI darstellen.

### Häufig verwendete Abkürzungen:

- DMF: N, N-Dimethylformamid
- Äquiv.: Äquivalente
- DMAP: 4-Dimethylamino-pyridin

### Allgemeine Synthesevorschrift

Das entsprechende Amin IIa-d (1 Äquiv.) wird vorgelegt und mit DMF (2 ml/mmol) versetzt. Dazu wird nacheinander das entsprechende Isothiocyanat (1.1 Äquiv.) in DMF (2 ml/mmol) und DMAP (1.1 Äquiv.) in DMF (1 mL/mmol) gegeben und die Mischung 12 h bei 100 °C Badtemperatur gerührt. Zur Aufarbeitung wird abgekühlt, mit Methanol (5 mL/mmol) versetzt und im Vakuum eingeengt. Der Rückstand wird mit präparativer HPLC-MS aufgereinigt und die Produkte mittels analytischer HPLC-MS charakterisiert [Methode 1: Säule HiBar RT 125-4 (125x4.5 mm, RP18e, 5 µm), Gradient 5-95% Acetonitril (0.1 % Trifluoressigsäure) in Wasser (0.1 % Trifluoressigsäure) (10 min.), Flußrate 1.0 mL/min, MS ES+; Methode 2: Säule Aquity UPLC BEH (2,1x50mm C18 1,7 um), Gradient: Start 98 % A (Wasser + 0,05 % Ameisensäure) + 2 % B (Acetonitril + 0,05 % Ameisensäure), in 1,7 min to 10 % A + 90 % B, 0,2 min isocratic, Flußrate: 1,3 ml/min, MS ES+].

| Beispiel | Struktur | Name | MW ber. | MW ES+ | RT [min., (Methode)] |
|---|---|---|---|---|---|
| 1 | 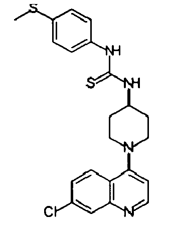 | 1-[1-(7-Chloroquinolin-4-yl)-piperidin-4-yl]-3-(4-methylsulfanylphenyl)-thiourea | 443.036 | 443 | 0.94 (2) |
| 2 | 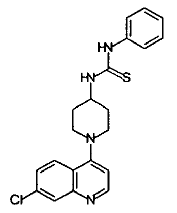 | 1-[1-(7-Chloroquinolin-4-yl)-piperidin-4-yl]-3-phenyl-thiourea | 396.943 | 397 | 0,86 (2) |
| 3 | 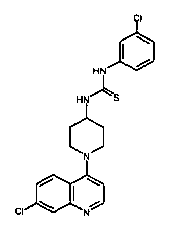 | 1-(3-Chlorophenyl)-3-[1-(7-chloro-quinolin-4-yl)-piperidin-4-yl]-thiourea | 431.388 | 431 | 7.62(1) |
| 4 | 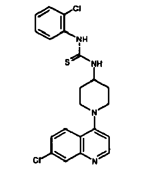 | 1-(2-Chlorophenyl)-3-[1-(7-chloro-quinolin-4-yl)-piperidin-4-yl]-thiourea | 431.388 | 431 | 7.24 (1) |
| 5 | 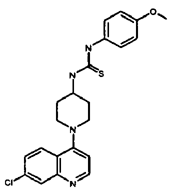 | 1-(1-(7-Chloroquinolin-4-yl)-piperidin-4-yl)-3-(4-methoxyphenyl)-thiourea | 426.97 | 427 | 6.90 (1) |
| 6 | 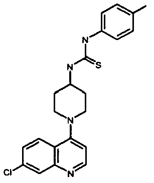 | 1-(1-(7-Chloroquinolin-4-yl)-piperidin-4-yl)-3-p-tolyl-thiourea | 410.97 | 411 | 7.24 (1) |
| 7 | 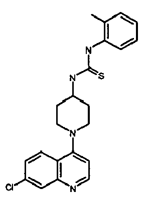 | 1-(1-(7-Chloroquinolin-4-yl)-piperidin-4-yl)-3-o-tolyl-thiourea | 410.97 | 411 | 6.99 (1) |
| 8 | 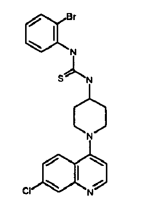 | 1-(2-Bromophenyl)-3-(1-(7-chloro-quinolin-4-yl)-piperidin-4-yl)-thiourea | 475.839 | 477 | 7.19 (1) |
| 9 | 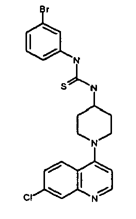 | 1-(3-Bromophenyl)-3-(1-(7-chloro-quinolin-4-yl)-piperidin-4-yl)-thiourea | 475.839 | 477 | 7.70 (1) |
| 10 | 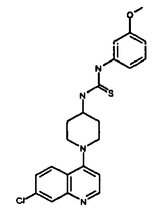 | 1-(1-(7-Chloroquinolin-4-yl)-piperidin-4-yl)-3-(3-methoxy-phenyl)-thiourea | 426.97 | 427 | 7.12(1) |
| 11 | 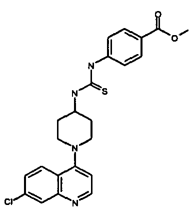 | 4-(3-(1-(7-Chloroquinolin-4-yl)-piperidin-4-yl)-thioureido)-benzoic acid methyl ester | 454.98 | 455 | 7.22(1) |
| 12 | 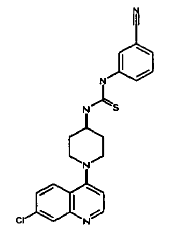 | 1-(1-(7-Chloroquinolin-4-yl)-piperidin-4-yl)-3-(3-cyano-phenyl)-thiourea | 421.953 | 422 | 7.09(1) |
| 13 | 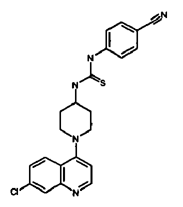 | 1-(1-(7-Chloroquinolin-4-yl)-piperidin-4-yl)-3-(4-cyano-phenyl)-thiourea | 421.953 | 422 | 7.16 (1) |
| 14 | 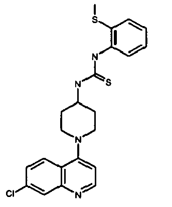 | 1-(1-(7-Chloroquinolin-4-yl)-piperidin-4-yl)-3-(2-methylsulfanylphenyl)-thiourea | 443.036 | 443 | 7.18 (1) |
| 15 | 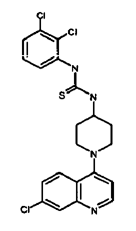 | 1-(1-(7-Chloroquinolin-4-yl)-piperidin-4-yl)-3-(2,3-dichloro-phenyl)-thiourea | 465.833 | 467 | 7.76(1) |
| 16 | 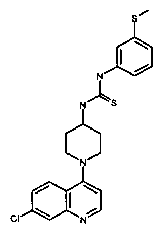 | 1-(1-(7-Chloro-quinolin-4-yl)-piperidin-4-yl)-3-(3-methylsulfanyl-phenyl)-thiourea | 443.036 | 443 | 7.47(1) |
| 17 | 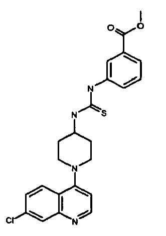 | 3-(3-(1-(7-Chloroquinolin-4-yl)-piperidin-4-yl)-thioureido)-benzoic acid methyl ester | 454.98 | 455 | 7.14(1) |
| 18 | 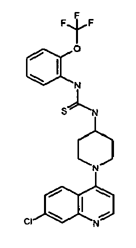 | 1-(1-(7-Chloroquinolin-4-yl)-piperidin-4-yl)-3-(2-trifluoromethoxyphenyl)-thiourea | 480.941 | 481 | 7.70(1) |

### Syntheseschemata

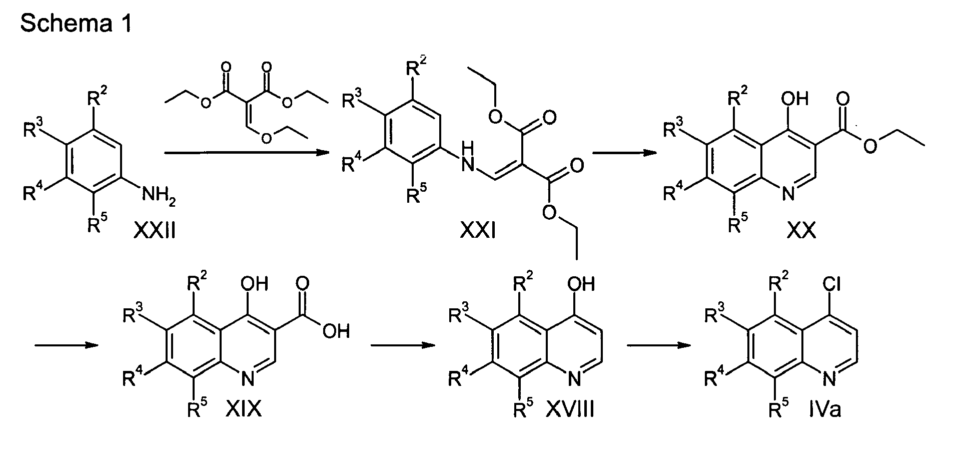

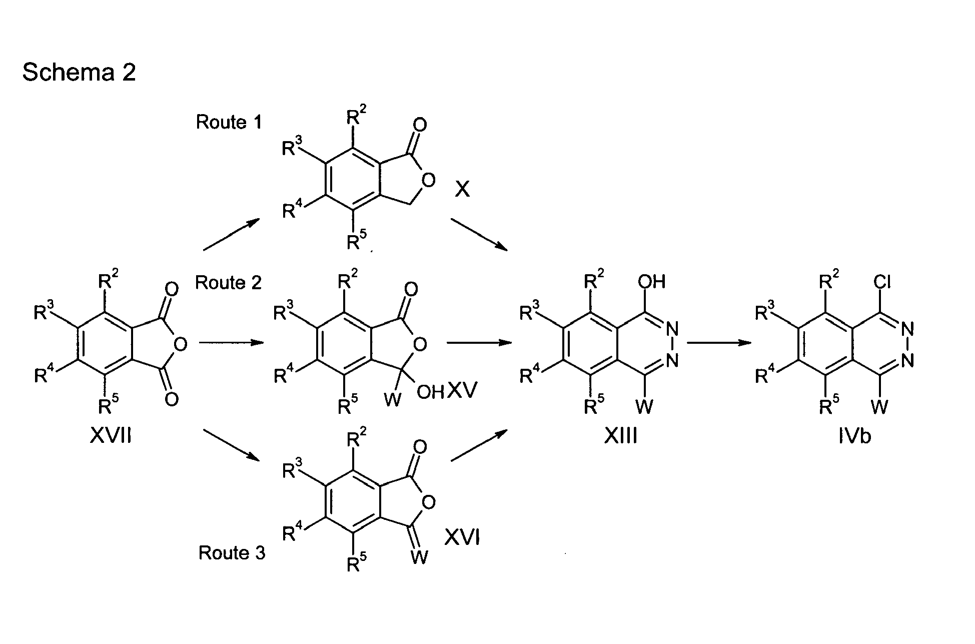

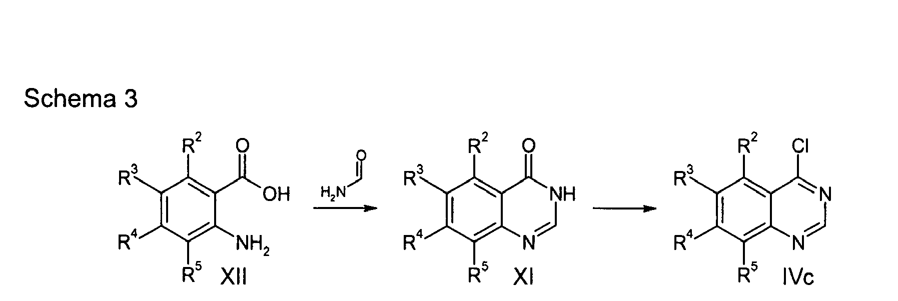

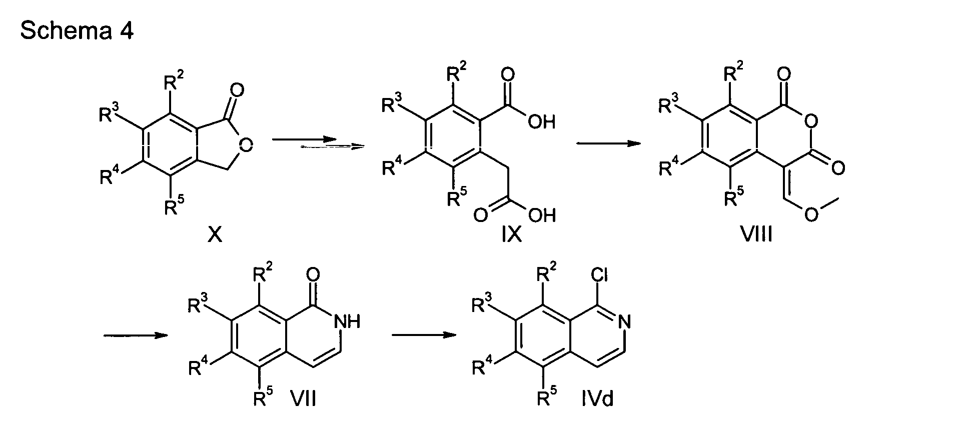

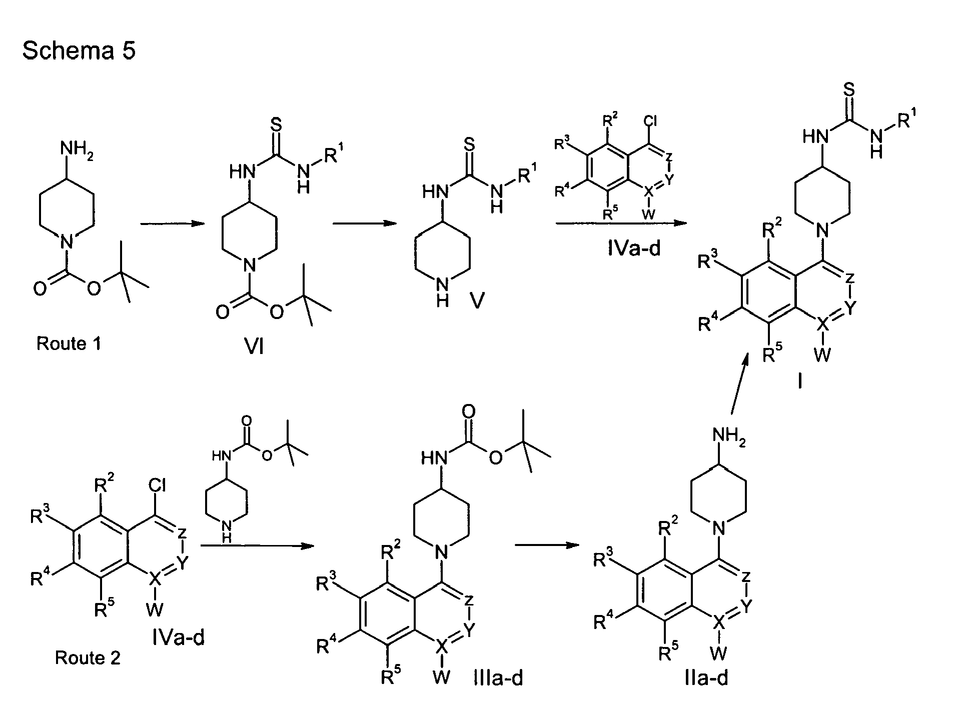

### Biologische Beispiele:

### 1. Nachweis des Antagonismus des humanen Prostaglandin E₂ (Subtyp EP₂) Rezeptorsignals

### 1.1 Nachweisprinzip

Die Bindung von PGE₂ an den EP₂-Subtyp des humanen PGE₂-Rezeptors induziert die Aktivierung membranständiger Adenylatzyklasen und führt zur Bildung von cAMP. In Gegenwart des Phosphodiesteraseinhibitors IBMX wird das durch diese Stimulation akkumulierte und mittels Zell-Lyse freigesetzte cAMP in einem kompetitiven Nachweisverfahren eingesetzt. In diesem Test konkurriert das cAMP im Lysat mit cAMP-XL665 um die Bindung eines Eu-Kryptat markierten Anti-cAMP Antikörpers.
Dabei entsteht in Abwesenheit von zellulärem cAMP ein maximales Signal, welches auf der Kopplung dieses Antikörpers an das cAMP-XL665 Molekül beruht. Dadurch entsteht nach Anregung bei 337 nm ein auf FRET (fluorescence resonance energy transfer) basierendes, langanhaltendes Emmisionssignal bei 665 nm (und bei 620 nM). Beide Signale werden in einem geeigneten Messgerät zeitlich versetzt, d. h. nach Abklingen der Hintergrundsfluoreszenz, gemessen. Jegliche Erhöhung des durch Prostaglandin-E₂-Gabe bedingten niedrigen FRET-Signals (gemessen als Well-Ratio-Veränderung = Emmision₆₆₅ₙₘ/Emmision₆₂₀ₙₘ * 10000) zeigt die Wirkung von Antagonisten.

### 1.2. Nachweisverfahren

### 1.2.1. Test auf Antagonismus (Angaben pro Vertiefung einer 384-Loch Platte):

Die in einer Testplatte und 30 % DMSO vorgelegten Substanzlösungen (0.75 µl) werden in 16 µl einer KRSB+IBMX-Stimulationslösung gelöst (1 X Krebs-Ringer Bicarbonate Buffer; Sigma-Aldrich # K-4002; inklusive 750 µM 3-Isobutyl-1-methylxanthine Sigma-Aldrich # 1-7018), anschließend werden 15 µl davon in eine medienfreie Zellkulturplatte überführt, die kurz zuvor mit KRSB gewaschen worden war.

Nach einer 30-minütigen Vorinkubation bei Raumtemperatur (RT) werden 5 µl einer 4xPGE₂ Lösung (11 nM) zugegeben und in Gegenwart des Agonisten für weitere 60 min bei RT inkubiert (Volumen: ~20 µl), bevor die Reaktion anschließend durch Zugabe von 5 µl Lysispuffer gestoppt und für weitere 20 min bei RT inkubiert wird (Volumen: ~25 µl). Das Zell-Lysat wird anschließend in eine Messplatte überführt und entsprechend den Herstellerangaben (cyclic AMP kit Cisbio International # 62AMPPEC) gemessen.

### 1.2.2. Test auf Agonismus (Angaben pro Vertiefung einer 384-Loch Platte):

Die in einer in einer Testplatte und 30 % DMSO vorgelegten Substanzlösungen (0.75µl) werden in 16 µl einer KRSB+IBMX-Stimulationslösung gelöst (1 X Krebs-Ringer Bicarbonate Buffer; Sigma-Aldrich # K-4002; inklusive 750 µM 3-Isobutyl-1-methylxanthine Sigma-Aldrich # I-7018), anschließend werden 15 µl davon in eine medienfreie Zellkulturplatte überführt, die kurz zuvor mit KRSB gewaschen worden war.
Nach einer 60-minütigen Inkubation bei Raumtemperatur (RT; Volumen: ~15 µl) wird die Reaktion anschließend durch Zugabe von 5 µl Lysisbuffer gestoppt und für weitere 20 min bei RT inkubiert (Volumen: ~20 µl). Das Zell-Lysat wird anschließend in eine Messplatte überführt und entsprechend den Herstellerangaben (cyclic AMP kit Cisbio International # 62AMPPEC) gemessen.

### 2. Der EP₂-Subtyp des PGE₂-Rezeptors und die prä-ovulatorische Kumulus Expansion

### 2.1. Hintergrund:

Im prä-ovulatorischen antralen Follikel ist die Eizelle von Kumulus Zellen umgeben, die einen dichten Zellkranz um die Eizelle bilden. Nach dem LH-Peak (Lutenisierendes Hormon) wird eine Reihe von Prozessen aktiviert, die eine starke morphologische Veränderung dieses Zellkranzes aus Kumulus Zellen zur Folge hat. Hierbei bilden die Kumulus Zellen eine extrazelluläre Matrix, die zur sogenannten Kumulus Expansion führt (Vanderhyden et al. Dev Biol. 1990 Aug;140(2):307-317). Diese Kumulus Expansion ist ein wichtiger Bestandteil des ovulatorischen Prozesses und der nachfolgenden Möglichkeit zur Fertilisation.
Bei der Kumulus Expansion sind Prostaglandine und hier das Prostaglandin E₂, dessen Synthese durch den LH-Peak induziert wird, von entscheidender Bedeutung. Prostanoid EP₂ Knock-out Mäuse (Hizaki et al. Proc Natl Acad Sci U S A. 1999 Aug 31;96(18):10501-6.) zeigen eine deutlich verminderte Kumulus Expansion und starke Subfertilität, was die Bedeutung des Prostanoid EP₂-Rezeptors für diesen Prozess demonstriert.

### 2.2. Kumulus Expansions Test in vitro

In immaturen weiblichen Mäusen (Stamm : CD1 (ICR) von Charles River) wird in einem Alter von 14-18 Tagen die Follikulogenese durch eine einmalige Gabe (intraperitoneal) von 10 I.E. PMSG (Pregnant Mare Serum Gonadotropine; Sigma G-4877, Lot 68H0909) induziert. 47 - 50 Stunden nach der Injektion werden die Ovarien entnommen und die Kumulus-Eizell Komplexe entnommen. Der Kumulus Komplex ist in diesem Stadium noch nicht expandiert.
Die Kumulus-Eizell Komplexe werden nun für 20 - 24 Stunden mit Prostaglandin E₂ (PGE₂) (1 µM), Vehikel Kontrolle (Ethanol) oder Testsubstanzen inkubiert. Medium: alpha- MEM Medium mit 0,1 mM IBMX, Pyruvate (0,23 mM) Glutamine (2 mM), Pen/Strep 100 IU/ml Pen. und 100 µg/ml Strep.) und HSA (8 mg/ml)). Danach wird die Kumulus Expansion durch die Einteilung in vier Stadien (nach Vanderhyden et al. Dev Biol. 1990 Aug;140(2):307-317) festgestellt.

**Tabelle 1: Beispiel für die biologische Wirksamkeit der erfindungsgemäßen Verbindungen (gemessen mittels cAMP Antagonismustest):**

| Substanz gemäß Beispiel | Antagonismus [IC₅₀, µM] |
|---|---|
| 8 | 3,8 |
| 1 | 2,7 |
| 14 | 3,4 |

## Patentansprüche

1. Verbindungen der allgemeinen Formel l 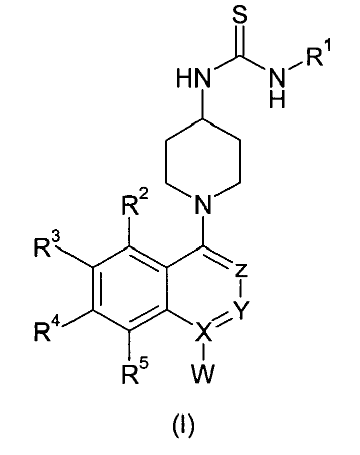 wobei
X, Y, Z unabhängig voneinander ein Stickstoffrest oder ein Kohlenstoffrest -C-R⁸, wobei R⁸ Wasserstoff oder ein C₁-C₄-Alkylrest sein kann, unter der Voraussetzung, dass mindestens ein, höchstens jedoch 2 der Gruppen X, Y, Z ein Stickstoffrest ist,
R¹ ein 5-12-gliedriger, mono- oder bicyclischer Aryl- oder Heteroarylring, der unsubstituiert oder gegebenenfalls ein- bis dreifach substituiert sein kann,
R²- R⁵ unabhängig voneinander ein Wasserstoff, Halogen, Cyano,
- oder eine OR⁶, OC(O)R⁶, S(O)ₙR⁶, wobei n = 0, 1, 2 bedeutet, SO₂NHR⁶, SO₂NHC(O)R⁶, NR⁶R⁷, NHC(O)R⁶, CH₂NR⁶R⁷, CH₂NHC(O)R⁶, C(OH)R⁶R⁷, C(O)R⁶, CO₂R⁶, C(O)NR⁶R⁷-Gruppe,
- eine C₁-C₆-Alkylgruppe, welche unsubstituiert oder gegebenenfalls substituiert sein kann,
- ein C₃-C₁₀-Cydoalkylring, der unsubstituiert oder gegebenenfalls substituiert sein kann,
- eine C₂-C₆-Alkenyl-oder C₂-C₆-Alkinylgruppe, die unsubstituiert oder gegebenenfalls substituiert sein kann,
- ein 5-12-gliedriger, mono-ober bicyclischer Aryl- oder Heteroarylring, der unsubstituiert oder gegebenenfalls substituiert sein kann,
R⁶, R⁷ ein Wasserstoff, ein C₁-C₆-Alkyl, ein C₃-C₁₀-Cycloalkylring, ein 5-12-gliedriger mono- oder bicyclischer Aryl- oder Heteroarylring,
wobei die Alkyl-, Cycloalkyl- und (Het)arylgruppen unsubstituiert oder gegebenenfalls substituiert sein können, oder
R⁶, R⁷ zusammen einen 3-8-gliedrigen Ring bilden,
bedeuten, sowie deren Isomere, Salze und deren Cyclodextrinclathraten,
die bekannten Nachteile überwinden, und eine bessere Selektivität zum EP₂ Rezeptor und somit eine bessere Wirksamkeit und längere Wirkdauer erzielen können.

2. Verbindungen gemäß Anspruch 1, wobei
X ein Stickstoffrest,
Y, Z ein Kohlenstoffrest -C-R⁸,
R⁸ ein Wasserstoff, ein C₁-C₄-Alkylrest,
_{R}¹ ein 5-12-gliedriger, mono- oder bicyclischer, Aryl- oder Heteroarylring, der unsubstituiert und gegebenenfalls ein- bis dreifach substituiert sein kann,
R² - R⁵ unabhängig voneinander ein Wasserstoff, Halogen, Cyano,
- oder eine OR⁶, OC(O)R⁶, S(O)ₙR⁶, wobei n = 0, 1, 2 bedeutet, SO₂NHR⁶, SO₂NHC(O)R⁶, NR⁶R⁷, NHC(O)R⁶, CH₂NR⁶R⁷, CH₂NHC(O)R⁶, C(OH)R⁶R⁷, C(O)R⁶, CO₂R⁶, C(O)NR⁶R⁷-Gruppe,
- eine C₁-C₆-Alkylgruppe, die unsubstituiert ist oder gegebenenfalls substituiert sein kann,
- ein C₃-C₁₀-Cycloalkylring, der unsubstituiert ist oder gegebenenfalls substituiert sein kann,
- eine C₂-C₆-Alkenyl- oder C₂-C₆-Alkinylgruppe, die unsubstituiert ist oder gegebenenfalls substituiert sein kann,
- ein 5-12-gliedriger mono- oder bicyclischer Aryl- oder
Heteroarylring, der unsubstituiert ist oder gegebenenfalls substituiert sein kann,
R⁶, R⁷ ein Wasserstoff, ein C₁-C₆-Alkyl, ein C₃-C₁₀-Cycloalkylring, ein 5-12-gliedriger mono- oder bicyclischer Aryl- oder Heteroarylring,
wobei die Alkyl-, Cycloalkyl- und (Het)arylgruppen unsubstituiert sind oder gegebenenfalls substituiert sein können, oder
R⁶, R⁷ zusammen einen 3-8-gliedrigen Ring bilden, bedeuten.

3. Verbindungen gemäß Anspruch 1, wobei
Y und Z ein Stickstoffrest,
X ein Kohlenstoffrest -C-R⁸,
R⁸ ein Wasserstoff oder ein C₁-C₄-Alkylrest,
R¹ ein 5-12-gliedriger mono oder bicyclischer Aryl- oder Heteroarylring, der unsubstituiert ist oder gegebenenfalls ein- bis dreifach substituiert sein kann,
R²-R⁵ unabhängig voneinander ein Wasserstoff, Halogen, Cyano,
- oder eine OR⁶, OC(O)R⁶, S(O)ₙR⁶, wobei n = 0, 1, 2 bedeutet, SO₂NHR⁶, SO₂NHC(O)R⁶, NR⁶R⁷, NHC(O)R⁶, CH₂NR⁶R⁷, CH₂NHC(O)R⁶, C(OH)R⁶R⁷, C(O)R⁶, CO₂R⁶, C(O)NR⁶R⁷-Gruppe,
- eine C₁-C₆-Alkylgruppe, die unsubstituiert ist oder gegebenenfalls substituiert sein kann,
- ein C₃-C₁₀-Cycloalkylring, der unsubstituiert ist oder gegebenenfalls substituiert sein kann,
- eine C₂-C₆-Alkenyl-oder C₂-C₆-Alkinylgruppe, die unsubstituiert ist oder gegebenenfalls substituiert sein kann,
- ein 5-12-gliedriger mono- oder bicyclischer Aryl- oder
Heteroarylring, der unsubstituiert ist oder gegebenenfalls substituiert sein kann,
R⁶, R⁷ ein Wasserstoff, ein C₁-C₆-Alkyl, ein C₃-C₁₀-Cycloalkylring, ein 5-12-gliedriger mono- oder bicyclischer Aryl- oder Heteroarylring,
wobei die Alkyl-, Cycloalkyl- und (Het)arylgruppen unsubstituiert sind oder gegebenenfalls substituiert sein können, oder
R⁶, R⁷ zusammen einen 3-8-gliedrigen Ring bilden,
bedeuten.

4. Verbindungen, gemäß Anspruch 1, wobei
X und Z jeweils ein Stickstoffrest,
Y ein Kohlenstoffrest -C-R⁸,
R⁸ ein Wasserstoff, ein C₁-C₄-Alkylrest,
R¹ ein 5-12-gliedriger mono- oder bicyclischer Aryl- oder Heteroarylring, der unsubstituiert ist und gegebenenfalls ein- bis dreifach substituiert sein kann,
R² - R⁵ unabhängig voneinander ein Wasserstoff, Halogen, Cyano,
- oder eine OR⁶, OC(O)R⁶, S(O)ₙR⁶, wobei n = 0, 1, 2 bedeutet, SO₂NHR⁶, SO₂NHC(O)R⁶, NR⁶R⁷, NHC(O)R⁶, CH₂NR⁶R⁷, CH₂NHC(O)R⁶, C(OH)R⁶R⁷, C(O)R⁶, CO₂R⁶, C(O)NR⁶R⁷-Gruppe,
- eine C₁-C₆-Alkylgruppe, die unsubstituiert ist oder gegebenenfalls substituiert sein kann,
- ein C₃-C₁₀-Cycloalkylring, der unsubstituiert ist oder gegebenenfalls substituiert sein kann,
- eine C₂-C₆-Alkenyl-oder C₂-C₆-Alkinylgruppe, die unsubstituiert ist oder gegebenenfalls substituiert sein kann,
- ein 5-12-gliedriger mono- oder bicyclischer Aryl- oder
Heteroarylring, der unsubstituiert ist oder gegebenenfalls substituiert sein kann,
R⁶, R⁷ ein Wasserstoff, ein C₁-C₆-Alkyl, ein C₃-C₁₀-Cycloalkylring, ein 5-12-gliedriger, mono- oder bicyclischer Aryl- oder Heteroarylring,
wobei die Alkyl-, Cycloalkyl- und (Het)arylgruppen unsubstituiert sind oder gegebenenfalls substituiert sein können, oder
R⁶, R⁷ zusammen einen 3-8-gliedrigen Ring bilden,
bedeuten.

5. Verbindungen, gemäß Anspruch 1, wobei
X und Y jeweils ein Kohlenstoffrest -C-R⁸,
Z ein Stickstoffrest,
R⁸ ein Wasserstoff, ein C₁-C₄-Alkylrest,
R¹ ein 5-12-gliedriger oder mono- oder bicyclischer Aryl- oder Heteroarylring, der unsubstituiert ist oder gegebenenfalls ein- bis dreifach substituiert sein kann,
R² - R⁵ unabhängig voneinander ein Wasserstoff, Halogen, Cyano,
- oder eine OR⁶, OC(O)R⁶, S(O)ₙR⁶, wobei n = 0, 1, 2 bedeutet, SO₂NHR⁶, SO₂NHC(O)R⁶, NR⁶R⁷, NHC(O)R⁶, CH₂NR⁶R⁷, CH₂NHC(O)R⁶, C(OH)R⁶R⁷, C(O)R⁶, CO₂R⁶, C(O)NR⁶R⁷-Gruppe,
- eine C₁-C₆-Alkylgruppe, die unsubstituiert ist oder gegebenenfalls substituiert sein kann,
- ein C₃-C₁₀-Cycloalkylring, der unsubstituiert ist oder gegebenenfalls substituiert sein kann,
- eine C₂-C₆-Alkenyl-oder C₂-C₆-Alkinylgruppe, die unsubstituiert ist oder gegebenenfalls substituiert sein kann,
- ein 5-12-gliedriger mono- oder bicyclischer Aryl- oder
Heteroarylring, der unsubstituiert ist oder gegebenenfalls substituiert sein kann,
R⁶, R⁷ ein Wasserstoff, ein C₁-C₆-Alkyl, ein C₃-C₁₀-Cycloalkylring, ein 5-12-gliedriger, mono- oder bicyclischer Aryl- oder Heteroarylring,
wobei die Alkyl-, Cycloalkyl- und (Het)arylgruppen unsubstituiert sind oder gegebenenfalls substituiert sein können, oder
R⁶, R⁷ zusammen einen 3-8-gliedrigen Ring bilden,
bedeuten.

6. Verbindungen gemäß den Ansprüchen 1 und 2, wobei
X ein Stickstoffrest,
Y, Z ein Kohlenstoffrest -C-R⁸,
R⁸ ein Wasserstoff,
R¹ ein unsubstituierter 5-12-gliedriger mono- oder bicyclischer Aryl-oder Heteroarylring, der gegebenenfalls ein- bis dreifach substituiert ist,
wobei die Substituenten ausgewählt sein können aus der
Gruppe
Halogen,
-C₁-C₄-Alkyl, welches unsubstituiert ist und gegebenenfalls substituiert sein kann,
-OR⁶, -OC(O)R⁶, -S(O)ₙR⁶, wobei n = 0, 1, 2 bedeutet, -SO₂NHR⁶, -SO₂NHC(O)R⁶, NR⁶R⁷, -NHC(O)R⁶, -CN, -CO₂-R⁶, -C(O)-N-R⁶R⁷, -C(O)R⁶, -C(OH)R⁶R⁷,
wobei es sich bei dem 5-12-gliedrigen mono- oder bicyclischen Aryl- oder Heteroarylring beispielsweise, aber nicht ausschließlich, um eine Naphthyl-, Chinolinyl-, Isochinolinyl-, Phthalazinyl-, Chinazolinyl-, Chinoxalinyl-, Cinnolinyl-, Benzothiophenyl-, 1,3-Benzodioxolyl-, 2,1,3-Benzothiadiazolyl-, Phenyl-, Pyridinyl-, Pyrimidinyl-, Furanyl-, Thiophenyl-, Oxazolyl-, Isoxazolyl-, Thiazolyl-, Pyrrolyl-, Pyrazolyl-, Imidazolyl-, Pyrazinyl-, Pyridazinyl-, Triazolyl-, Tetrazolyl-, Indolyl-, Benzofuranyl- oder Benzimidazolyl-Gruppe handeln kann,
R² ein Wasserstoff,
R³ - R⁵ unabhängig voneinander ein Wasserstoff, Halogen, Cyano,
oder eine OR⁶, OC(O)R⁶, S(O)ₙR⁶ wobei n = 0, 1, 2 bedeutet, SO₂NHR⁶, SO₂NHC(O)R⁶, NR⁶R⁷, NHC(O)R⁶, CH₂NR⁶R⁷, CH₂NHC(O)R⁶, C(OH)R⁶R⁷, C(O)R⁶, CO₂R⁶, C(O)NR⁶R⁷-Gruppe,
eine C₁-C₆-Alkylgruppe, welche gegebenenfalls substituiert oder unsubstituiert ist,
ein C₃-C₁₀-Cycloalkylring, der gegebenenfalls substituiert oder unsubstituiert ist,
eine C₂-C₆-Alkenyl-oder C₂-C₆-Alkinylgruppe, die gegebenenfalls substituiert oder unsubstituiert ist,
ein 5-12-gliedriger mono- oder bicyclischer Aryl- oder Heteroarylring, der gegebenenfalls ein- oder mehrfach substituiert ist,
wobei die Substituenten ausgewählt sein können aus der
Gruppe
- Halogen,
- -C₁-C₄-Alkyl, welches unsubstituiert ist und gegebenenfalls substituiert sein kann,
- -OR⁹, -OC(O)R⁹, -S(O)ₙR⁹, wobei n = 0, 1, 2 bedeutet, -SO₂NHR⁹, -SO₂NHC(O)R⁹, NR⁹R¹⁰,-NHC(O)R⁹, -CN, -CO₂-R⁹, -C(O)-N-R⁹R¹⁰, -C(O)R⁹,-C(OH)R⁹R¹⁰,
wobei es sich bei dem 5-12-gliedrigen mono oder bicyclischen Aryl- oder Heteroarylring beispielsweise, aber nicht ausschließlich, um eine Naphthyl-, Chinolinyl-, Isochinolinyl-, Phthalazinyl-, Chinazolinyl-, Chinoxalinyl-, Cinnolinyl-, Benzothiophenyl-, 1,3-Benzodioxolyl-, 2,1,3-Benzothiadiazolyl-, Phenyl-, Pyridinyl-, Pyrimidinyl-, Furanyl-, Thiophenyl-, Oxazolyl-, lsoxazolyl-, Thiazolyl-, Pyrrolyl-, Pyrazolyl-, Imidazolyl-, Pyrazinyl-, Pyridazinyl-, Triazolyl-, Tetrazolyl-, Indolyl-, Benzofuranyl- oder Benzimidazolyl-Gruppe handeln kann,
R⁶, R⁷ ein Wasserstoff, eine C₁-C₆-Alkylgruppe, welche unsubstituiert ist oder gegebenenfalls bis zu 5fach halogeniert sein kann,
ein C₃-C₈-Cycloalkylrest,
ein 5-12-gliedriger, mono-oder bicyclischer Aryl- oder Heteroarylring, der gegebenenfalls ein- oder mehrfach substituiert
ist,
wobei die Substituenten ausgewählt sein können aus der
Gruppe
- Halogen,
- Cyano,
- R⁹, -OR⁹, -OC(O)R⁹, -S(O)ₙR⁹, wobei n = 0, 1, 2 bedeutet, -SO₂NHR⁹, NR⁹R¹⁰, -NHC(O)R⁹, -CO₂-R⁹, -C(O)-N-R⁹R¹⁰,
wobei es sich bei dem 5-12-gliedrigen mono- oder bicyclischen Aryl- oder Heteroarylring beispielsweise, aber nicht ausschließlich, um eine Naphthyl- Chinolinyl-, Isochinolinyl-, Phthalazinyl-, Chinazolinyl-, Chinoxalinyl-, Cinnolinyl-, Benzothiophenyl-, 1,3-Benzodioxolyl-, 2,1,3-Benzothiadiazolyl-, Phenyl-, Pyridinyl-, Pyrimidinyl-, Furanyl-, Thiophenyl-, Oxazolyl-, Isoxazolyl-, Thiazolyl-, Pyrrolyl-, Pyrazolyl-, Imidazolyl-, Pyrazinyl-, Pyridazinyl-, Triazolyl-, Tetrazolyl-, Indolyl-, Benzofuranyl- oder Benzimidazolyl Gruppe handeln kann, oder
R⁶, R⁷ zusammen einen 3-8-gliedrigen Ring bilden,
R⁹, R¹⁰ unabhängig voneinander Wasserstoff,
- eine C₁-C₄-Alkylgruppe, welche unsubstituiert ist oder gegebenenfalls bis zu fünffach fluoriert sein kann,
- eine C₂-C₄-Alkenylgruppe, welche unsubstituiert ist oder gegebenenfalls bis zu dreifach fluoriert sein kann,
- eine C₂-C₄-Alkinylgruppe, welche unsubstituiert ist oder gegebenenfalls einfach fluoriert sein kann,
- eine C₃-C₆-Cycloalkylgruppe,
- ein 5-6-gliedriger Aryl- oder Heteroarylring, wobei es sich dabei beispielsweise, aber nicht ausschließlich, um einen Phenyl-, Pyridinyl-, Pyrimidinyl-, Furanyl-, Thiophenyl-, Oxazolyl-, Isoxazolyl-, Thiazolyl-, Pyrrolyl-, Pyrazolyl-, Imidazolyl-, Pyrazinyl-, Pyridazinyl-, Triazolyl-, Tetrazolyl-Ring handeln kann, der unsubstituiert ist oder gegebenenfalls bis zu zweifach mit Fluor, Chlor, Trifluormethyl substituiert sein kann, oder
R⁹, R¹⁰ zusammen einen 3-8-gliedrigen Ring bilden,
bedeuten.

7. Verbindungen gemäß den Ansprüchen 1 und 3, wobei
Y und Z ein Stickstoffrest,
X ein Kohlenstoffrest -C-R⁸,
R⁸ ein Wasserstoff oder eine Methylgruppe,
R¹ ein 5-12-gliedriger mono- oder bicyclischer Aryl- oder Heteroarylring, der gegebenenfalls ein- bis dreifach substituiert ist,
wobei die Substituenten ausgewählt sein können aus der Gruppe Halogen,
-C₁-C₄-Alkyl, welches unsubstituiert ist und gegebenenfalls substituiert sein kann,
-OR⁶, -OC(O)R⁶, -S(O)ₙR⁶, wobei n = 0, 1, 2 bedeutet,-SO₂NHR⁶, -SO₂NHC(O)R⁶, NR⁶R⁷, -NHC(O)R⁶, -CN, -CO₂-R⁶, -C(O)-N-R⁶R⁷, -C(O)R⁶, -C(OH)R⁶R⁷,
wobei es sich bei dem 5-12-gliedrigen mono- oder bicyclischen Aryl- oder Heteroarylring beispielsweise, aber nicht ausschließlich, um eine Naphthyl-, Chinolinyl-, Isochinolinyl-, Phthalazinyl-, Chinazolinyl-, Chinoxalinyl-, Cinnolinyl-, Benzothiophenyl-, 1,3-Benzodioxolyl-, 2,1,3-Benzothiadiazolyl-, Phenyl-, Pyridinyl-, Pyrimidinyl-, Furanyl-, Thiophenyl-, Oxazolyl-, Isoxazolyl-, Thiazolyl-, Pyrrolyl-, Pyrazolyl-, Imidazolyl-, Pyrazinyl-, Pyridazinyl-, Triazolyl-, Tetrazolyl-, Indolyl-, Benzofuranyl-, oder Benzimidazolyl-Gruppe handeln kann,
R² ein Wasserstoff,
R³ - R⁵ unabhängig voneinander ein Wasserstoff, Halogen, Cyano,
- oder eine OR⁶, OC(O)R⁶, S(O)ₙR⁶, wobei n = 0, 1, 2 bedeutet, SO₂NHR⁶, SO₂NHC(O)R⁶, NR⁶R⁷, NHC(O)R⁶, CH₂NR⁶R⁷, CH₂NHC(O)R⁶, C(OH)R⁶R⁷, C(O)R⁶, CO₂R⁶, C(O)NR⁶R⁷ Gruppe,
- eine C₁-C₆-Alkylgruppe, die unsubstituiert ist oder gegebenenfalls substituiert sein kann,
- ein C₃-C₁₀-Cycloalkylring, der unsubstituiert ist oder gegebenenfalls substituiert sein kann,
- eine C₂-C₆-Alkenyl-oder C₂-C₆-Alkinylgruppe, die unsubstituiert ist oder gegebenenfalls substituiert sein kann,
- ein unsubstituierter 5-12-gliedriger mono- oder bicyclischer Aryl- oder Heteroarylring, der gegebenenfalls ein- oder mehrfach substituiert sein kann,
wobei die Substituenten ausgewählt sein können aus der
Gruppe
Halogen,
-C₁-C₄-Alkyl, welches unsubstituiert ist und gegebenenfalls substituiert sein kann,
-OR⁹, -OC(O)R⁹, -S(O)ₙR⁹ wobei n = 0, 1, 2 bedeutet, -SO₂NHR⁹, -SO₂NHC(O)R⁹, NR⁹R¹⁰, -NHC(O)R⁹, -CN,-CO₂-R⁹, -C(O)-N-R⁹R¹⁰, -C(O)R⁹, -C(OH)R⁹R¹⁰,
wobei es sich bei dem 5-12-gliedrigen mono- oder bicyclischen Aryl- oder Heteroarylring beispielsweise, aber nicht ausschließlich, um eine Naphthyl-, Chinolinyl-, Isochinolinyl-, Phthalazinyl-, Chinazolinyl-, Chinoxalinyl-, Cinnolinyl-, Benzothiophenyl-, 1,3-Benzodioxolyl-, 2,1,3-Benzothiadiazolyl-, Phenyl-, Pyridinyl-, Pyrimidinyl-, Furanyl-, Thiophenyl-, Oxazolyl-, Isoxazolyl-, Thiazolyl-, Pyrrolyl-, Pyrazolyl-, Imidazolyl-, Pyrazinyl-, Pyridazinyl-, Triazolyl-, Tetrazolyl-, Indolyl-, Benzofuranyl- oder Benzimidazolyl-Gruppe handeln kann,
R⁶, R⁷ ein Wasserstoff, eine unsubstituierte C₁-C₆-Alkylgruppe, die gegebenenfalls bis zu fünffach halogeniert sein kann, ein C₃-C₈-Cycloalkylrest,
ein unsubstituierter 5-12-gliedriger, mono-oder bicyclischer Aryl-oder Heteroarylring, der gegebenenfalls ein- oder mehrfach substituiert sein kann, wobei die Substituenten ausgewählt sein können aus der Gruppe
- Halogen,
- Cyano,
- R⁹, -OR⁹, -OC(O)R⁹, -S(O)ₙR⁹, wobei n = 0, 1, 2 bedeutet, -SO₂NHR⁹, NR⁹R¹⁰, -NHC(O)R⁹, -CO₂-R⁹, -C(O)-N-R⁹R¹⁰,
wobei es sich bei dem 5-12-gliedrigen mono oder bicyclischen Aryl- oder Heteroarylring beispielsweise, aber nicht ausschließlich, um eine Naphthyl-, Chinolinyl-, Isochinolinyl-, Phthalazinyl-, Chinazolinyl-, Chinoxalinyl-, Cinnolinyl-, Benzothiophenyl-, 1,3-Benzodioxolyl-, 2,1,3-Benzothiadiazolyl-, Phenyl-, Pyridinyl-, Pyrimidinyl-, Furanyl-, Thiophenyl-, Oxazolyl-, Isoxazolyl-, Thiazolyl-, Pyrrolyl-, Pyrazolyl-, Imidazolyl-, Pyrazinyl-, Pyridazinyl-, Triazolyl-, Tetrazolyl-, Indolyl-, Benzofuranyl- oder Benzimidazolyl-Gruppe handeln kann, oder
R⁶, R⁷ zusammen einen 3-8-gliedrigen Ring bilden,
R⁹, R¹⁰ unabhängig voneinander Wasserstoff,
- eine C₁-C₄-Alkylgruppe, welche unsubstituiert ist oder gegebenenfalls bis zu fünffach fluoriert sein kann,
- eine C₂-C₄-Alkenylgruppe, welche unsubstituiert ist oder gegebenenfalls bis zu dreifach fluoriert sein kann,
- eine C₂-C₄-Alkinylgruppe, welche unsubstituiert ist oder gegebenenfalls einfach fluoriert sein kann,
- eine C₃-C₆-Cycloalkylgruppe,
- ein 5-6gliedriger Aryl- oder Heteroarylring, wobei es sich dabei beispielsweise, aber nicht ausschließlich, um einen Phenyl-, Pyridinyl-, Pyrimidinyl-, Furanyl-, Thiophenyl-, Oxazolyl-, Isoxazolyl-, Thiazolyl-, Pyrrolyl-, Pyrazolyl-, Imidazolyl-, Pyrazinyl-, Pyridazinyl-, Triazolyl-, Tetrazolyl-Ring handeln kann, der unsubstituiert ist oder gegebenenfalls bis zu zweifach mit Fluor, Chlor, Trifluormethyl substituiert sein kann,
oder
R⁹, R¹⁰ zusammen einen 3-8-gliedrigen Ring bilden,
bedeuten.

8. Verbindungen gemaß den Ansprüchen 1 und 4, wobei
X und Z jeweils ein Stickstoffrest,
Y ein Kohlenstoffrest -C-R⁸,
R⁸ ein Wasserstoff,
R¹ ein unsubstituierter 5-12-gliedriger mono oder bicyclischer Aryl-oder Heteroarylring, der gegebenenfalls ein- bis dreifach substituiert ist,
wobei die Substituenten ausgewählt sein können aus der Gruppe Halogen,
-C₁-C₄-Alkyl, welches unsubstituiert ist und gegebenenfalls substituiert sein kann,
-OR⁶, -OC(O)R⁶, -S(O)ₙR⁶, wobei n = 0, 1, 2 bedeutet,-SO₂NHR⁶, -SO₂NHC(O)R⁶, NR⁶R⁷, -NHC(O)R⁶, -CN, -CO₂-R⁶, -C(O)-N-R⁶R⁷, -C(O)R⁶, -C(OH)R⁶R⁷,
wobei es sich bei dem 5-12-gliedrigen mono oder bicyclischen Aryl- oder Heteroarylring beispielsweise, aber nicht ausschließlich, um eine Naphthyl-, Chinolinyl-, Isochinolinyl-, Phthalazinyl-, Chinazolinyl-, Chinoxalinyl-, Cinnolinyl-, Benzothiophenyl-, 1,3-Benzodioxolyl-, 2,1,3-Benzothiadiazolyl-, Phenyl-, Pyridinyl-, Pyrimidinyl-, Furanyl-, Thiophenyl-, Oxazolyl-, Isoxazolyl-, Thiazolyl-, Pyrrolyl-, Pyrazolyl-, Imidazolyl-, Pyrazinyl-, Pyridazinyl-, Triazolyl-, Tetrazolyl-, Indolyl-, Benzofuranyl- oder Benzimidazolyl-Gruppe handeln kann,
R² ein Wasserstoff,
R³ - R⁵ unabhängig voneinander ein Wasserstoff, Halogen, Cyano,
- oder eine OR⁶, OC(O)R⁶, S(O)ₙR⁶, wobei n = 0, 1, 2 bedeutet, SO₂NHR⁶, SO₂NHC(O)R⁶, NR⁶R⁷, NHC(O)R⁶, CH₂NR⁶R⁷, CH₂NHC(O)R⁶, C(OH)R⁶R⁷, C(O)R⁶, CO₂R⁶, C(O)NR⁶R⁷ Gruppe,
- eine unsubstituierte C₁-C₆-Alkylgruppe, die substituiert sein kann,
- ein unsubstituierter C₃-C₁₀-Cycloalkylring, der gegebenenfalls substituiert sein kann,
- eine unsubstituierte C₂-C₆-Alkenyl-oder C₂-C₆-Alkinylgruppe, die gegebenenfalls substituiert sein kann,
- ein unsubstituierter 5-12gliedriger, mono- oder bicyclischer Aryl- oder Heteroarylring, der gegebenenfalls ein-oder mehrfach substituiert sein kann,
wobei die Substituenten ausgewählt sein können aus der Gruppe Halogen, -C₁-C₄-Alkyl, welches unsubstituiert ist und gegebenenfalls substituiert sein kann,
-OR⁹, -OC(O)R⁹, -S(O)ₙR⁹, wobei = 0, 1, 2 bedeutet,-SO₂NHR⁹, -SO₂NHC(O)R⁹, NR⁹R¹⁰, -NHC(O)R⁹, -CN, -CO₂-R⁹, -C(O)-N-R⁹R¹⁰, -C(O)R⁹, -C(OH)R⁹R¹⁰,
wobei es sich bei dem 5-12-gliedrigen mono- oder bicyclischen Aryl- oder Heteroarylring beispielsweise, aber nicht ausschließlich, um eine Naphthyl-, Chinolinyl-, Isochinolinyl-, Phthalazinyl-, Chinazolinyl-, Chinoxalinyl-, Cinnolinyl-, Benzothiophenyl-, 1,3-Benzodioxolyl-, 2,1,3-Benzothiadiazolyl-, Phenyl-, Pyridinyl-, Pyrimidinyl-, Furanyl-, Thiophenyl-, Oxazolyl-, Isoxazolyl-, Thiazolyl-, Pyrrolyl-, Pyrazolyl-, Imidazolyl-, Pyrazinyl-, Pyridazinyl-, Triazolyl-, Tetrazolyl-, Indolyl-, Benzofuranyl- oder Benzimidazolyl-Gruppe handeln,
R⁶, R⁷ ein Wasserstoff, eine C₁-C₆-Alkylgruppe, die unsubstituiert oder gegebenenfalls bis zu fünffach halogeniert sein kann, ein unsubstituierter C₃-C₈-Cycloalkylrest,
ein unsubstituierter 5-12-gliedriger mono- oder bicyclischer Aryl-oder Heteroarylring, der gegebenenfalls ein- oder mehrfach substituiert sein kann, wobei die Substituenten ausgewählt sein können aus der Gruppe
- Halogen,
- Cyano,
- R⁹, -OR⁹, -OC(O)R⁹, -S(O)ₙR⁹, wobei n = 0, 1, 2 bedeutet, -SO₂NHR⁹, NR⁹R¹⁰, -NHC(O)R⁹, -CO₂-R⁹, -C(O)-N-R⁹R¹⁰,
wobei es sich bei dem 5-12-gliedrigen mono- oder bicyclischen Aryl- oder Heteroarylring beispielsweise, aber nicht ausschließlich, um eine Naphthyl-, Chinolinyl-, Isochinolinyl-, Phthalazinyl-, Chinazolinyl-, Chinoxalinyl-, Cinnolinyl-, Benzothiophenyl-, 1,3-Benzodioxolyl-, 2,1,3-Benzothiadiazolyl-, Phenyl-, Pyridinyl-, Pyrimidinyl-, Furanyl-, Thiophenyl-, Oxazolyl-, Isoxazolyl-, Thiazolyl-, Pyrrolyl-, Pyrazolyl-, Imidazolyl-, Pyrazinyl-, Pyridazinyl-, Triazolyl-, Tetrazolyl-, Indolyl-, Benzofuranyl- oder Benzimidazolyl-Gruppe handeln kann, oder
R⁶, R⁷ zusammen einen 3-8-gliedrigen Ring bilden,
R⁹, R¹⁰ unabhängig voneinander Wasserstoff,
- eine unsubstituierte C₁-C₄-Alkylgruppe, welche gegebenenfalls bis zu fünffach fluoriert sein kann,
- eine unsubstituierte C₂-C₄-Alkenylgruppe, welche gegebenenfalls bis zu dreifach fluoriert sein kann,
- eine unsubstituierte C₂-C₄-Alkinylgruppe, welche gegebenenfalls einfach fluoriert sein kann,
- eine unsubstituierte C₃-C₆-Cycloalkylgruppe,
- ein unsubstituierter 5-6gliedriger Aryl- oder Heteroarylring, wobei es sich dabei beispielsweise, aber nicht ausschließlich, um einen Phenyl-, Pyridinyl-, Pyrimidinyl-, Furanyl-, Thiophenyl-, Oxazolyl-, Isoxazolyl-, Thiazolyl-, Pyrrolyl-, Pyrazolyl-, Imidazolyl-, Pyrazinyl-, Pyridazinyl-, Triazolyl-, Tetrazolyl-Ring handeln kann, der gegebenenfalls bis zu zweifach mit Fluor, Chlor, Trifluormethyl substituiert sein kann, oder
R⁹, R¹⁰ zusammen einen 3-8-gliedrigen Ring bilden,
bedeuten.

9. Verbindungen gemaß den Ansprüchen 1 und 5, wobei
X und Y jeweils ein Kohlenstoffrest -C-R⁸,
Z ein Stickstoffrest,
R⁸ eine Wasserstoff- oder Methylgruppe,
R¹ ein unsubstituierter 5-12-gliedriger, mono- oder bicyclischer Aryl-oder Heteroarylring, der gegebenenfalls ein- bis dreifach substituiert ist,
wobei die Substituenten ausgewählt sein können aus der Gruppe Halogen, -C₁-C₄-Alkyl, das unsubstituiert ist oder gegebenenfalls substituiert sein kann,
-OR⁶, -OC(O)R⁶, -S(O)ₙR⁶ , wobei n = 0, 1, 2 bedeutet,-SO₂NHR⁶, -SO₂NHC(O)R⁶, NR⁶R⁷, -NHC(O)R⁶, -CN, -CO₂-R⁶, -C(O)-N-R⁶R⁷, -C(O)R⁶, -C(OH)R⁶R⁷,
wobei es sich bei dem 5-12-gliedrigen mono- oder bicyclischen Aryl- oder Heteroarylring beispielsweise, aber nicht ausschließlich, um eine Naphthyl-, Chinolinyl-, Isochinolinyl-, Phthalazinyl-, Chinazolinyl-, Chinoxalinyl-, Cinnolinyl-, Benzothiophenyl-, 1,3-Benzodioxolyl-, 2,1,3-Benzothiadiazolyl-, Phenyl-, Pyridinyl-, Pyrimidinyl-, Furanyl-, Thiophenyl-, Oxazolyl-, Isoxazolyl-, Thiazolyl-, Pyrrolyl-, Pyrazolyl-, Imidazolyl-, Pyrazinyl-, Pyridazinyl-, Triazolyl-, Tetrazolyl-, Indolyl-, Benzofuranyl- oder Benzimidazolyl-Gruppe handeln,
R² ein Wasserstoff,
R³ R⁵ unabhängig voneinander ein Wasserstoff, Halogen, Cyano, oder
- eine OR⁶, OC(O)R⁶, S(O)ₙR⁶, wobei n = 0, 1, 2, bedeutet, SO₂NHR⁶, SO₂NHC(O)R⁶, NR⁶R⁷, NHC(O)R⁶, CH₂NR⁶R⁷, CH₂NHC(O)R⁶, C(OH)R⁶R⁷, C(O)R⁶, CO₂R⁶, C(O)NR⁶R⁷ Gruppe,
- eine unsubstituierte C₁-C₆-Alkylgruppe, die gegebenenfalls substituiert sein kann,
- ein unsubstituierter C₃-C₁₀-Cycloalkylring, gegebenenfalls substituiert sein kann,
- eine unsubstituierte C₂-C₆-Alkenyl-oder C₂-C₆-Alkinylgruppe, die gegebenenfalls substituiert sein kann,
- ein unsubstituierter 5-12gliedriger mono- oder bicyclischer Aryl- oder Heteroarylring, der gegebenenfalls ein- oder mehrfach substituiert ist,
wobei die Substituenten ausgewählt sein können aus der Gruppe Halogen, -C₁-C₄-Alkyl, das unsubstituiert ist oder gegebenenfalls substituiert sein kann,
-OR⁹, -OC(O)R⁹, -S(O)ₙR⁹ wobei n = 0, 1, 2 bedeutet, -SO₂NHR⁹, -SO₂NHC(O)R⁹, NR⁹R¹⁰, -NHC(O)R⁹, -CN,-CO₂-R⁹, -C(O)-N-R⁹R¹⁰, -C(O)R⁹, -C(OH)R⁹R¹⁰,
wobei es sich bei dem 5-12-gliedrigen mono- oder bicyclischen Aryl- oder Heteroarylring beispielsweise, aber nicht ausschließlich, um eine Naphthyl-, Chinolinyl-, Isochinolinyl-, Phthalazinyl-, Chinazolinyl-, Chinoxalinyl-, Cinnolinyl-, Benzothiophenyl-, 1,3-Benzodioxolyl-, 2,1,3-Benzothiadiazolyl-, Phenyl-, Pyridinyl-, Pyrimidinyl-, Furanyl-, Thiophenyl-, Oxazolyl-, Isoxazolyl-, Thiazolyl-, Pyrrolyl-, Pyrazolyl-, Imidazolyl-, Pyrazinyl-, Pyridazinyl-, Triazolyl-, Tetrazolyl-, Indolyl-, Benzofuranyl- oder Benzimidazolyl-Gruppe handeln kann,
R⁶, R⁷ ein Wasserstoff, eine C₁-C₆-Alkylgruppe, die unsubstituiert ist oder gegebenenfalls bis zu fünffach halogeniert sein kann, ein unsubstituierter C₃-C₈-Cycloalkylrest,
ein unsubstituierter 5-12-gliedriger, mono- oder bicyclischer Aryl-oder Heteroarylring, der gegebenenfalls ein- oder mehrfach substituiert sein kann, wobei die Substituenten ausgewählt sein können aus der Gruppe
Halogen,
Cyano,
R⁹, -OR⁹, -OC(O)R⁹, -S(O)ₙR⁹ wobei n = 0, 1, 2, bedeutet, -SO₂NHR⁹, -SO₂NHC(O)R⁹, NR⁹R¹⁰,-NHC(O)R⁹, -CO₂-R⁹, -C(O)-N-R⁹R¹⁰,
wobei es sich bei dem 5-12-gliedrigen mono- oder bicyclischen Aryl- oder Heteroarylring beispielsweise, aber nicht ausschließlich, um eine Naphthyl-, Chinolinyl-, Isochinolinyl-, Phthalazinyl-, Chinazolinyl-, Chinoxalinyl-, Cinnolinyl-, Benzothiophenyl-, 1,3-Benzodioxolyl-, 2,1,3-Benzothiadiazolyl-, Phenyl-, Pyridinyl-, Pyrimidinyl-, Furanyl-, Thiophenyl-, Oxazolyl-, Isoxazolyl-, Thiazolyl-, Pyrrolyl-, Pyrazolyl-, Imidazolyl-, Pyrazinyl-, Pyridazinyl-, Triazolyl-, Tetrazolyl-, Indolyl-, Benzofuranyl- oder Benzimidazolyl-Gruppe handeln kann, oder
R⁶, R⁷ zusammen einen 3-8-gliedrigen Ring bilden,
R⁹, R¹⁰ unabhängig voneinander Wasserstoff,
- eine unsubstituierte C₁-C₄-Alkylgruppe, die gegebenenfalls bis zu fünffach fluoriert sein kann,
- eine unsubstituierte C₂-C₄-Alkenylgruppe, welche gegebenenfalls bis zu dreifach fluoriert sein kann,
- eine unsubstituierte C₂-C₄-Alkinylgruppe, welche gegebenenfalls einfach fluoriert sein kann,
- eine unsubstituierte C₃-C₆-Cycloalkylgruppe,
- ein unsubstituierter 5-6 gliedriger Aryl- oder Heteroarylring, wobei es sich dabei beispielsweise, aber nicht ausschließlich, um einen Phenyl-, Pyridinyl-, Pyrimidinyl-, Furanyl-, Thiophenyl-, Oxazolyl-, Isoxazolyl-, Thiazolyl-, Pyrrolyl-, Pyrazolyl-, Imidazolyl-, Pyrazinyl-, Pyridazinyl-, Triazolyl-, Tetrazolyl-Ring handeln kann, der gegebenenfalls bis zu zweifach mit Fluor, Chlor, Trifluormethyl substituiert sein kann, oder
R⁹, R¹⁰ zusammen einen 3-8-gliedrigen Ring bilden,
bedeuten.

10. Verbindungen gemaß den Ansprüchen 1, 2 und 6, wobei
X ein Stickstoffrest,
Y und Z ein Kohlenstoffrest -C-R⁸,
R⁸ ein Wasserstoff,
R¹ ein unsubstituierter 5-6-gliedriger Aryl- oder Heteroarylring, der gegebenenfalls ein- bis dreifach substituiert ist,
wobei die Substituenten ausgewählt sein können aus der Gruppe Halogen, -C₁-C₄-Alkyl, das unsubstituiert ist oder gegebenenfalls substituiert sein kann,
-OR⁶, -OC(O)R⁶, -S(O)ₙR⁶, wobei n = 0, 1, 2 bedeutet, -SO₂NHR⁶, -SO₂NHC(O)R⁶, NR⁶R⁷, -NHC(O)R⁶, -CN, -CO₂-R⁶, -C(O)-N-R⁶R⁷, -C(O)R⁶, -C(OH)R⁶R⁷,
wobei es sich bei dem 5-6-gliedrigen Aryl- oder Heteroarylring beispielsweise, aber nicht ausschließlich, um eine Phenyl-, Pyridinyl-, Pyrimidinyl-, Furanyl-, Thiophenyl-, Oxazolyl-, Isoxazolyl-, Thiazolyl-, Pyrrolyl-, Pyrazolyl-, Imidazolyl-, Pyrazinyl-, Pyridazinyl-,
Triazolyl-, Tetrazolyl- Gruppe handeln kann,
R² ein Wasserstoffrest,
R³ - R⁵ unabhängig voneinander ein Wasserstoff, Halogen, Cyano, oder
- eine OR⁶, OC(O)R⁶, S(O)ₙR⁶, wobei n = 0, 1, 2, bedeutet, SO₂NHR⁶, SO₂NHC(O)R⁶, NR⁶R⁷, NHC(O)R⁶, CH₂NR⁶R⁷, CH₂NHC(O)R⁶, C(OH)R⁶R⁷, C(O)R⁶, CO₂R⁶, C(O)NR⁶R⁷⁻ Gruppe,
- eine unsubstituierte C₁-C₆-Alkylgruppe, die gegebenenfalls substituiert sein kann,
- ein unsubstituierter C₃-C₁₀-Cycloalkylring, der gegebenenfalls substituiert sein kann,
- eine unsubstituierte C₂-C₆-Alkenyl-oder C₂-C₆-Alkinylgruppe, die gegebenenfalls substituiert sein kann,
- ein unsubstituierter 5-6-gliedriger Aryl- oder Heteroarylring, der gegebenenfalls ein-oder mehrfach substituiert ist, wobei die Substituenten ausgewählt sein können aus der
Gruppe
Halogen, -C₁-C₄-Alkyl, das unsubstituiert ist und gegebenenfalls auch halogeniert oder aber mit -OH,
-CN, -CO₂H substituiert sein kann, -OR⁹, -OC(O)R⁹, -S(O)ₙR⁹, wobei n = 0, 1, 2 bedeutet, -SO₂NHR⁹, -SO₂NHC(O)R⁹, NR⁹R¹⁰,-NHC(O)R⁹, -CN, -CO₂-R⁹, -C(O)-N-R⁹R¹⁰, -C(O)R⁹,-C(OH)R⁹R¹⁰,
wobei es sich bei dem 5-6-gliedrigen Aryl- oder Heteroarylring beispielsweise, aber nicht ausschließlich, um eine Phenyl-, Pyridinyl-, Pyrimidinyl-, Furanyl-, Thiophenyl-, Oxazolyl-, Isoxazolyl-, Thiazolyl-, Pyrrolyl-, Pyrazolyl-, Imidazolyl-, Pyrazinyl-, Pyridazinyl-,Triazolyl-, Tetrazolyl-, Gruppe handeln kann,
R⁶ R⁷ ein Wasserstoff,
eine unsubstituierte C₁-C₆-Alkylgruppe, die gegebenenfalls bis zu fünffach halogeniert sein kann, ein unsubstituierter C₃-C₈-Cycloalkylrest,
ein unsubstituierter 5-6-gliedriger Aryl- oder Heteroarylring, der gegebenenfalls ein- oder mehrfach substituiert sein kann, wobei die Substituenten ausgewählt sein können aus der Gruppe
Halogen, Cyano, -R⁹ -OR⁹, -OC(O)R⁹, -S(O)ₙR⁹ wobei n = 0, 1, 2 bedeutet, -SO₂NHR⁹, NR⁹R¹⁰, -NHC(O)R⁹, -CO₂-R⁹, -C(O)-N-R⁹R¹⁰,
wobei es sich bei dem 5-6-gliedrigen Aryl- oder Heteroarylring beispielsweise, aber nicht ausschließlich, um eine Phenyl-, Pyridinyl-, Pyrimidinyl-, Furanyl-, Thiophenyl-, Oxazolyl-, Isoxazolyl-, Thiazolyl-, Pyrrolyl-, Pyrazolyl-, Imidazolyl-, Pyrazinyl-, Pyridazinyl-Triazolyl-, Tetrazolyl-Gruppe handeln kann, oder
R⁶, R⁷ zusammen einen 3-8-gliedrigen Ring bilden,
R⁹, R¹⁰ unabhängig voneinander Wasserstoff, eine C₁-C₄-Alkylgruppe, welche unsubstituiert ist oder gegebenenfalls bis zu fünffach fluoriert sein kann,
eine unsubstituierte C₂-C₄-Alkenylgruppe, welche gegebenenfalls bis zu dreifach fluoriert sein kann,
eine unsubstituierte C₂-C₄-Alkinylgruppe, welche gegebenenfalls einfach fluoriert sein kann, eine unsubstituierte C₃-C₆-Cycloalkylgruppe,
ein unsubstituierter 5-6-gliedriger Aryl- oder Heteroarylring, wobei es sich dabei beispielsweise, aber nicht ausschließlich, um einen Phenyl-, Pyridinyl-, Pyrimidinyl-, Furanyl-, Thiophenyl-, Oxazolyl-, Isoxazolyl-, Thiazolyl-, Pyrrolyl-, Pyrazolyl-, Imidazolyl-, Pyrazinyl-, Pyridazinyl-;Triazolyl-, Tetrazolyl-Ring handeln kann, der gegebenenfalls bis zu zweifach mit Fluor, Chlor, Trifluormethyl substituiert sein kann, oder
R⁹, R¹⁰ zusammen einen 3-8-gliedrigen Ring bilden,
bedeuten.

11. Verbindungen gemaß den Ansprüchen 1, 3 und 7, wobei
Y und Z ein Stickstoffrest,
X ein Kohlenstoffrest -C-R⁸,
R⁸ ein Wasserstoff,
R¹ ein 5-6-gliedriger Aryl- oder Heteroarylring, der gegebenenfalls ein- bis dreifach substituiert ist,
wobei die Substituenten ausgewählt sein können aus der Gruppe Halogen, -C₁-C₄-Alkyl, das unsubstituiert ist oder gegebenenfalls substituiert sein kann,
-OR⁶, -OC(O)R⁶, -S(O)ₙR⁶, wobei n = 0, 1, 2 bedeutet, -SO₂NHR⁶, -SO₂NHC(O)R⁶, NR⁶R⁷, -NHC(O)R⁶, -CN, -CO₂-R⁶, -C(O)-N-R⁶R⁷, -C(O)R⁶, -C(OH)R⁶R⁷,
wobei es sich bei dem 5-6-gliedrigen Aryl- oder Heteroarylring beispielsweise, aber nicht ausschließlich, um eine Phenyl-, Pyridinyl-, Pyrimidinyl-, Furanyl-, Thiophenyl-, Oxazolyl-, Isoxazolyl-, Thiazolyl-, Pyrrolyl-, Pyrazolyl-, Imidazolyl-, Pyrazinyl-, Pyridazinyl-,
Triazolyl-, Tetrazolyl- Gruppe handeln kann,
R² ein Wasserstoffrest,
R³ - R⁵ unabhängig voneinander ein Wasserstoff, Halogen, Cyano, oder
- eine OR⁶, OC(O)R⁶, S(O)ₙR⁶, wobei n = 0, 1, 2, bedeutet, SO₂NHR⁶, SO₂NHC(O)R⁶, NR⁶R⁷, NHC(O)R⁶, CH₂NR⁶R⁷, CH₂NHC(O)R⁶, C(OH)R⁶R⁷, C(O)R⁶, CO₂R⁶, C(O)NR⁶R⁷ Gruppe,
- eine unsubstituierte C₁-C₆-Alkylgruppe, die gegebenenfalls substituiert sein kann,
- ein unsubstituierter C₃-C₁₀-Cycloalkylring, der gegebenenfalls substituiert sein kann,
- eine unsubstituierte C₂-C₆-Alkenyl-oder C₂-C₆-Alkinylgruppe, die gegebenenfalls substituiert sein kann,
- ein unsubstituierter 5-6gliedriger Aryl- oder Heteroarylring, der gegebenenfalls ein- oder mehrfach substituiert ist, wobei die Substituenten ausgewählt sein können aus der Gruppe
Halogen,
-C₁-C₄-Alkyl, das unsubstituiert ist und gegebenenfalls halogeniert oder aber mit -OH, -CN, -CO₂H substituiert sein kann,
-OR⁹, -OC(O)R⁹, -S(O)ₙR⁹ wobei n = 0, 1, 2 bedeutet,-SO₂NHR⁹, -SO₂NHC(O)R⁹, NR⁹R¹⁰, -NHC(O)R⁹, -CN,-CO₂-R⁹, -C(O)-N-R⁹R¹⁰, -C(O)R⁹, -C(OH)R⁹R¹⁰,
wobei es sich bei dem 5-6-gliedrigen Aryl- oder Heteroarylring beispielsweise, aber nicht ausschließlich, um eine Phenyl-, Pyridinyl-, Pyrimidinyl-, Furanyl-, Thiophenyl-, Oxazolyl-, Isoxazolyl-, Thiazolyl-, Pyrrolyl-, Pyrazolyl-, Imidazolyl-, Pyrazinyl-, Pyridazinyl-,Triazolyl-, Tetrazolyl-, Gruppe handeln kann,
R⁶, R⁷ ein Wasserstoff,
eine unsubstituierte C₁-C₆-Alkylgruppe, die gegebenenfalls bis zu fünffach halogeniert sein kann,
ein unsubstituierter C₃-C₈-Cycloalkylrest,
ein unsubstituierter 5-6gliedriger Aryl- oder Heteroarylring, der gegebenenfalls ein- oder mehrfach substituiert sein kann, wobei die Substituenten ausgewählt sein können aus der Gruppe
Halogen, Cyano, -R⁹ -OR⁹, -OC(O)R⁹, -S(O)ₙR⁹ wobei n = 0, 1, 2 bedeutet, -SO₂NHR⁹, NR⁹R¹⁰, -NHC(O)R⁹, -CO₂-R⁹, -C(O)-N-R⁹R¹⁰,
wobei es sich bei dem 5-6-gliedrigen Aryl- oder Heteroarylring beispielsweise, aber nicht ausschließlich, um eine Phenyl-, Pyridinyl-, Pyrimidinyl-, Furanyl-, Thiophenyl-, Oxazolyl-, Isoxazolyl-, Thiazolyl-, Pyrrolyl-, Pyrazolyl-, Imidazolyl-, Pyrazinyl-, Pyridazinyl-,
Triazolyl-, Tetrazolyl-Gruppe handeln kann, oder
R⁶, R⁷ zusammen einen 3-8-gliedrigen Ring bilden,
R⁹, R¹⁰ unabhängig voneinander Wasserstoff, eine C₁-C₄-Alkylgruppe, welche unsubstituiert oder gegebenenfalls bis zu fünffach fluoriert sein kann,
- eine unsubstituierte C₂-C₄-Alkenylgruppe, welche gegebenenfalls bis zu dreifach fluoriert sein kann,
- eine unsubstituierte C₂-C₄-Alkinylgruppe, welche gegebenenfalls einfach fluoriert sein kann,
- eine unsubstituierte C₃-C₆-Cycloalkylgruppe,
- ein unsubstituierter 5-6-gliedriger Aryl- oder Heteroarylring,
wobei es sich dabei beispielsweise, aber nicht ausschließlich, um einen Phenyl-, Pyridinyl-, Pyrimidinyl-, Furanyl-, Thiophenyl-, Oxazolyl-, Isoxazolyl-, Thiazolyl-, Pyrrolyl, Pyrazolyl-, Imidazolyl-, Pyrazinyl-, Pyridazinyl-, Triazolyl-, Tetrazolyl-Ring handeln kann, der gegebenenfalls bis zu zweifach mit Fluor, Chlor, Trifluormethyl substituiert sein kann, oder
R⁹, R¹⁰ zusammen einen 3-8-gliedrigen Ring bilden,
bedeuten.

12. Verbindungen gemäß den Anspüchen 1, 4 und 8, wobei
X und Z ein Stickstoffrest,
Y und Z ein Kohlenstoffrest -C-R⁸,
R⁸ ein Wasserstoff,
R¹ ein unsubstituierter 5-6-gliedriger Aryl- oder Heteroarylring, der gegebenenfalls ein- bis dreifach substituiert ist,
wobei die Substituenten ausgewählt sein können aus der Gruppe Halogen, -C₁-C₄-Alkyl, das unsubstituiert ist oder gegebenenfalls substituiert sein kann,
-OR⁶, -OC(O)R⁶, -S(O)ₙR⁶, wobei n = 0, 1, 2 bedeutet,-SO₂NHR⁶, -SO₂NHC(O)R⁶, NR⁶R⁷, -NHC(O)R⁶, -CN, -CO₂-R⁶, -C(O)-N-R⁶R⁷, -C(O)R⁶, -C(OH)R⁶R⁷,
wobei es sich bei dem 5-6-gliedrigen Aryl- oder Heteroarylring beispielsweise, aber nicht ausschließlich, um eine Phenyl-, Pyridinyl-, Pyrimidinyl-, Furanyl-, Thiophenyl-, Oxazolyl-, Isoxazolyl-, Thiazolyl-, Pyrrolyl-, Pyrazolyl-, Imidazolyl-, Pyrazinyl-, Pyridazinyl-,
Triazolyl-, Tetrazolyl- Gruppe handeln kann,
R² ein Wasserstoffrest,
R³- R⁵ unabhängig voneinander ein Wasserstoff, Halogen, Cyano, oder
- eine OR⁶, OC(O)R⁶, S(O)ₙR⁶, wobei n = 0, 1, 2, bedeutet, SO₂NHR⁶, SO₂NHC(O)R⁶, NR⁶R⁷, NHC(O)R⁶, CH₂NR⁶R⁷, CH₂NHC(O)R⁶, C(OH)R⁶R⁷, C(O)R⁶, CO₂R⁶, C(O)NR⁶R⁷-Gruppe,
- eine unsubstituierte C₁-C₆-Alkylgruppe, die gegebenenfalls substituiert sein kann,
- ein unsubstituierter C₃-C₁₀-Cycloalkylring, der gegebenenfalls substituiert sein kann,
- eine unsubstituierte C₂-C₆-Alkenyl-oder C₂-C₆-Alkinylgruppe, die gegebenenfalls substituiert sein kann,
- ein unsubstituierter 5-6-gliedriger Aryl- oder Heteroarylring, der gegebenenfalls ein- oder mehrfach substituiert ist, wobei die Substituenten ausgewählt sein können aus der Gruppe
Halogen,
-C₁-C₄-Alkyl, das unsubstituiert ist und gegebenenfalls auch halogeniert oder aber mit -OH, -CN, -CO₂H substituiert sein kann,
-OR⁹, -OC(O)R⁹, -S(O)ₙR⁹ wobei n = 0, 1, 2 bedeutet, -SO₂NHR⁹, -SO₂NHC(O)R⁹, NR⁹R¹⁰, -NHC(O)R⁹, -CN, -CO₂-R⁹, -C(O)-N-R⁹R¹⁰, -C(O)R⁹, -C(OH)R⁹R¹⁰,
wobei es sich bei dem 5-6-gliedrigen Aryl- oder Heteroarylring beispielsweise, aber nicht ausschließlich, um eine Phenyl-, Pyridinyl-, Pyrimidinyl-, Furanyl-, Thiophenyl-, Oxazolyl-, Isoxazolyl-, Thiazolyl-, Pyrrolyl-, Pyrazolyl-, Imidazolyl-, Pyrazinyl-, Pyridazinyl-,Triazolyl-, Tetrazolyl-, Gruppe handeln kann,
R⁶, R⁷ ein Wasserstoff,
eine unsubstituierte C₁-C₆-Alkylgruppe, die gegebenenfalls bis zu fünffach halogeniert sein kann,
ein unsubstituierter C₃-C₈-Cycloalkylrest,
ein unsubstituierter 5-6-gliedriger Aryl- oder Heteroarylring, der gegebenenfalls ein- oder mehrfach substituiert sein kann, wobei die Substituenten ausgewählt sein können aus der Gruppe
Halogen, Cyano, -R⁹ -OR⁹, -OC(O)R⁹, -S(O)ₙR⁹ wobei n = 0, 1, 2 bedeutet, -SO₂NHR⁹, NR⁹R¹⁰, -NHC(O)R⁹, -CO₂-R⁹, -C(O)-N-R⁹R¹⁰,
wobei es sich bei dem 5-6-gliedrigen Aryl- oder Heteroarylring beispielsweise, aber nicht ausschließlich, um eine Phenyl-, Pyridinyl-, Pyrimidinyl-, Furanyl-, Thiophenyl-, Oxazolyl-, Isoxazolyl-, Thiazolyl-, Pyrrolyl-, Pyrazolyl-, Imidazolyl-, Pyrazinyl-, Pyridazinyl-,
Triazolyl-, Tetrazolyl-Gruppe handeln kann, oder
R⁶, R⁷ zusammen einen 3-8-gliedrigen Ring bilden,
R⁹, R¹⁰ unabhängig voneinander Wasserstoff, eine C₁-C₄-Alkylgruppe, welche unsubstituiert oder gegebenenfalls bis zu fünffach fluoriert sein kann,
- eine unsubstituierte C₂-C₄-Alkenylgruppe, welche gegebenenfalls bis zu dreifach fluoriert sein kann,
- eine unsubstituierte C₂-C₄-Alkinylgruppe, welche gegebenenfalls einfach fluoriert sein kann, eine C₃-C₆-Cycloalkylgruppe,
- ein unsubstituierter 5-6-gliedriger Aryl- oder Heteroarylring,
wobei es sich dabei beispielsweise, aber nicht ausschließlich, um einen Phenyl-, Pyridinyl-, Pyrimidinyl-, Furanyl-, Thiophenyl-, Oxazolyl-, Isoxazolyl-, Thiazolyl-, Pyrrolyl-Pyrazolyl-, Imidazolyl-, Pyrazinyl-, Pyridazinyl-,Triazolyl-, Tetrazolyl-Ring handeln kann, der gegebenenfalls bis zu zweifach mit Fluor, Chlor, Trifluormethyl substituiert sein kann,oder
R⁹, R¹⁰ zusammen einen 3-8-gliedrigen Ring bilden,
bedeuten.

13. Verbindungen gemäß den Anspüchen 1, 5 und 9, wobei
X und Y ein Kohlenstoffrest -C-R⁸,
Z ein Stickstoffrest,
R⁸ ein Wasserstoff,
R¹ ein unsubstituierter 5-6-gliedriger Aryl- oder Heteroarylring, der gegebenenfalls ein- bis dreifach substituiert ist,
wobei die Substituenten ausgewählt sein können aus der Gruppe
Halogen,
-C₁-C₄-Alkyl, das unsubstituiert ist oder gegebenenfalls substituiert sein kann,
-OR⁶, -OC(O)R⁶, -S(O)ₙR⁶, wobei n = 0, 1, 2 bedeutet, -SO₂NHR⁶, -SO₂NHC(O)R⁶, NR⁶R⁷,-NHC(O)R⁶, -CN, -CO₂-R⁶, -C(O)-N-R⁶R⁷, -C(O)R⁶,-C(OH)R⁶R⁷,
wobei es sich bei dem 5-6-gliedrigen Aryl- oder Heteroarylring beispielsweise, aber nicht ausschließlich, um eine Phenyl-, Pyridinyl-, Pyrimidinyl-, Furanyl-, Thiophenyl-, Oxazolyl-, Isoxazolyl-, Thiazolyl-, Pyrrolyl-, Pyrazolyl-, Imidazolyl-, Pyrazinyl-, Pyridazinyl-,
Triazolyl-, Tetrazolyl- Gruppe handeln kann,
R² ein Wasserstoffrest,
R³ - R⁵ unabhängig voneinander ein Wasserstoff, Halogen, Cyano, oder
- eine OR⁶, OC(O)R⁶, S(O)ₙR⁶, wobei n = 0, 1, 2, bedeutet, SO₂NHR⁶, SO₂NHC(O)R⁶, NR⁶R⁷, NHC(O)R⁶, CH₂NR⁶R⁷, CH₂NHC(O)R⁶, C(OH)R⁶R⁷, C(O)R⁶, CO₂R⁶, C(O)NR⁶R⁷ Gruppe,
- eine unsubstituierte C₁-C₆-Alkylgruppe, die gegebenenfalls substituiert sein kann,
- ein unsubstituierter C₃-C₁₀-Cycloalkylring, der gegebenenfalls substituiert sein kann,
- eine unsubstituierte C₂-C₆-Alkenyl-oder C₂-C₆-Alkinylgruppe, die gegebenenfalls substituiert sein kann,
- ein unsubstituierter 5-6-gliedriger Aryl- oder Heteroarylring, der gegebenenfalls ein-oder mehrfach substituiert ist,
wobei die Substituenten ausgewählt sein können aus der Gruppe
Halogen,
-C₁-C₄-Alkyl, das unsubstituiert ist und gegebenenfalls auch halogeniert oder aber mit -OH, -CN, -CO₂H substituiert sein kann,
-OR⁹, -OC(O)R⁹, -S(O)ₙR⁹ wobei n = 0, 1, 2 bedeutet, -SO₂NHR⁹, -SO₂NHC(O)R⁹, NR⁹R¹⁰, -NHC(O)R⁹, -CN, -CO₂-R⁹, -C(O)-N-R⁹R¹⁰, -C(O)R⁹, -C(OH)R⁹R¹⁰,
wobei es sich bei dem 5-6-gliedrigen Aryl- oder
Heteroarylring beispielsweise, aber nicht ausschließlich, um eine Phenyl-, Pyridinyl-, Pyrimidinyl-, Furanyl-, Thiophenyl-, Oxazolyl-, Isoxazolyl-, Thiazolyl-, Pyrrolyl-, Pyrazolyl-, Imidazolyl-, Pyrazinyl-, Pyridazinyl-,Triazolyl-, Tetrazolyl-, Gruppe handeln kann,
R⁶, R⁷ ein Wasserstoff,
eine unsubstituierte C₁-C₆-Alkylgruppe, die gegebenenfalls bis zu fünffach halogeniert sein kann,
ein unsubstituierter C₃-C₈-Cycloalkylrest,
ein unsubstituierter 5-6-gliedriger Aryl- oder Heteroarylring, der gegebenenfalls ein- oder mehrfach substituiert sein kann, wobei die Substituenten ausgewählt sein können aus der Gruppe
Halogen, Cyano, -R⁹ -OR⁹, -OC(O)R⁹, -S(O)ₙR⁹ wobei n = 0, 1, 2 bedeutet, -SO₂NHR⁹, NR⁹R¹⁰, -NHC(O)R⁹, -CO₂-R⁹, -C(O)-N-R⁹R¹⁰,
wobei es sich bei dem 5-6-gliedrigen Aryl- oder Heteroarylring beispielsweise, aber nicht ausschließlich, um eine Phenyl-, Pyridinyl-, Pyrimidinyl-, Furanyl-, Thiophenyl-, Oxazolyl-, Isoxazolyl-, Thiazolyl-, Pyrrolyl-, Pyrazolyl-, Imidazolyl-, Pyrazinyl-, Pyridazinyl-,
Triazolyl-, Tetrazolyl--Gruppe handeln kann, oder
R⁶, R⁷ zusammen einen 3-8-gliedrigen Ring bilden,
R⁹, R¹⁰ unabhängig voneinander Wasserstoff, eine C₁-C₄-Alkylgruppe, welche unsubstituiert oder gegebenenfalls bis zu fünffach fluoriert sein kann,
- eine unsubstituierte C₂-C₄-Alkenylgruppe, welche gegebenenfalls bis zu dreifach fluoriert sein kann,
- eine unsubstituierte C₂-C₄-Alkinylgruppe, welche gegebenenfalls einfach fluoriert sein kann,
- eine unsubstituierte C₃-C₆-Cycloalkylgruppe,
- ein unsubstituierter 5-6-gliedriger Aryl- oder Heteroarylring,
wobei es sich dabei beispielsweise, aber nicht ausschließlich, um einen Phenyl-, Pyridinyl-, Pyrimidinyl-, Furanyl-, Thiophenyl-, Oxazolyl-, Isoxazolyl-, Thiazolyl-, Pyrrolyl-, Pyrazolyl-, Imidazolyl-, Pyrazinyl-, Pyridazinyl-, Triazolyl-, Tetrazolyl-Ring handeln kann, der gegebenenfalls bis zu zweifach mit Fluor, Chlor, Trifluormethyl substituiert sein kann, oder
R⁹, R¹⁰ zusammen einen 3-8-gliedrigen Ring bilden,
bedeuten.

14. Verbindungen gemäß den Anspüchen 1, 2, 6 und 10, wobei
X ein Stickstoffrest,
Y und Z ein Kohlenstoffrest -C-R⁸,
R⁸ ein Wasserstoff,
R¹ ein Phenyl-, Thiophen-2-yl, Thiophen-3-yl, Furan-2-yl-, Furan-3-yl-, Pyrid-3-yl-, Pyrid-4-ylring, der gegebenenfalls ein- bis dreifach substituiert ist,
wobei die Substituenten ausgewählt sein können aus der Gruppe
Halogen,
-C₁-C₄-Alkyl, das unsubstituiert oder gegebenenfalls substituiert sein kann,
-OR⁶, -OC(O)R⁶, -S(O)ₙR⁶, wobei n = 0, 1, 2 bedeutet, -SO₂NHR⁶, -SO₂NHC(O)R⁶, NR⁶R⁷,-NHC(O)R⁶, -CN, -CO₂-R⁶, -C(O)-N-R⁶R⁷, -C(O)R⁶,-C(OH)R⁶R⁷,
R² ein Wasserstoffrest,
R³ - R⁵ unabhängig voneinander ein Wasserstoff, Halogen, Cyano, oder
- eine OR⁶, OC(O)R⁶, S(O)ₙR⁶, wobei n = 0, 1, 2, bedeutet, SO₂NHR⁶, SO₂NHC(O)R⁶, NR⁶R⁷, NHC(O)R⁶, CH₂NR⁶R⁷, CH₂NHC(O)R⁶, C(OH)R⁶R⁷, C(O)R⁶, CO₂R⁶, C(O)NR⁶R⁷ Gruppe,
- eine unsubstituierte C₁-C₆-Alkylgruppe, die gegebenenfalls bis zu fünffach halogeniert oder aber mit, -CN, -CO₂H substituiert sein kann,
- ein unsubstituierter C₃-C₁₀-Cycloalkylring,
- eine unsubstituierte C₂-C₆-Alkenylgruppe, die gegebenenfalls bis zu dreifach halogeniert oder aber mit-CN, -CO₂H substituiert sein kann,
- eine unsubstituierte C₂-C₆-Alkinylgruppe, die gegebenenfalls einfach halogeniert oder aber mit -CN,-CO₂H substituiert sein kann und,
- ein unsubstituierter 5-6-gliedriger Aryl- oder Heteroarylring, der gegebenenfalls ein-oder mehrfach substituiert ist,
wobei die Substituenten ausgewählt sein können aus der
Gruppe
Halogen,
-C₁-C₄-Alkyl, das unsubstituiert ist und gegebenenfalls bis zu fünffach halogeniert oder aber mit-OH, -CN, -CO₂H substituiert sein kann,
-OR⁹, -OC(O)R⁹, -S(O)ₙR⁹, wobei n = 0, 1, 2 bedeutet, -SO₂NHR⁹, -SO₂NHC(O)R⁹, NR⁹R¹⁰,-NHC(O)R⁹, -CN, -CO₂-R⁹, -C(O)-N-R⁹R¹⁰, -C(O)R⁹,-C(OH)R⁹R¹⁰,
wobei es sich bei dem 5-6-gliedrigen Aryl- oder Heteroarylring beispielsweise, aber nicht ausschließlich, um eine Phenyl-, Pyridinyl-, Pyrimidinyl-, Furanyl-, Thiophenyl-, Oxazolyl-, Isoxazolyl-, Thiazolyl-, Pyrrolyl-, Pyrazolyl-, Imidazolyl-, Pyrazinyl-, Pyridazinyl-,
Triazolyl-, Tetrazolyl-, -Gruppe handeln kann,
R⁶, R⁷ ein Wasserstoff,
eine unsubstituierte C₁-C₆-Alkylgruppe, die gegebenenfalls bis zu fünffach halogeniert sein kann,
eine unsubstituierte C₃-C₈-Cycloalkylgruppe,
ein unsubstituierter 5-6-gliedriger Aryl- oder Heteroarylring, der gegebenenfalls ein- oder mehrfach substituiert sein kann, wobei die Substituenten ausgewählt sein können aus der Gruppe
Halogen,
Cyano,
R⁹ -OR⁹, -OC(O)R⁹, -S(O)ₙR⁹, wobei n = 0, 1, 2 bedeutet,-SO₂NHR⁹, - NR⁹R¹⁰, -NHC(O)R⁹, -CO₂-R⁹, -C(O)-N-R⁹R¹⁰,
wobei es sich bei dem 5-6-gliedrigen Aryl- oder Heteroarylring beispielsweise, aber nicht ausschließlich, um eine Phenyl-, Pyridinyl-, Pyrimidinyl-, Furanyl-, Thiophenyl-, Oxazolyl-, Isoxazolyl-, Thiazolyl-, Pyrrolyl-, Pyrazolyl-, Imidazolyl-, Pyrazinyl-, Pyridazinyl-,Triazolyl-, Tetrazolyl-, Gruppe handeln kann, oder
R⁶, R⁷ zusammen einen 3-8-gliedrigen Ring bilden,
R⁹, R¹⁰ unabhängig voneinander Wasserstoff, eine C₁-C₄-Alkylgruppe, welche unsubstituiert ist oder gegebenenfalls bis zu fünffach fluoriert sein kann,
eine unsubstituierte C₂-C₄-Alkenylgruppe, welche gegebenenfalls bis zu dreifach fluoriert sein kann,
eine unsubstituierte C₂-C₄-Alkinylgruppe, welche gegebenenfalls einfach fluoriert sein kann,
eine unsubstituierte C₃-C₆-Cycloalkylgruppe,
ein unsubstituierter 5-6-gliedriger Aryl- oder Heteroarylring, wobei es sich dabei beispielsweise, aber nicht ausschließlich, um einen Phenyl-, Pyridinyl-, Pyrimidinyl-, Furanyl-, Thiophenyl-, Oxazolyl-, Isoxazolyl-, Thiazolyl-, Pyrrolyl-, Pyrazolyl-, Imidazolyl-, Pyrazinyl-, Pyridazinyl-, Triazolyl-, Tetrazolyl-Ring handeln kann, der gegebenenfalls bis zu zweifach mit Fluor, Chlor, Trifluormethyl substituiert sein kann, oder
R⁹, R¹⁰ zusammen einen 3-8-gliedrigen Ring bilden,
bedeuten.

15. Verbindungen gemäß den Anspüchen 1, 3, 7 und 11, wobei
Y und Z ein Stickstoffrest,
X ein Kohlenstoffrest -C-R⁸,
R⁸ ein Wasserstoff,
R¹ ein Phenyl-, Thiophen-2-yl, Thiophen-3-yl, Furan-2-yl-, Furan-3-yl-, Pyrid-3-yl-, Pyrid-4-ylring, der gegebenenfalls ein- bis dreifach substituiert ist,
wobei die Substituenten ausgewählt sein können aus der Gruppe Halogen, -C₁-C₄-Alkyl, das unsubstituiert oder gegebenenfalls substituiert sein kann,
-OR⁶, -OC(O)R⁶, -S(O)ₙR⁶, wobei n = 0, 1, 2 bedeutet,-SO₂NHR⁶, -SO₂NHC(O)R⁶, NR⁶R⁷, -NHC(O)R⁶, -CN, -CO₂-R⁶, -C(O)-N-R⁶R⁷, -C(O)R⁶, -C(OH)R⁶R⁷,
R² ein Wasserstoffrest,
R³- R⁵ unabhängig voneinander ein Wasserstoff, Halogen, Cyano, oder
- eine OR⁶, OC(O)R⁶, S(O)ₙR⁶, wobei n = 0, 1, 2, bedeutet, SO₂NHR⁶, SO₂NHC(O)R⁶, NR⁶R⁷, NHC(O)R⁶, CH₂NR⁶R⁷, CH₂NHC(O)R⁶, C(OH)R⁶R⁷, C(O)R⁶, CO₂R⁶, C(O)NR⁶R⁷ Gruppe,
- eine unsubstituierte C₁-C₆-Alkylgruppe, die gegebenenfalls bis zu fünffach halogeniert oder aber mit -CN, -CO₂H substituiert sein kann,
- ein unsubstituierter C₃-C₁₀-Cycloalkylring,
- eine unsubstituierte C₂-C₆-Alkenylgruppe, die gegebenenfalls bis zu dreifach halogeniert oder aber mit-CN, -CO₂H substituiert sein kann,
- eine unsubstituierte C₂-C₆-Alkinylgruppe, die gegebenenfalls einfach halogeniert oder aber mit -CN,-CO₂H substituiert sein kann und,
- ein unsubstituierter 5-6-gliedriger Aryl- oder Heteroarylring, der gegebenenfalls ein-oder mehrfach substituiert ist,
wobei die Substituenten ausgewählt sein können aus der
Gruppe
Halogen,
C₁-C₄-Alkyl, das unsubstituiert ist und gegebenenfalls bis zu fünffach halogeniert oder aber mit -OH, -CN,-CO₂H substituiert sein kann,
-OR⁹, -OC(O)R⁹, -S(O)ₙR⁹, wobei n = 0, 1, 2 bedeutet, -SO₂NHR⁹, -SO₂NHC(O)R⁹, NR⁹R¹⁰,-NHC(O)R⁹, -CN, -CO₂-R⁹, -C(O)-N-R⁹R¹⁰, -C(O)R⁹,-C(OH)R⁹R¹⁰,
wobei es sich bei dem 5-6-gliedrigen Aryl- oder Heteroarylring beispielsweise, aber nicht ausschließlich, um eine Phenyl-, Pyridinyl-, Pyrimidinyl-, Furanyl-, Thiophenyl-, Oxazolyl-, Isoxazolyl-, Thiazolyl-, Pyrrolyl-, Pyrazolyl-, Imidazolyl-, Pyrazinyl-, Pyridazinyl-,
Triazolyl-, Tetrazolyl-, -Gruppe handeln kann,
R⁶, R⁷ ein Wasserstoff,
eine unsubstituierte C₁-C₆-Alkylgruppe, die gegebenenfalls bis zu fünffach halogeniert sein kann,
eine unsubstituierte C₃-C₈-Cycloalkylgruppe,
ein unsubstituierter 5-6-gliedriger Aryl- oder Heteroarylring, der gegebenenfalls ein- oder mehrfach substituiert sein kann, wobei die Substituenten ausgewählt sein können aus der Gruppe
- Halogen,
- Cyano,
R⁹, -OR⁹, -OC(O)R⁹, -S(O)ₙR⁹, wobei n = 0, 1, 2 bedeutet,-SO₂NHR⁹, - NR⁹R¹⁰, -NHC(O)R⁹, -CO₂-R⁹, -C(O)-N-R⁹R¹⁰,
wobei es sich bei dem 5-6-gliedrigen Aryl- oder Heteroarylring beispielsweise, aber nicht ausschließlich, um eine Phenyl-, Pyridinyl-, Pyrimidinyl-, Furanyl-, Thiophenyl-, Oxazolyl-, Isoxazolyl-, Thiazolyl-, Pyrrolyl-, Pyrazolyl-, Imidazolyl-, Pyrazinyl-, Pyridazinyl-,
Triazolyl-, Tetrazolyl-, Gruppe handeln kann, oder
R⁶, R⁷ zusammen einen 3-8-gliedrigen Ring bilden,
R⁹, R¹⁰ unabhängig voneinander Wasserstoff, eine C₁-C₄-Alkylgruppe, welche unsubstituiert ist oder gegebenenfalls bis zu fünffach fluoriert sein kann,
eine unsubstituierte C₂-C₄-Alkenylgruppe, welche gegebenenfalls bis zu dreifach fluoriert sein kann,
eine unsubstituierte C₂-C₄-Alkinylgruppe, welche gegebenenfalls einfach fluoriert sein kann,
eine unsubstituierte C₃-C₆-Cycloalkylgruppe,
ein unsubstituierter 5-6-gliedriger Aryl- oder Heteroarylring, wobei es sich dabei beispielsweise, aber nicht ausschließlich, um einen Phenyl-, Pyridinyl-, Pyrimidinyl-, Furanyl-, Thiophenyl-, Oxazolyl-, Isoxazolyl-, Thiazolyl-, Pyrrolyl-, Pyrazolyl-, Imidazolyl-, Pyrazinyl-, Pyridazinyl-, Triazolyl-, Tetrazolyl-Ring handeln kann, der gegebenenfalls bis zu zweifach mit Fluor, Chlor, Trifluormethyl substituiert sein kann, oder
R⁹, R¹⁰ zusammen einen 3-8-gliedrigen Ring bilden,
bedeuten.

16. Verbindungen gemäß den Anspüchen 1, 4, 8 und 12, wobei
X und Z ein Stickstoffrest,
Y ein Kohlenstoffrest -C-R⁸,
R⁸ ein Wasserstoff,
R¹ ein Phenyl-, Thiophen-2-yl, Thiophen-3-yl, Furan-2-yl-, Furan-3-yl-, Pyrid-3-yl-, Pyrid-4-ylring, der gegebenenfalls ein- bis dreifach substituiert ist,
wobei die Substituenten ausgewählt sein können aus der Gruppe Halogen, -C₁-C₄-Alkyl, das unsubstituiert oder gegebenenfalls substituiert sein kann,
-OR⁶, -OC(O)R⁶, -S(O)ₙR⁶, wobei n = 0, 1, 2 bedeutet,-SO₂NHR⁶, -SO₂NHC(O)R⁶, NR⁶R⁷, -NHC(O)R⁶, -CN, -CO₂-R⁶, -C(O)-N-R⁶R⁷, -C(O)R⁶, -C(OH)R⁶R⁷,
R² ein Wasserstoffrest,
R³ - R⁵ unabhängig voneinander ein Wasserstoff, Halogen, Cyano, oder
- eine OR⁶, OC(O)R⁶, S(O)ₙR⁶, wobei n = 0, 1, 2, bedeutet, SO₂NHR⁶, SO₂NHC(O)R⁶, NR⁶R⁷, NHC(O)R⁶, CH₂NR⁶R⁷, CH₂NHC(O)R⁶, C(OH)R⁶R⁷, C(O)R⁶, CO₂R⁶, C(O)NR⁶R⁷⁻ Gruppe,
- eine unsubstituierte C₁-C₆-Alkylgruppe, die gegebenenfalls bis zu fünffach halogeniert oder aber mit -CN, -CO₂H substituiert sein kann,
- ein unsubstituierter C₃-C₁₀-Cycloalkylring,
- eine unsubstituierte C₂-C₆-Alkenylgruppe, die gegebenenfalls bis zu dreifach halogeniert oder aber mit-CN, -CO₂H substituiert sein kann,
- eine unsubstituierte C₂-C₆-Alkinylgruppe, die gegebenenfalls einfach halogeniert oder aber mit -CN,-CO₂H substituiert sein kann, und
- ein unsubstituierter 5-6-gliedriger Aryl- oder Heteroarylring, der gegebenenfalls ein-oder mehrfach substituiert ist,
wobei die Substituenten ausgewählt sein können aus der Gruppe
- Halogen,
-C₁-C₄-Alkyl, das unsubstituiert ist und gegebenenfalls bis zu fünffach halogeniert oder aber mit -OH, -CN, -CO₂H substituiert sein kann,
-OR⁹, -OC(O)R⁹, -S(O)ₙR⁹, wobei n = 0, 1, 2 bedeutet, -SO₂NHR⁹, -SO₂NHC(O)R⁹, NR⁹R¹⁰,-NHC(O)R⁹, -CN, -CO₂-R⁹, -C(O)-N-R⁹R¹⁰, -C(O)R⁹,-C(OH)R⁹R¹⁰,
wobei es sich bei dem 5-6-gliedrigen Aryl- oder Heteroarylring beispielsweise, aber nicht ausschließlich, um eine Phenyl-, Pyridinyl-, Pyrimidinyl-, Furanyl-, Thiophenyl-, Oxazolyl-, Isoxazolyl-, Thiazolyl-, Pyrrolyl-, Pyrazolyl-, Imidazolyl-, Pyrazinyl-, Pyridazinyl-,
Triazolyl-, Tetrazolyl-, -Gruppe handeln kann,
R⁶, R⁷ ein Wasserstoff,
eine C₁-C₆-Alkylgruppe, die unsubstituiert oder gegebenenfalls bis zu fünffach halogeniert sein kann,
eine C₃-C₈-Cycloalkylgruppe,
ein 5-6-gliedriger Aryl- oder Heteroarylring, der gegebenenfalls ein- oder mehrfach substituiert sein kann, wobei die Substituenten ausgewählt sein können aus der Gruppe
Halogen,
Cyano,
eine unsubstituierte C₃-C₈-Cycloalkylgruppe, R⁹, -OR⁹, -OC(O)R⁹, -S(O)ₙR⁹, wobei n = 0, 1, 2 bedeutet,-SO₂NHR⁹, - NR⁹R¹⁰, -NHC(O)R⁹, -CO₂-R⁹, -C(O)-N-R⁹R¹⁰,
wobei es sich bei dem 5-6-gliedrigen Aryl- oder Heteroarylring beispielsweise, aber nicht ausschließlich, um eine Phenyl-, Pyridinyl-, Pyrimidinyl-, Furanyl-, Thiophenyl-, Oxazolyl-, Isoxazolyl-, Thiazolyl-, Pyrrolyl-, Pyrazolyl-, Imidazolyl-, Pyrazinyl-, Pyridazinyl-,
Triazolyl-, Tetrazolyl-, Gruppe handeln kann, oder
R⁶, R⁷ zusammen einen 3-8-gliedrigen Ring bilden,
R⁹, R¹⁰ unabhängig voneinander Wasserstoff, eine C₁-C₄-Alkylgruppe, welche unsubstituiert ist oder gegebenenfalls bis zu fünffach fluoriert sein kann,
eine unsubstituierte C₂-C₄-Alkenylgruppe, welche gegebenenfalls bis zu dreifach fluoriert sein kann,
eine unsubstituierte C₂-C₄-Alkinylgruppe, welche gegebenenfalls einfach fluoriert sein kann,
eine unsubstituierte C₃-C₆-Cycloalkylgruppe,
ein unsubstituierter 5-6-gliedriger Aryl- oder Heteroarylring, wobei es sich dabei beispielsweise, aber nicht ausschließlich, um einen Phenyl-, Pyridinyl-, Pyrimidinyl-, Furanyl-, Thiophenyl-, Oxazolyl-, Isoxazolyl-, Thiazolyl-, Pyrrolyl-, Pyrazolyl-, Imidazolyl-, Pyrazinyl-, Pyridazinyl-, Triazolyl-, Tetrazolyl-Ring handeln kann, der gegebenenfalls bis zu zweifach mit Fluor, Chlor, Trifluormethyl substituiert sein kann, oder
R⁹, R¹⁰ zusammen einen 3-8-gliedrigen Ring bilden,
bedeuten.

17. Verbindungen gemäß den Anspüchen 1, 5, 9 und 13, wobei
X und Y ein Kohlenstoffrest -C-R⁸,
Z ein Stickstoffrest,
R⁸ ein Wasserstoff,
R¹ ein Phenyl-, Thiophen-2-yl, Thiophen-3-yl, Furan-2-yl-, Furan-3-yl-, Pyrid-3-yl-, Pyrid-4-ylring, der gegebenenfalls ein- bis dreifach substituiert ist,
wobei die Substituenten ausgewählt sein können aus der Gruppe Halogen, -C₁-C₄-Alkyl, das unsubstituiert oder gegebenenfalls substituiert sein kann,
-OR⁶, -OC(O)R⁶, -S(O)ₙR⁶, wobei n = 0, 1, 2 bedeutet,-SO₂NHR⁶, -SO₂NHC(O)R⁶, NR⁶R⁷, -NHC(O)R⁶, -CN, -CO₂-R⁶, -C(O)-N-R⁶R⁷, -C(O)R⁶, -C(OH)R⁶R⁷,
R² ein Wasserstoffrest,
R³ - R⁵ unabhängig voneinander ein Wasserstoff, Halogen, Cyano, oder
- eine OR⁶, OC(O)R⁶, S(O)ₙR⁶, wobei n = 0, 1, 2, bedeutet, SO₂NHR⁶, SO₂NHC(O)R⁶, NR⁶R⁷, NHC(O)R⁶, CH₂NR⁶R⁷, CH₂NHC(O)R⁶, C(OH)R⁶R⁷, C(O)R⁶, CO₂R⁶, C(O)NR⁶R⁷⁻ Gruppe,
- eine unsubstituierte C₁-C₆-Alkylgruppe, die gegebenenfalls bis zu fünffach halogeniert oder aber mit -CN, -CO₂H substituiert sein kann,
- ein unsubstituierter C₃-C₁₀-Cycloalkylring,
- eine unsubstituierte C₂-C₆-Alkenylgruppe,die gegebenenfalls bis zu dreifach halogeniert oder aber mit-CN, -CO₂H substituiert sein kann,
- eine unsubstituierte C₂-C₆-Alkinylgruppe, die gegebenenfalls einfach halogeniert oder aber mit -CN,-CO₂H substituiert sein kann und,
- ein unsubstituierter 5-6-gliedriger Aryl- oder Heteroarylring, der gegebenenfalls ein-oder mehrfach substituiert ist,
wobei die Substituenten ausgewählt sein können aus der Gruppe
- Halogen,
-C₁-C₄-Alkyl, das unsubstituiert ist und gegebenenfalls bis zu fünffach halogeniertoder aber mit -OH, -CN, -CO₂H substituiert sein kann,
-OR⁹, -OC(O)R⁹, -S(O)ₙR⁹, wobei n = 0, 1, 2 bedeutet, -SO₂NHR⁹, -SO₂NHC(O)R⁹, NR⁹R¹⁰,-NHC(O)R⁹, -CN, -CO₂-R⁹, -C(O)-N-R⁹R¹⁰, -C(O)R⁹,-C(OH)R⁹R¹⁰,
wobei es sich bei dem 5-6-gliedrigen Aryl- oder Heteroarylring beispielsweise, aber nicht ausschließlich, um eine Phenyl-, Pyridinyl-, Pyrimidinyl-, Furanyl-, Thiophenyl-, Oxazolyl-, Isoxazolyl-, Thiazolyl-, Pyrrolyl-, Pyrazolyl-, Imidazolyl-, Pyrazinyl-, Pyridazinyl-,Triazolyl-, Tetrazolyl-, -Gruppe handeln kann,
R⁶, R⁷ ein Wasserstoff,
eine C₁-C₆-Alkylgruppe, die unsubstituiert ist oder gegebenenfalls bis zu fünffach halogeniert sein kann,
ein 5-6-gliedriger Aryl- oder Heteroarylring, der gegebenenfalls ein- oder mehrfach substituiert sein kann, wobei die Substituenten ausgewählt sein können aus der Gruppe
Halogen,
Cyano,
eine C₃-C₈-Cycloalkylgruppe,
R⁹, -OR⁹, -OC(O)R⁹, -S(O)ₙR⁹, wobei n = 0, 1, 2 bedeutet, -SO₂NHR⁹, - NR⁹R¹⁰, -NHC(O)R⁹, -CO₂-R⁹, -C(O)-N-R⁹R¹⁰,
wobei es sich bei dem 5-6-gliedrigen Aryl- oder Heteroarylring beispielsweise, aber nicht ausschließlich, um eine Phenyl-, Pyridinyl-, Pyrimidinyl-, Furanyl-, Thiophenyl-, Oxazolyl-, Isoxazolyl-, Thiazolyl-, Pyrrolyl-, Pyrazolyl-, Imidazolyl-, Pyrazinyl-, Pyridazinyl-,
Triazolyl-, Tetrazolyl-, Gruppe handeln kann, oder
R⁶, R⁷ zusammen einen 3-8-gliedrigen Ring bilden,
R⁹, R¹⁰ unabhängig voneinander Wasserstoff, eine C₁-C₄-Alkylgruppe, welche unsubstituiert ist oder gegebenenfalls bis zu fünffach fluoriert sein kann,
eine unsubstituierte C₂-C₄-Alkenylgruppe, welche gegebenenfalls bis zu dreifach fluoriert sein kann,
eine unsubstituierte C₂-C₄-Alkinylgruppe, welche gegebenenfalls einfach fluoriert sein kann,
eine unsubstituierte C₃-C₆-Cycloalkylgruppe,
ein unsubstituierter 5-6-gliedriger Aryl- oder Heteroarylring, wobei es sich dabei beispielsweise, aber nicht ausschließlich, um einen Phenyl-, Pyridinyl-, Pyrimidinyl-, Furanyl-, Thiophenyl-, Oxazolyl-, Isoxazolyl-, Thiazolyl-, Pyrrolyl-, Pyrazolyl-, Imidazolyl-, Pyrazinyl-, Pyridazinyl-,Triazolyl-, Tetrazolyl-Ring handeln kann, der gegebenenfalls bis zu zweifach mit Fluor, Chlor, Trifluormethyl substituiert sein kann, oder
R⁹, R¹⁰ zusammen einen 3-8-gliedrigen Ring bilden,
bedeuten.

18. Verbindungen gemäß den Ansprüchen 1 - 17, ausgewählt aus einer Gruppe, die die folgenden Verbindungen enthält:
- 1-[1-(7-Chloro-quinolin-4-yl)-piperidin-4-yl]-3-(4-methylsulfanyl-phenyl)-thiourea
- 1-[1-(7-Chloro-quinolin-4-yl)-piperidin-4-yl]-3-phenyl-thiourea
- 1-(3-Chloro-phenyl)-3-[1-(7-chloro-quinolin-4-yl)-piperidin-4-yl]-thiourea
- 1-(2-Chloro-phenyl)-3-[1-(7-chloro-quinolin-4-yl)-piperidin-4-yl]-thiourea
- 1-(1-(7-Chloro-quinolin-4-yl)-piperidin-4-yl)-3-(4-methoxy-phenyl)-thiourea
- 1-(1-(7-Chloro-quinolin-4-yl)-piperidin-4-yl)-3-p-tolyl-thiourea
- 1-(1-(7-Chloro-quinolin-4-yl)-piperidin-4-yl)-3-o-tolyl-thiourea
- 1-(2-Bromo-phenyl)-3-(1-(7-chloro-quinolin-4-yl)-piperidin-4-yl)-thiourea
- 1-(3-Bromo-phenyl)-3-(1-(7-chloro-quinolin-4-yl)-piperidin-4-yl)-thiourea
- 1-(1-(7-Chloro-quinolin-4-yl)-piperidin-4-yl)-3-(3-methoxy-phenyl)-thiourea
- 4-(3-(1-(7-Chloro-quinolin-4-yl)-piperidin-4-yl)-thioureido)-benzoic acid methyl ester
- 1-(1-(7-Chloro-quinolin-4-yl)-piperidin-4-yl)-3-(3-cyano-phenyl)-thiourea
- 1-(1-(7-Chloro-quinolin-4-yl)-piperidin-4-yl)-3-(4-cyano-phenyl)-thiourea
- 1-(1-(7-Chloro-quinolin-4-yl)-piperidin-4-yl)-3-(2-methylsulfanyl-phenyl)-thiourea
- 1-(1-(7-Chloro-quinolin-4-yl)-piperidin-4-yl)-3-(2,3-dichloro-phenyl)-thiourea
- 1-(1-(7-Chloro-quinolin-4-yl)-piperidin-4-yl)-3-(3-methylsulfanyl-phenyl)-thiourea
- 3-(3-(1-(7-Chloro-quinolin-4-yl)-piperidin-4-yl)-thioureido)-benzoic acid methyl ester
- 1-(1-(7-Chloro-quinolin-4-yl)-piperidin-4-yl)-3-(2-trifluoromethoxy-phenyl)-thiourea
- 1-[1-(8-Chloro-quinolin-4-yl)-piperidin-4-yl]-3-(4-methylsulfanyl-phenyl)-thiourea
- 1-[1-(8-Chloro-quinolin-4-yl)-piperidin-4-yl]-3-phenyl-thiourea
- 1-(3-Chloro-phenyl)-3-[1-(8-chloro-quinolin-4-yl)-piperidin-4-yl]-thiourea
- 1-(2-Chloro-phenyl)-3-[1-(8-chloro-quinolin-4-yl)-piperidin-4-yl]-thiourea
- 1-(1-(8-Chloro-quinolin-4-yl)-piperidin-4-yl)-3-(4-methoxy-phenyl)-thiourea
- 1-(1-(8-Chloro-quinolin-4-yl)-piperidin-4-yl)-3-p-tolyl-thiourea
- 1-(1-(8-Chloro-quinolin-4-yl)-piperidin-4-yl)-3-o-tolyl-thiourea
- 1-(2-Bromo-phenyl)-3-(1-(8-chloro-quinolin-4-yl)-piperidin-4-yl)-thiourea
- 1-(3-Bromo-phenyl)-3-(1-(8-chloro-quinolin-4-yl)-piperidin-4-yl)-thiourea
- 1-(1-(8-Chloro-quinolin-4-yl)-piperidin-4-yl)-3-(3-methoxy-phenyl)-thiourea
- 4-(3-(1-(8-Chloro-quinolin-4-yl)-piperidin-4-yl)-thioureido)-benzoic acid methyl ester
- 1-(1-(8-Chloro-quinolin-4-yl)-piperidin-4-yl)-3-(3-cyano-phenyl)-thiourea
- 1-(1-(8-Chloro-quinolin-4-yl)-piperidin-4-yl)-3-(4-cyano-phenyl)-thiourea
- 1-(1-(8-Chloro-quinolin-4-yl)-piperidin-4-yl)-3-(2-methylsulfanyl-phenyl)-thiourea
- 1-(1-(8-Chloro-quinolin-4-yl)-piperidin-4-yl)-3-(2,3-dichloro-phenyl)-thiourea
- 1-(1-(8-Chloro-quinolin-4-yl)-piperidin-4-yl)-3-(3-methylsulfanyl-phenyl)-thiourea
- 3-(3-(1-(8-Chloro-quinolin-4-yl)-piperidin-4-yl)-thioureido)-benzoic acid methyl ester
- 1-(1-(8-Chloro-quinolin-4-yl)-piperidin-4-yl)-3-(2-trifluoromethoxy-phenyl)-thiourea
- 1-[1-(6-Chloro-quinolin-4-yl)-piperidin-4-yl]-3-(4-methylsulfanyl-phenyl)-thiourea
- 1-[1-(6-Chloro-quinolin-4-yl)-piperidin-4-yl]-3-phenyl-thiourea
- 1-(3-Chloro-phenyl)-3-[1-(6-chloro-quinolin-4-yl)-piperidin-4-yl]-thiourea
- 1-(2-Chloro-phenyl)-3-[1-(6-chloro-quinolin-4-yl)-piperidin-4-yl]-thiourea
- 1-(1-(6-Chloro-quinolin-4-yl)-piperidin-4-yl)-3-(4-methoxy-phenyl)-thiourea
- 1-(1-(6-Chloro-quinolin-4-yl)-piperidin-4-yl)-3-p-tolyl-thiourea
- 1-(1-(6-Chloro-quinolin-4-yl)-piperidin-4-yl)-3-o-tolyl-thiourea
- 1-(2-Bromo-phenyl)-3-(1-(6-chloro-quinolin-4-yl)-piperidin-4-yl)-thiourea
- 1-(3-Bromo-phenyl)-3-(1-(6-chloro-quinolin-4-yl)-piperidin-4-yl)-thiourea
- 1-(1-(6-Chloro-quinolin-4-yl)-piperidin-4-yl)-3-(3-methoxy-phenyl)-thiourea
- 4-(3-(1 -(6-Chloro-quinolin-4-yl)-piperidin-4-yl)-thioureido)-benzoic acid methyl ester
- 1-(1-(6-Chloro-quinolin-4-yl)-piperidin-4-yl)-3-(3-cyano-phenyl)-thiourea
- 1-(1-(6-Chloro-quinolin-4-yl)-piperidin-4-yl)-3-(4-cyano-phenyl)-thiourea
- 1-(1-(6-Chloro-quinolin-4-yl)-piperidin-4-yl)-3-(2-methylsulfanyl-phenyl)-thiourea
- 1-(1-(6-Chloro-quinolin-4-yl)-piperidin-4-yl)-3-(2,3-dichloro-phenyl)-thiourea
- 1-(1-(6-Chloro-quinolin-4-yl)-piperidin-4-yl)-3-(3-methylsulfanyl-phenyl)-thiourea
- 3-(3-(1 -(6-Chloro-quinolin-4-yl)-piperidin-4-yl)-thioureido)-benzoic acid methyl ester
- 1-(1-(6-Chloro-quinolin-4-yl)-piperidin-4-yl)-3-(2-trifluoromethoxy-phenyl)-thiourea
- 1-[1-(6-Bromo-phthalazin-1-yl)-piperidin-4-yl]-3-(4-methylsulfanyl-phenyl)-thiourea
- 1-[1-(6-Bromo-phthalazin-1-yl)-piperidin-4-yl]-3-phenyl-thiourea
- 1-(3-Chloro-phenyl)-3-[1-(6-bromo-phthalazin-1-yl)-piperidin-4-yl]-thiourea
- 1-(2-Chloro-phenyl)-3-[1-(6-bromo-phthalazin-1-yl)-piperidin-4-yl]-thiourea
- 1-(1-(6-Bromo-phthalazin-1-yl)-piperidin-4-yl)-3-(4-methoxy-phenyl)-thiourea
- 1-(1-(6-Bromo-phthalazin-1-yl)-piperidin-4-yl)-3-p-tolyl-thiourea
- 1-(1-(6-Bromo-phthalazin-1-yl)-piperidin-4-yl)-3-o-tolyl-thiourea
- 1-(2-Bromo-phenyl)-3-(1-(6-bromo-phthalazin-1-yl)-piperidin-4-yl)-thiourea
- 1-(3-Bromo-phenyl)-3-(1-(6-bromo-phthalazin-1-yl)-piperidin-4-yl)-thiourea
- 1-(1-(6-Bromo-phthalazin-1-yl)-piperidin-4-yl)-3-(3-methoxy-phenyl)-thiourea
- 4-(3-(1-(6-Bromo-phthalazin-1-yl)-piperidin-4-yl)-thioureido)-benzoic acid methyl ester
- 1-(1-(6-Bromo-phthalazin-1-yl)-piperidin-4-yl)-3-(3-cyano-phenyl)-thiourea
- 1-(1-(6-Bromo-phthalazin-1-yl)-piperidin-4-yl)-3-(4-cyano-phenyl)-thiourea
- 1-(1-(6-Bromo-phthalazin-1-yl)-piperidin-4-yl)-3-(2-methylsulfanyl-phenyl)-thiourea
- 1-(1-(6-Bromo-phthalazin-1-yl)-piperidin-4-yl)-3-(2,3-dichloro-phenyl)-thiourea
- 1-(1-(6-Bromo-phthalazin-1-yl)-piperidin-4-yl)-3-(3-methylsulfanyl-phenyl)-thiourea
- 3-(3-(1-(6-Bromo-phthalazin-1-yl)-piperidin-4-yl)-thioureido)-benzoic acid methyl ester
- 1-(1-(6-Bromo-phthalazin-1-yl)-piperidin-4-yl)-3-(2-trifluoromethoxy-phenyl)-thiourea
- 1-[1-(7-Bromo-phthalazin-1-yl)-piperidin-4-yl]-3-(4-methylsulfanyl-phenyl)-thiourea
- 1-[1-(7-Bromo-phthalazin-1-yl)-piperidin-4-yl]-3-(4-methylsulfanyl-phenyl)-thiourea
- 1-[1-(7-Bromo-phthalazin-1-yl)-piperidin-4-yl]-3-phenyl-thiourea
- 1-(3-Chloro-phenyl)-3-[1-(7-bromo-phthalazin-1-yl)-piperidin-4-yl]-thiourea
- 1-(2-Chloro-phenyl)-3-[1-(7-bromo-phthalazin-1-yl)-piperidin-4-yl]-thiourea
- 1-(1-(7-Bromo-phthalazin-1-yl)-piperidin-4-yl)-3-(4-methoxy-phenyl)-thiourea
- 1-(1-(7-Bromo-phthalazin-1-yl)-piperidin-4-yl)-3-p-tolyl-thiourea
- 1-(1-(7-Bromo-phthalazin-1-yl)-piperidin-4-yl)-3-o-tolyl-thiourea
- 1-(2-Bromo-phenyl)-3-(1-(7-bromo-phthalazin-1-yl)-piperidin-4-yl)-thiourea
- 1-(3-Bromo-phenyl)-3-(1-(7-bromo-phthalazin-1-yl)-piperidin-4-yl)-thiourea
- 1-(1-(7-Bromo-phthalazin-1-yl)-piperidin-4-yl)-3-(3-methoxy-phenyl)-thiourea
- 4-(3-(1-(7-Bromo-phthalazin-1-yl)-piperidin-4-yl)-thioureido)-benzoic acid methyl ester
- 1-(1-(7-Bromo-phthalazin-1-yl)-piperidin-4-yl)-3-(3-cyano-phenyl)-thiourea
- 1-(1-(7-Bromo-phthalazin-1-yl)-piperidin-4-yl)-3-(4-cyano-phenyl)-thiourea
- 1-(1-(7-Bromo-phthalazin-1-yl)-piperidin-4-yl)-3-(2-methylsulfanyl-phenyl)-thiourea
- 1-(1-(7-Bromo-phthalazin-1-yl)-piperidin-4-yl)-3-(2,3-dichtoro-phenyl)-thiourea
- 1-(1-(7-Bromo-phthalazin-1-yl)-piperidin-4-yl)-3-(3-methylsulfanyl-phenyl)-thiourea
- 3-(3-(1-(7-Bromo-phthatazin-1-yl)-piperidin-4-yl)-thioureido)-benzoic acid methyl ester
- 1-(1-(7-Bromo-phthalazin-1-yl)-piperidin-4-yl)-3-(2-trifluoromethoxy-phenyl)-thiourea
- 1-[1-(7-Chloro-quinazolin-4-yl)-piperidin-4-yl]-3-(4-methylsulfanyl-phenyl)-thiourea
- 1-[1-(7-Chloro-quinazolin-4-yl)-piperidin-4-yl]-3-phenyl-thiourea
- 1-(3-Chloro-phenyl)-3-[1-(7-chloro-quinazolin-4-yl)-piperidin-4-yl]-thiourea
- 1-(2-Chloro-phenyl)-3-[1-(7-chloro-quinazolin-4-yl)-piperidin-4-yl]-thiourea
- 1-(1-(7-Chloro-quinazolin-4-yl)-piperidin-4-yl)-3-(4-methoxy-phenyl)-thiourea
- 1-(1-(7-Chloro-quinazolin-4-yl)-piperidin-4-yl)-3-p-tolyl-thiourea
- 1-(1-(7-Chloro-quinazolin-4-yl)-piperidin-4-yl)-3-o-tolyl-thiourea
- 1-(2-Bromo-phenyl)-3-(1-(7-chloro-quinazolin-4-yl)-piperidin-4-yl)-thiourea
- 1-(3-Bromo-phenyl)-3-(1-(7-chloro-quinazolin-4-yl)-piperidin-4-yl)-thiourea
- 1-(1 -(7-Chloro-quinazolin-4-yl)-piperidin-4-yl)-3-(3-methoxy-phenyl)-thiourea
- 4-(3-(1-(7-Chloro-quinazolin-4-yl)-piperidin-4-yl)-thioureido)-benzoic acid methyl ester
- 1-(1-(7-Chloro-quinazolin-4-yl)-piperidin-4-yl)-3-(3-cyano-phenyl)-thiourea
- 1-(1-(7-Chloro-quinazolin-4-yl)-piperidin-4-yl)-3-(4-cyano-phenyl)-thiourea
- 1-(1-(7-Chloro-quinazolin-4-yl)-piperidin-4-yl)-3-(2-methylsulfanyl-phenyl)-thiourea
- 1-(1-(7-Chloro-quinazolin-4-yl)-piperidin-4-yl)-3-(2,3-dichloro-phenyl)-thiourea
- 1-(1-(7-Chloro-quinazolin-4-yl)-piperidin-4-yl)-3-(3-methylsulfanyl-phenyl)-thiourea
- 3-(3-(1-(7-Chloro-quinazolin-4-yl)-piperidin-4-yl)-thioureido)-benzoic acid methyl ester
- 1-(1-(7-Chloro-quinazolin-4-yl)-piperidin-4-yl)-3-(2-trifluoromethoxy-phenyl)-thiourea
- 1-(1-Isoquinolin-1-yl-piperidin-4-yl)-3-(4-methylsulfanyl-phenyl)-thiourea
- 1-(1-Isoquinolin-1-yl-piperidin-4-yl)-3-phenyl-thiourea
- 1-(3-Chloro-phenyl)-3-(1-Isoquinolin-1-yl-piperidin-4-yl)-thiourea
- 1-(2-Chloro-phenyl)-3-(1-Isoquinolin-1-yl-piperidin-4-yl)-thiourea
- 1-(1-Isoquinolin-1-yl-piperidin-4-yl)-3-(4-methoxy-phenyl)-thiourea
- 1-(1-Isoquinolin-1-yl-piperidin-4-yl)-3-p-tolyl-thiourea
- 1-(1-Isoquinolin-1-yl-piperidin-4-yl)-3-o-tolyl-thiourea
- 1-(2-Bromo-phenyl)-3-(1-Isoquinolin-1-yl-piperidin-4-yl)-thiourea
- 1-(3-Bromo-phenyl)-3-(1-Isoquinolin-1-yl-piperidin-4-yl)-thiourea
- 1-(1-Isoquinolin-1-yl-piperidin-4-yl)-3-(3-methoxy-phenyl)-thiourea
- 4-(3-(1-Isoquinolin-1-yl-piperidin-4-yl)-thioureido)-benzoic acid methyl ester
- 1-(1-Isoquinolin-1-yl-piperidin-4-yl)-3-(3-cyano-phenyl)-thiourea
- 1-(1-Isoquinolin-1-yl-piperidin-4-yl)-3-(4-cyano-phenyl)-thiourea
- 1-(1-Isoquinolin-1-yl-piperidin-4-yl)-3-(2-methylsulfanyl-phenyl)-thiourea
- 1-(1-Isoquinolin-1-yl-piperidin-4-yl)-3-(2,3-dichloro-phenyl)-thiourea
- 1-(1-Isoquinolin-1-yl-piperidin-4-yl)-3-(3-methylsulfanyl-phenyl)-thiourea
- 3-(3-(1-Isoquinolin-1-yl-piperidin-4-yl)-thioureido)-benzoic acid methyl ester
- 1-(1-Isoquinolin-1-yl-piperidin-4-yl)-3-(2-trifluoromethoxy-phenyl)-thiourea

19. Verwendung der Verbindungen gemäß der Ansprüche 1 - 18 zur Herstellung von Arzneimitteln, die mindestens eine der Verbindungen der Formel I enthalten.

20. Arzneimittel gemäß Anspruch 19 mit geeigneten Formulierungs- und Trägerstoffen.

21. Verwendung der Arzneimittel gemäß Anspruch 19 und 20, **dadurch gekennzeichnet, dass** das die Arzneimittel zur Behandlung und Prophylaxe von Erkrankungen verwendet wird.

22. Verwendung gemäß Anspruch 21, zur Behandlung und Prophylaxe von Erkrankungen, die im Zusammenhang mit dem EP₂-Rezeptor stehen.

23. Verwendung gemäß Anspruch 21 zur Behandlung und Prophylaxe von Fertilitätsstörungen.

24. Verwendung gemäß Anspruch 21 zur Behandlung und Prophylaxe von Menstruationsbeschwerden.

25. Verwendung gemäß Anspruch 21 zur Behandlung und Prophylaxe von Endometriose.

26. Verwendung der Verbindungen gemäß Anspruch 1-18 zur Modulation des EP₂ Rezeptors.

27. Verwendung gemäß Anspruch 21 zur Behandlung und Prophylaxe von Schmerz.

28. Verwendung der Verbindungen gemäß Anspruch 1 - 18 und der Arzneimittel gemäß Anspruch 19 und 20 zur Fertilitätskontrolle.

29. Verwendung gemäß Anspruch 21 zur Behandlung und Prophylaxe von Osteoporose.

30. Verwendung gemäß Anspruch 21 zur Behandlung und Prophylaxe von Krebs.

31. Verwendung der Verbindungen der allgemeinen Formel I, gemäß den Ansprüchen 1 - 18, in Form eines pharmazeutischen Präparates für die enterale, parenterale, vaginale und orale Applikation.
